Europäisches Patentamt

European Patent Office

Office européen des brevets

(11) Publication number: **0 269 127 B1**

(19)

## EUROPEAN PATENT SPECIFICATION

(45) Date of publication of patent specification: **23.02.94**

(51) Int. Cl.⁵: **C07K 15/00**, C12N 15/11, C12P 19/34, C07H 21/00, C12Q 1/68, G01N 33/577

(21) Application number: **87117619.4**

(22) Date of filing: **27.11.87**

(54) **Nucleotide sequences which are selectively expressed in pre-B cells and probes therefor.**

(30) Priority: **27.11.86 GB 8628433**
**14.07.87 GB 8716497**
**14.10.87 GB 8724100**

(43) Date of publication of application:
**01.06.88 Bulletin 88/22**

(45) Publication of the grant of the patent:
**23.02.94 Bulletin 94/08**

(84) Designated Contracting States:
**CH DE FR GB IT LI NL**

(56) References cited:

**THE EMBO JOURNAL, vol. 5, no. 9, September 1986, pages 2139-2147, IRL Press Ltd, Oxford, GB; N. SAKAGUCHI et al.: "Isolation of a cDNA copy of an RNA species expressed in murine pre-B cells"**

**NATURE, vol. 311, 25th October 1984, pages 727-733; G.D. YANCOPOULOS et al.: "Preferential utilization of the most JH-proximal VH gene segments in pre-B-cell lines"**

(73) Proprietor: **F. HOFFMANN-LA ROCHE AG**
**Postfach 3255**
**CH-4002 Basel(CH)**

(72) Inventor: **Bauer, Steven R., Dr.**
**37 Burenweg**
**CH-4127 Birsfelden(CH)**
Inventor: **Kudo, Akira, Dr.**
**114 Claragraben**
**CH-4057 Basle(CH)**
Inventor: **Melchers, Georg Friedrich, Prof. Dr.**
**29 Muttenzer Strasse**
**D-7889 Grenzach(DE)**
Inventor: **Sakaguchi, Nobuo, Dr. c/o Dept. of Immunology**
**Saga Medical School of Nabeshima**
**Saga 840-01(JP)**

(74) Representative: **Lederer, Franz, Dr. et al**
**Lederer, Keller & Riederer,**
**Patentanwälte,**
**Lucile-Grahn-Strasse 22**
**D-81675 München (DE)**

THE EMBO JOURNAL, vol. 6, no. 1, January 1987, pages 103-107, IRL Press Ltd, Oxford, GB; A. KUDO et al.: "Organization of the murine Ig-related lambda5 gene transcribed selectively in pre-B lymphocytes"

THE EMBO JOURNAL, vol. 6, no. 8, August 1987, pages 2267-2272, IRL Press Ltd, Oxford, GB; A. KUDO et al.: "A second gene, VpreB in the lambda5 locus of the mouse, which appears to be selectively expressed in pre-B lymphocytes"

## Description

The present invention relates to nucleotide sequences which are selectively expressed in human pre-B cells and to probes comprising a polynucleotide hybridizing specifically to such a nucleotide sequence. The invention further relates to replicable microbial vectors containing a polynucleotide hybridizing to a nucleotide sequence mentioned above, to host organisms transformed with such a replicable microbial vector, which host is capable of producing copies of said polynucleotide, to methods for the production of the probes and to a method and a test kit used for determining whether a cell is a pre-B cell using such a probe. Moreover the invention relates to replicable microbial vectors containing a polynucleotide having a sequence which is selectively expressed in pre-B cells, to host organisms transformed with such a replicable microbial vector, which host is capable of translating the amino acid sequence encoded by a transcript comprising said nucleotide sequence and to a polypeptide having said amino acid sequence. Furthermore the invention relates to methods for the production of said polypeptide, to methods for the production of antibodies against said polypeptide, to methods and a test kit used for the determination of said polypeptide and to the antibodies per se.

B-cells, a subclass of lymphocytes, develop from pluripotent stem cells in a series of differentiation steps in specialized organs such as fetal liver and bone marrow. During this development immunoglobulin (Ig) gene segments are rearranged to form complete Ig genes for heavy (H) and light (L) chains (Tonegawa, Nature 302, 575-581, [1983]). Rearrangements appear ordered in time of development: H chain gene segments are rearranged before L chain gene segments (Maki, et al., Science 209, 1366-1369, [1980]; Alt, et al., Cell 27, 381-390 [1981]; Perry, et al., Proc. Natl. Acad. Sci. USA 78, 247-251, [1981]; Coffman, et al., J. Mol. Cell. Immunol. 1, 31-38, [1983]; Reth, et al., Nature 317, 353-355, [1985]). This distinguishes different stages of precursor (pre)-B cell development. The most immature pre-B cell appears to have only $D_H$ segments rearranged to $J_H$ segments. Pre-B cells follow in which $V_H$ segments have been rearranged to $D_H J_H$ segments (Alt et al., supra; Sugiyama, et al., Nature 303, 812-815, [1983]; Alt, et al., EMBO J. 3, 1209-1219, [1984]). The next stage is a pre-B cell which has not only rearranged $V_H D_H J_H$, but also $V_K$ to $J_K$ (Maki, et al., supra; Lewis, et al., Cell 30, 807-816, [1982]; Burrows, et al., Nature 306, 243-246, [1983]), and finally, $V_\lambda$ to $J_\lambda$ (Hieter, et al.,
Nature 290, 368-372, [1981]). The result of this process of B cell development is a resting, IgM- and/or IgD-positive B cell which can be stimulated by antigens or mitogens to divide and to mature into memory cells and into Ig-secreting plasma cells which often have switched their IgH gene expression and therefore synthesize other classes of Ig. By analysing the rearrangement pattern of its Ig genes the stage of development of a given B cell may be determined. Since for each cell at a given stage of normal lymphocyte differentiation there exists a malignant counterpart, an analysis of the rearrangement pattern may also be used for the classification of leukemias as shown in European Patent Application, publication No. 149 547 (published July 24, 1985). However the Southern blot technique (Southern, J. Mol. Biol. 98, 503-517, [1975]) used in the method described in the above mentioned patent application is time consuming and relatively complicated in comparison with other methods used routinely in clinical laboratories.

The identification of stages of pre-B and B cell development (the B cell lineage) is also aided by monoclonal antibodies which characterize surface membrane markers that are preferentially expressed on subsets of pre-B and B lymphocytes or plasma cells (McKearn et al., J. Immunol. 132, 332-339, [1984]; Coffman, et al., Nature 289, 681-683, [1981]); Kincade, et al., J. Immunol. 127, 2262-2268, [1981]). But the markers recognized by these antibodies have been found to be also expressed on other lineages of blood cells and can accordingly not be used to specifically and unambiguously determine the stage of differentiation of a given B cell or its malignant counterpart.

In a new approach Sakaguchi et al. (EMBO J. 5, 3139-2147 [1986]) have constructed a library of cDNA clones from a murine pre-B cell line and were able to identify a cDNA which is a partial-sequence of a gene which is selectively expressed in murine pre-B lymphomas, but not in B-lymphomas, myelomas, T cell lymphomas, macrophage tumors or in a fibroblast line from mice. Further details on this cDNA, which is derived from the so-called λ5 gene, are given in the Example below. The complete nucleotide sequence of this cDNA is as follows:

3

GGAATAGCTTTTGGCCACCAGAGGAGGAACAATCCTTTTGCCGGGAGATCTACACTGCAA

GTGAGGCTAGAGTTGACTTTGGACTTGAGGGTCAATGAAGCTCAGAGTAGGACAGACTCT

GGGCACTATCCCCAGGCAGTGTGAAGTTCTCCTCCTGCTGCTGCTGTTGGGTCTAGTGGA

TGGTGTCCACCACATACTTTCCCCAAGCTCAGCAGAAAGGAGCAGAGCTGTGGGCCCTGG

AGCTTCAGTGGGAAGCAACAGGCCTAGCCTATGGGCCCTTCCCGGCAGGCTCCTGTTCCA

GATCATCCCACGGGGAGCAGGTCCCAGGTGCTCGCCCCATAGGCTTCCATCTAAGCCCCA

GTTTTGGTATGTCTTTGGTGGTGGGACCCAGCTCACAATCCTAGGTCAGCCCAAGTCTGA

CCCCTTGGTCACTCTGTTCCTGCCTTCCTTAAAGAATCTTCAGCCAACAAGGCCACACGT

AGTGTGTTTGGTGAGCGAATTCTACCCAGGTACTTTGGTGGTGGACTGGAAGGTAGATGG

GGTCCCTGTCACTCAGGGTGTAGAGACAACCCAACCCTCCAAACAGACCAACAACAAATA

CATGGTCAGCAGCTACCTGACACTGATATCTGACCAGTGGATGCCTCACAGTAGATACAG

CTGCCGGGTCACTCATGAAGGAAACACTGTGGAGAAGAGTGTGTCACCTGCTGAGTGTTC

TTAGAGCACAATCCTCCCTGAAGCCTCAGGGGCCTGGATCTGAAGTGCCAGAAAAAGTTG

TTTTTTGTTTTGTTTTTTGTTTTTTTTTCCCATTAACCATCTCACTGTCTTTCCTGTGCCT

AATACTCAATAAATATCTTACCACCAACC

The present invention provides a nucleotide sequence from a further gene which is selectively expressed in pre-B cells, i.e. from the so-called v$_{preB}$ gene. In mice two v$_{preB}$ genes were found, i.e. v$_{preB}$1 and v$_{preB}$2. The nucleotide sequence of a cDNA corresponding to the v$_{preB}$1 gene of the mouse was found to be as follows:

CCAGAAAGCCTGGGAGGGTGGTGAGCAGGAACCAGGGGTGCAGTGACCCTCTCCCCAAAG

CAGGGAGGAGAGTGCTTCCCAGCTGGTCAGGGCCCAGGAGCAGTGGCTGTAGGGGGCAGG

GTGCTGCAGGTCTGGAGCCATGGCCTGGACGTCTGTCCTGCTCATGCTGCTGGCCTATCT

CACAGGTTGTGGCCCTCAGCCCATGGTGCATCAGCCACCATTAGCATCTTCTTCCCTTGG

AGCCACCATCCGCCTCTCCTGTACCCTGAGCAACGACCATAACATTGGCATTTACAGCAT

TTACTGGTACCAGCAGAGGCCGGGCCACCCTCCCAGGTTCCTGCTGAGATACTTCTCACA

CTCAGACAAGCACCAGGGTCCCGATATCCCACCTCGCTTCTCCGGGTCCAAAGATACGAC

CAGGAACCTGGGGTATCTGAGCATCTCTGAACTGCAGCCTGAGGACGAGGCTGTGTATTA

CTGTGCCGTGGGGCTCCGGAGCCAGGAAAAGAAGAGGATGGAGAGGGAGTGGGAAGGAGA

AAAGTCGTATACAGATTTGGGATCTTAGGCTCTGGAGACATTCAGACCCTGAACTGAAGA

CAGAGTTTGCTTTGCTCGGCTAGTCTGGTATGGGAAGGAGGGGTAGAACGTGAGGTTTTG

CAGAGCCTAGAAGATGGAATTATGCAGCTTTTCCTTGTTCTGCGGTGTTGCTATGAGCCC

CCATTGGAGGCTGGATTGTAGAATTAAAGCTGTTTTTACTG

A search for a possible human equivalent of the v$_{preB}$ gene was successful, showing that surprisingly the v$_{preB}$ gene is not mouse specific, but occurs also in humans. However, in humans only one form of the v$_{preB}$ gene was found. Polynucleotides capable of hybridizing to the human v$_{preB}$ gene and/or its mRNA may be used as a new tool for the determination of the stage of differentiation of B cells, more precisely for the determination of human pre-B cells. Such a tool may be used to determine if a given leukemia is of the non-T acute lymphoblastic leukemia type (non-T ALL) or not.

Therefore, the present invention relates to polynucleotides which are capable of specifically hybridizing to a nucleotide sequence selectively expressed in human pre-B cells, which polynucleotide comprises a nucleotide sequence which is

GAGCTCTGCATGTCTGCACCATGTCCTGGGCTCCTGTCCTGCTCATGCACTTTGTCTACT

GCACAGGTGAGGGAACCCCCAGATCCCAAAGACTCCTGCCCCTTCCTTCATCCTGCCCTG

CCCCCACGGGCCACATGCATCTGTGTCACCAGGTTGTGGTCCTCAGCCGGTGCTACATCA

GCCGCCGGCCATGTCCTCGGCCCTTGGAACCACAATCCGCCTCACCTGCACCCTGAGGAA

CGACCATGACATCGGTGTGTACAGCGTCTACTGGTACCAGCAGAGGCCGGGCCACCCTCC

CAGGTTCCTGCTGAGATATTTCTCACAATCAGACAAGAGCCAGGGCCCCCAGGTCCCCCC

TCGCTTCTCTGGATCCAAAGATGTGGCCAGGAACAGGGGGTATTTGAGCATCTCTGAGCT

GCAGCCTGAGGACGAGGCTATGTATTACTGTGCTATGGGGGCCCGCAGCTCGGAGAAGGA

GGAGAGGGAGAGGGAGTGGGAGGAAGAAATGGAACCCACTGCAG

or a sub-part thereof or a homologous or degenerate sequence thereof. Said polynucleotides may be prepared by using conventional polynucleotide synthesis procedures or by culturing host organisms transformed with a replicable microbial vector comprising such a polynucleotide under conditions permitting production of multiple copies of said polynucleotide and isolating said polynucleotides from the culture.

The invention further relates to replicable microbial vectors, host organisms, polypeptides, antibodies and probes as well as to methods for the production of said polynucleotides, polypeptides, antibodies and probes as defined in the appended claims.

The invention further relates to a methods for determining the presence of a pre-B cell in a sample and to the use of said polynucleotides or probes for the determination of the presence of pre-B cells in a sample as well as to test kits for determining the presence of a pre-B cell in a sample as defined in the appended claims.

Preferably the sequence of the polynucleotide of the probe is complementary to one of the nucleotide sequences shown above or to parts thereof. Such a polynucleotide may be prepared by using conventional polynucleotide synthesis procedures or by methods known in the art of recombinant DNA technology for the production of multiple copies of said polynucleotide (see for example Maniatis, et al., "Molecular Cloning - A Laboratory Manual", Cold Spring Harbor Laboratory, [1982]).

The invention further relates to replicable microbial vectors containing the polynucleotide of the probe and to host organisms containing such a replicable microbial vector, which host is capable of producing copies of said polynucleotide. By incorporating a label into the polynucleotide obtained by the methods indicated above, a probe capable of showing hybridization to a nucleotide sequence which is selectively expressed in pre-B cells is obtained. The present invention relates also to such probes and to methods for their production. In addition to that the invention relates to a method and a test kit for determining whether an unknown cell is a pre-B cell using said probe. Moreover the invention relates to replicable microbial vectors containing a polynucleotide with a sequence which is selectively expressed in pre-B cells, to host organisms transformed with such a replicable microbial vector, which host is capable of expressing the amino acid sequence encoded by the polynucleotide of said replicable microbial vector and to a polypeptide having said amino acid sequence. Furthermore the invention relates to methods for the production of said polypeptide, to methods for the production of antibodies against said polypeptide, to methods and a test kit used for the determination of said polypeptide and to the antibodies per se.

The invention will be better understood on the basis of the following detailed description when considered in connection with the accompanying Figures 1-29. These Figures are meant to show

Fig. 1    Expression of pZ 183 transcripts in cell lines representing different stages of the B cell lineage and in cell lines of other lineages. Only pre-B cells show expression of pZ 183 transcripts.

Fig. 2    Quantitative dot blot analysis of pZ 183 transcripts in different cell lines. Such transcripts were only found in pre-B cells but not in mature B cells, plasma cells and not in various non-

5

B lineage cells.

Fig. 3      Northern blot analysis of pZ 183 transcripts in RNA isolated from unstimulated and LPS stimulated 70Z/3 cells. One pZ 183-specific transcript was found, with a size of 1,2 kb (1 kb = 1000 nucleotides). LPS stimulation (12 hrs., 10 μg/ml) of 70Z/3 cells showed no effect on the expression of this transcript.

Fig. 4      Northern blot analysis of pZ 183 transcripts in the cells of normal organs and tissues. Only in 70Z/3 cells such transcripts were detected. (a) Northern blot, (b) ethidium bromide stained gel.

Fig. 5      Detection of pZ 183 specific mRNA molecules in single cells of lymphoid organs by in situ hybridization. Bars 10 μm.

      a) Day 15 fetal liver cells hybridized with pZ 183 negative strand probe, pronase digestion 66 μg/ml, exposure time 62 days.

      b) As in a) but hybridized with pZ 183 positive strand probe.

      c) Adult bone marrow cells hybridized with pZ 183 negative strand probe, pronase digestion 33 μg/ml, exposure time 49 days.

      d) Small resting spleen cells hybridized with pZ 183 negative strand probe, pronase digestion 11 μg/ml, exposure time 49 days.

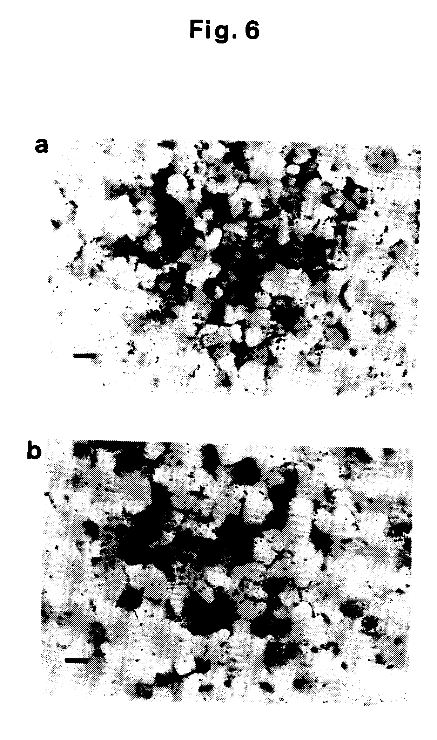

Fig. 6      Localization of cells containing pZ 183 specific mRNA molecules in day 16 fetal liver. Cells were digested with 30 μg/ml pronase. Exposure time 63 days. Bars 5 μm. a) Hybridization with pZ 183 negative strand probe. b) Hybridization with pZ 183 positive strand probe.

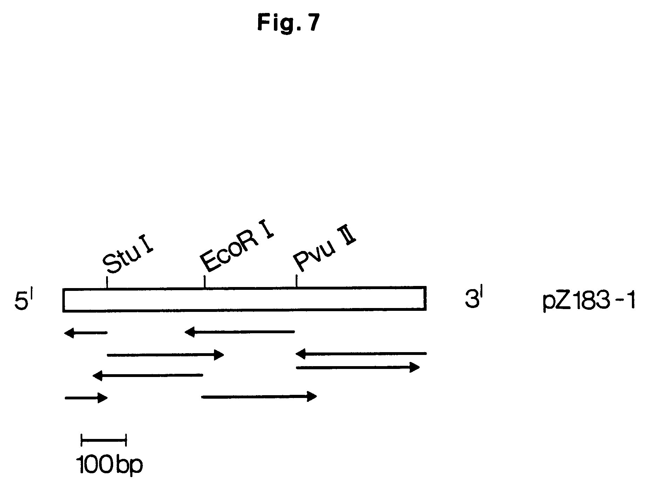

Fig. 7      Restriction enzyme map of the 0.7kb insert of clone pZ 183-1. The arrow show the length of the fragments used for subcloning into M13 and the direction in which the given fragment was sequenced.

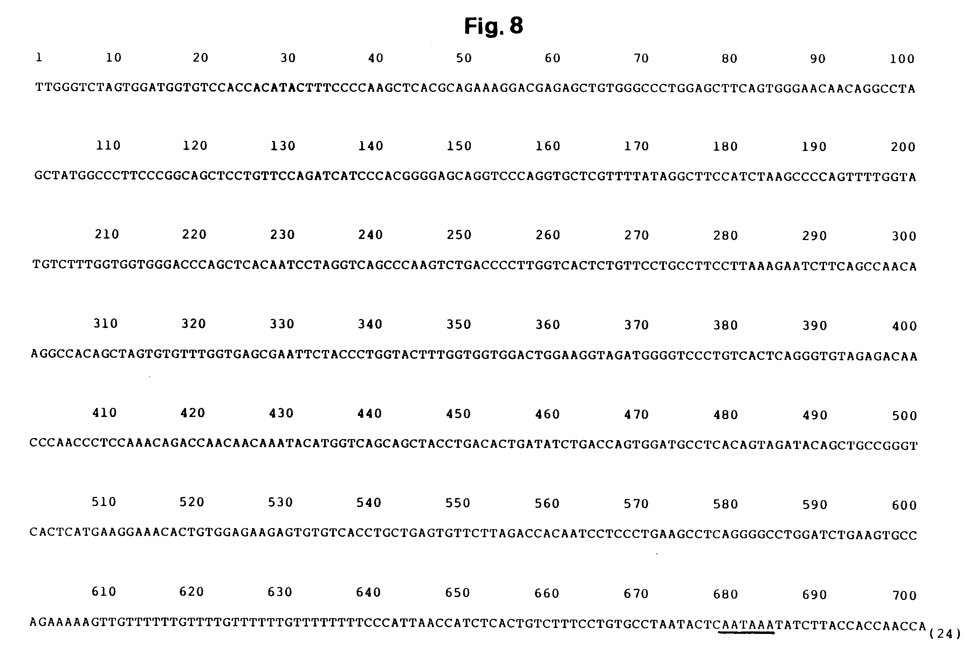

Fig. 8      Nucleotide sequence of the 0.7kb insert of clone pZ 183-1. The proposed poly(A) attachment site is underlined.

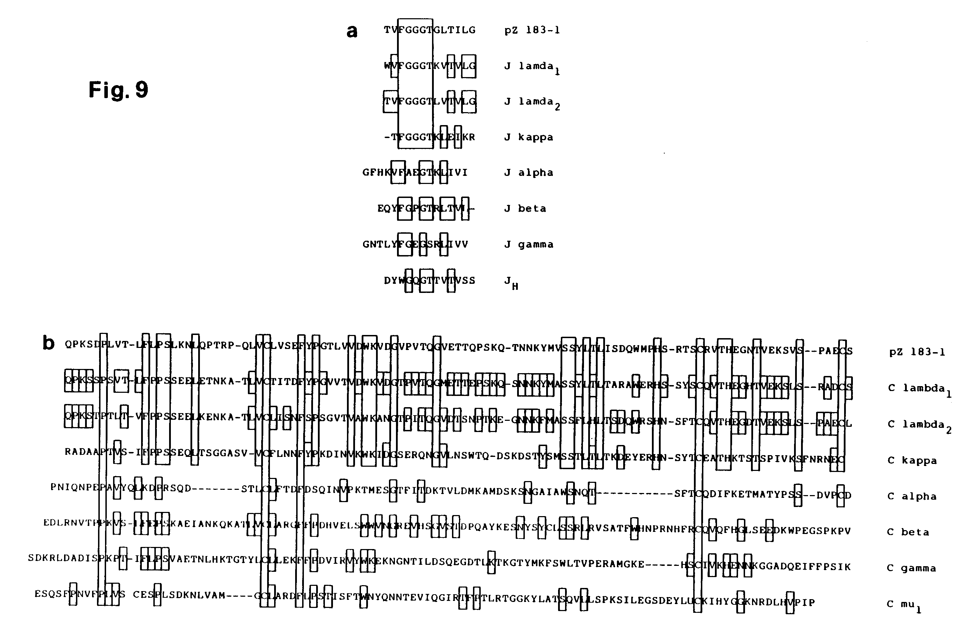

Fig. 9      Amino acid sequence of the J-like (a) and C-like (b) portions of pZ 183-1 encoded by the nucleotide sequence in fig. 8 in frame 1 and alignment with the corresponding sequences of λ1L, λ2L, ϰL, $J_H$ and $C_\mu 1$ (for references see: Bothwell, et al., Nature 298, 380-382, [1982] and Kabat, et al., in "Sequences of proteins of immunological interest", NIH, Bethesda, U.S.A., [1983]) and $J_\alpha C_\alpha 1$, $J_\beta C_\beta$ and $J_\gamma C_\gamma$ of the α, β and γ chains of the T cell receptor (Saito, et al., Nature 312, 36-40, [1984]; Hedrick, et al., Nature 308, 149-158, [1984]).

   For nucleotide positions corresponding to the first and the last deduced amino acid of the J-like and C-like sequences of pZ 183-1 see Table II. Those residues common between pZ 183-1 and any other sequence are boxed. Residues conserved in all sequences or conserved in light chains are boxed together.

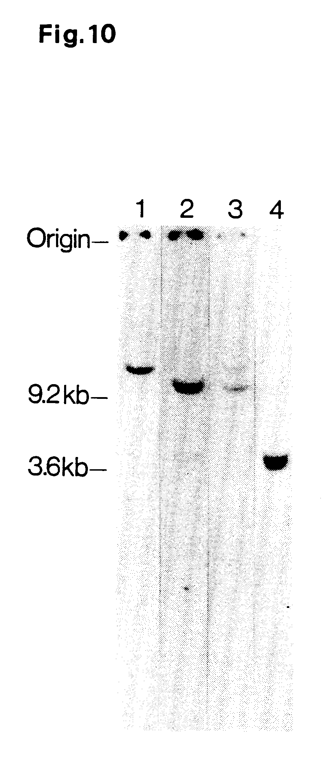

Fig. 10     Southern blot analysis of HindIII digested DNA from 70Z/3 cells. The pZ 183-1 gene is carried on a HindIII fragment in the genome of 70Z/3 cells which is different from the HindIII fragments carrying the cλ1 or the cλ2 sequences. The following probes were used for hybridization: pZ 183-1 (lane 1), cλ1 sequence on the XhoI-PstI fragment of pAB1-1 (Bothwell et al., supra) (lane 2), pZ 183-1 and cλ1 (lane 3) and cλ2 sequence on the 450 bp SacI fragment described in Weiss et al., Eur. J. Immun. 15, 765-768 [1985] (lane 4).

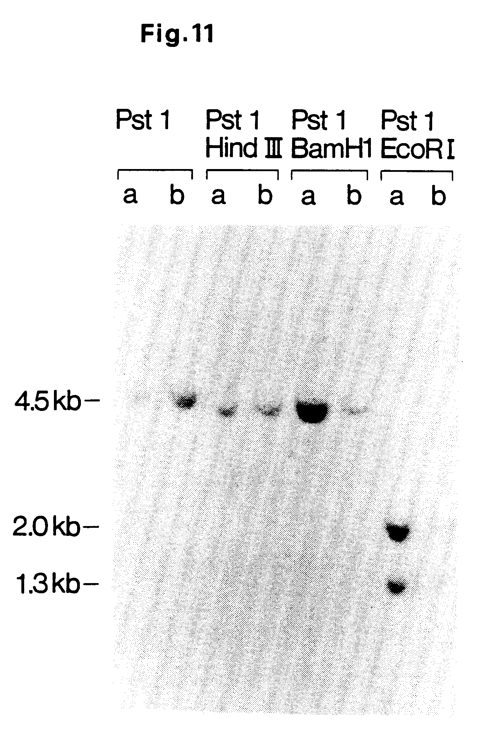

Fig. 11     Southern blot analyses of DNA from liver of (C57BL/6J x DBA/2) $F_1$ mice (a) and from 70Z/3 pre-B cells (b), showing that the gene is cut by PstI and EcoRI. DNA was digested with the indicated enzymes. The filter was hybridized with $^{32}$P-labelled insert DNA of cDNA clone pZ 183-1.

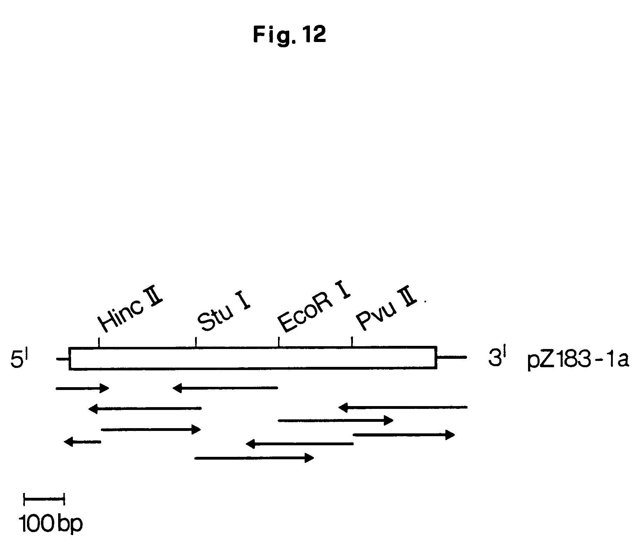

Fig. 12     Strategy for sequencing the cDNA clone pZ 183-1a. The arrows indicate the direction and the length of sequence determined by subcloning the indicated fragments into M13.

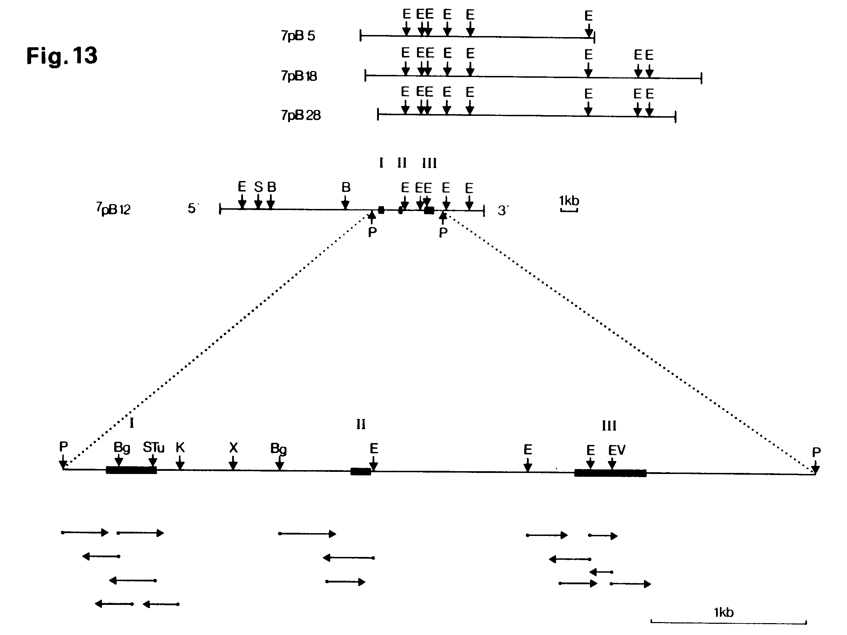

Fig. 13     The restriction maps of four overlapping λ5 genomic clones, 7pB5, 7pB12, 7pB18 and 7pB28 are shown. The 4.7 kbp PstI fragment of 7pB12 was subcloned into pUC-18 and a more detailed restriction map was determined.

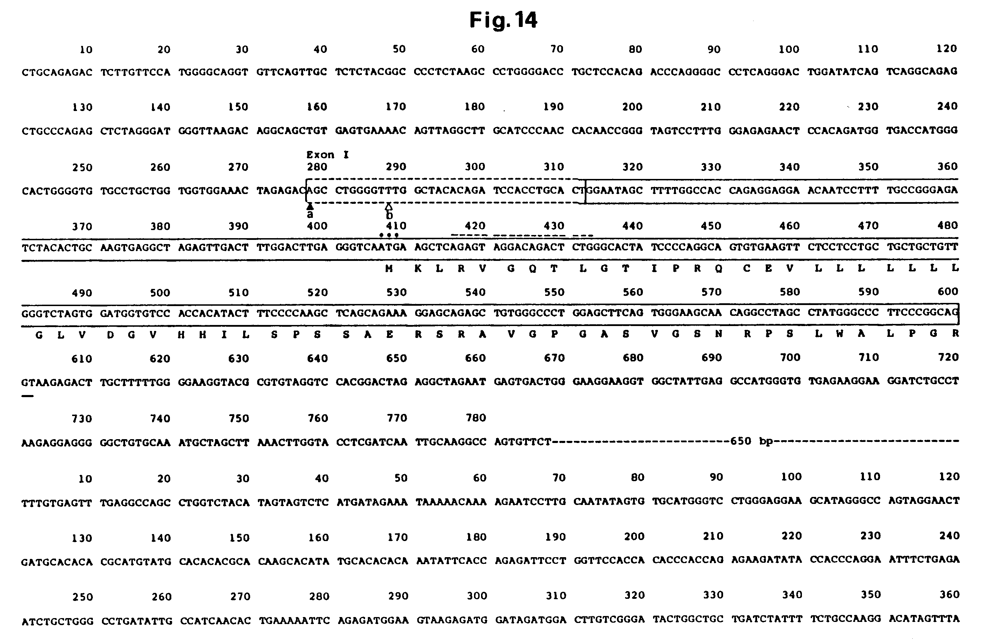

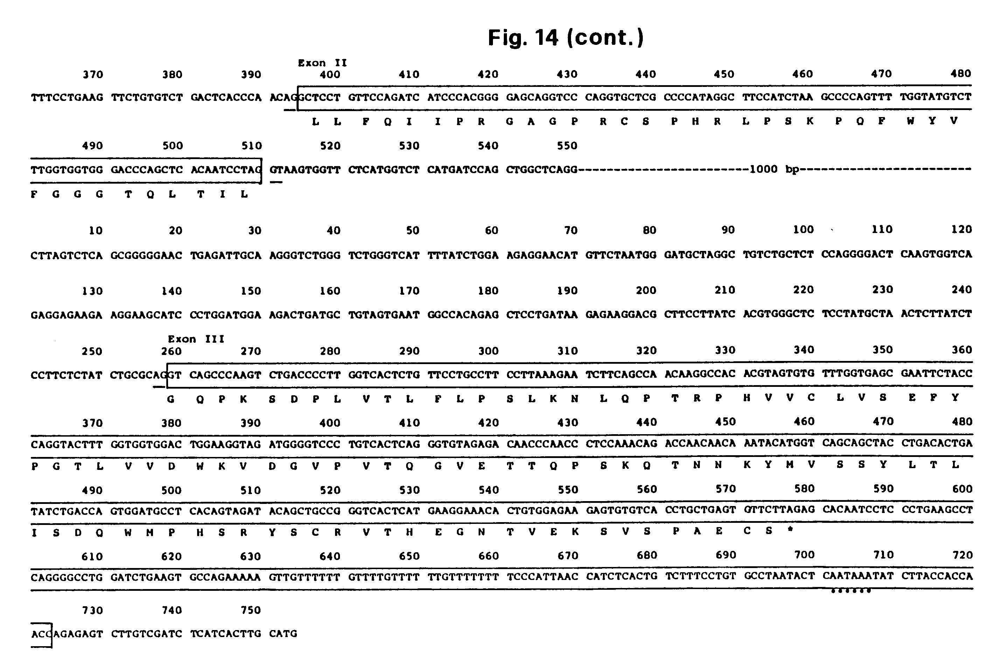

   Restriction sites are B = BamHI; Bg = BglII; P = PstI; E = EcoRI; EV = EcoRV; K = KpnI; S = SalI; STu = StuI; X = XbaI. The fragments indiated by arrows were sequenced by the M13 dideoxy chain termination method. The positions of the three exons in the λ5 gene (see Figure 14) are indicated with I, II and III.

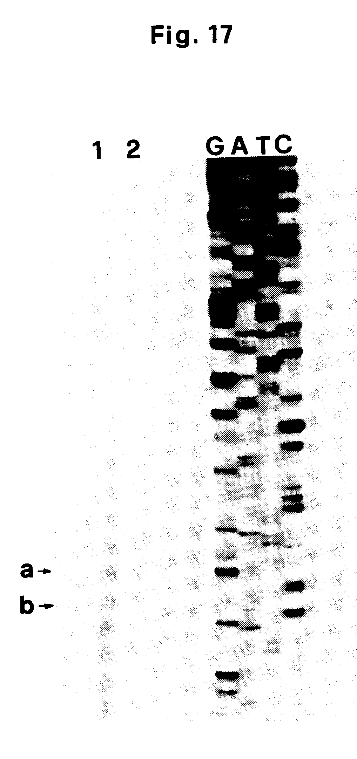

Fig. 14     Nucleotide sequence of the genomic form of λ5 containing all cDNA sequences and the amino acid sequences deduced from them. cDNA sequences of the pZ183-1a clone are boxed. The sequences are divided into three parts which show the three exons and genomic sequences 5′ and 3′ adjacent to them. a and b indicate the major sites of initiation of transcription determined by the primer extension experiment shown in Figure 17. The 18 nucleotides synthesized as a primer for the extension method are indicated with a broken line over the sequence. Sequences boxed in broken lines indicate the 5′ part of exon I determined by this primer extension method (see Fig. 17). GT (donor) and AG (acceptor) splicing signal sequences of introns are underlined. The triplet indicated by three closed circles (●) above the sequence shows the possible translation start codon ATG. The sequence underlined by six circles shows the poly (A) addition signal sequence.

Fig. 15

a) Homologies of λ5 genomic sequences with genomic λL-chain sequences. The homologies are listed in detail in Table III. In general, homologies of λ5 to λL chain sequences appear higher in exons than in introns.

b) Sequence homologies between areas in and around exon II of λ5 (7pB12), and areas in and around the exon for Jλ2. Genomic sequences in and around exon II of 7pB12 were aligned with those in and around the exon for Jλ2, allowing one deletion in the sequence of 7pB12, and two in Jλ2. Dashes indicate identity of nucleotide for the two sequences. Exons are boxed. Recombination signal heptamer and nonamer sequences (Sakano, et al., Nature, 280, 288-294, [1979]) adjacent to the exon of Jλ2 are underlined.

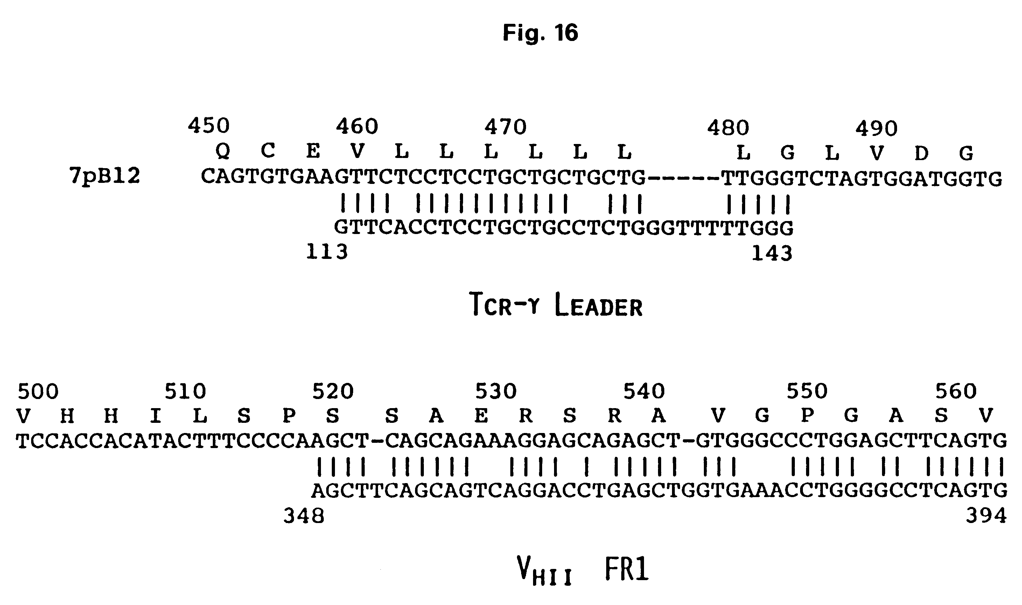

Fig. 16  Comparison of the nucleotide sequence of Exon I of the λ5 gene with those of the murine T cell receptor γ chain and the immunoglobulin heavy chain $V_{H11}$ segment. Dashes show identity of nucleotide. The sequences between position 448 and 562 in Exon I or 7pB12 are compared with murine T cell receptor γ chain leader sequences (TCR-γ leader, site: DFL12 113-143; Kranz, et al., Nature 313, 752-755, [1985]) and murine immunoglobulin heavy chain $V_{HII}$ framework 1 region ($V_{HII}$ FR1, site; $V_H$108 348-394; Givol, et al., Nature 292, 426-430, [1981]) by computer analysis (GenBank™, Los Alamos National Laboratory, Los Alamos, U.S.A., release 35.0, [1985]).

Fig. 17  Primer extension analysis of the 5′ end of λ5 mRNA. The synthetic oligonucleotide 5′-CAGAGTCTGTCCTACTCT-3′ complementary to a 18 nucleotide sequence in Exon I (see Fig. 14, position 416-433) was labeled and hybridized to pre-B cell line 40E-1 poly A containing RNA (lane 1) and yeast t-RNA (lane 2).

Sequencing of the 5′ region of the λ5 gene using the synthetic oligonucleotide was carried out to determine the transcription start site. The sequencing pattern shown for the comparison is that of the opposite strand of 7pB12 whose sequence is shown in Fig. 14.

Two major transcription start sites were found. The site located more 5′ contains an A (indicated by a), while the one located more 3′ does not (indicated by b).

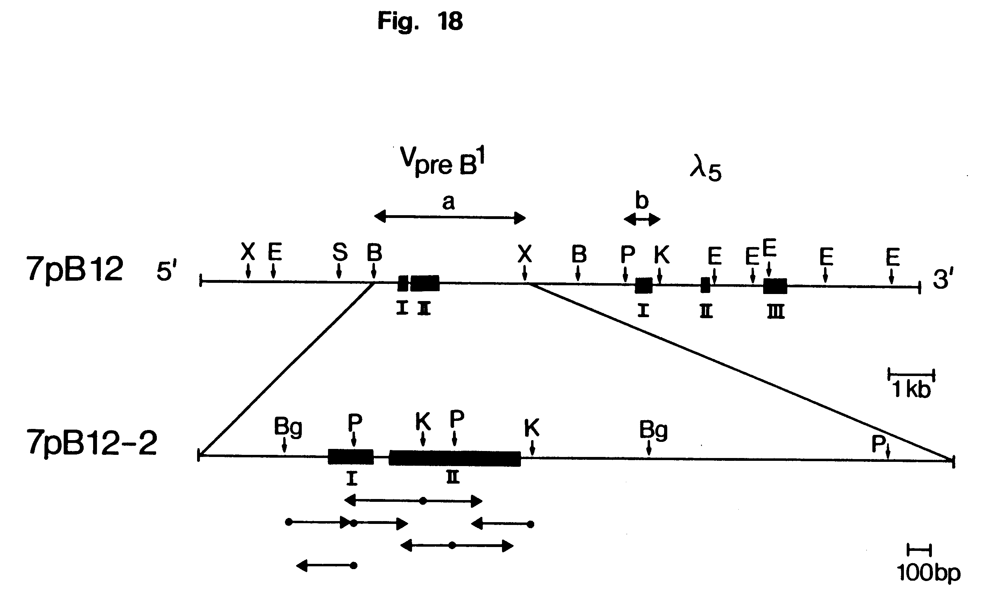

Fig. 18  Restriction enzyme map of the subclone 7pB12-2 containing a BamHI-XbaI fragment of the genomic clone 7pB12 cloned into pUC18. The arrows indicate the length of the fragments used for subcloning into M13 and the direction in which the given fragment was sequenced. The abbreviation for the restriction enzymes are the same as in Fig. 13. The positions of the two exons in the $V_{preB}1$ gene are indicated by I and II.

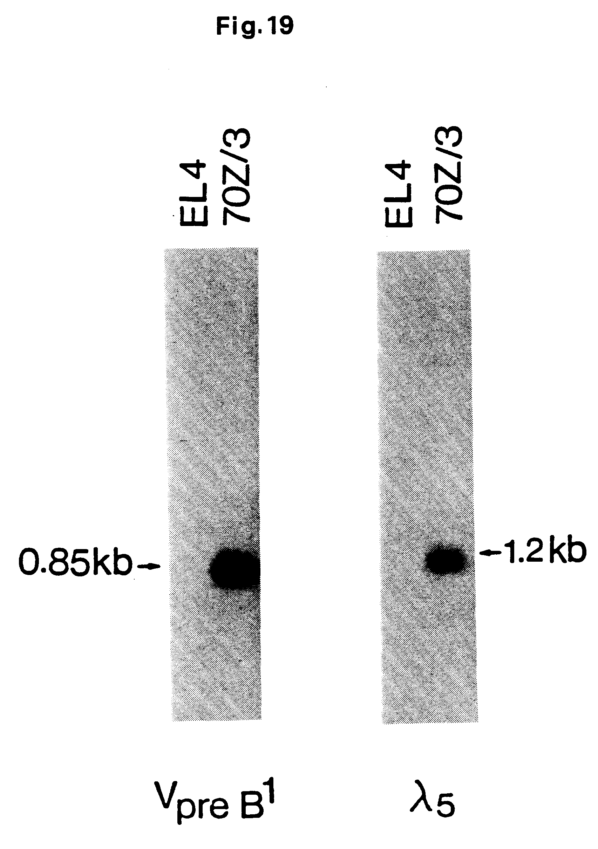

Fig. 19  Northern analysis (Rave, et al., Nucleic Acids Res. 6, 3559-3567, [1979]) of 10 μg total RNA from 70Z/3 pre-B lymphoma and from EL4 thymic lymphoma cells probed with the BamHI-XbaI fragment of 7pB12 (see Figure 18, subclone a) and with the λ5 KpnI-PstI fragment (see Figure 18, subclone b). The same filter was hybridized first with the oligolabelled pZ 121 in 50% formamide at 39°C, washed with 0.1 x SSC, 0.1% SDS at 50°C, exposed to X-ray film (Kodak XS-1) then the radiolabelled probe was washed off at 0.1 x SSC, 0.1% SDS at 95°C, and thereafter exposed to the second oligolabelled probe, pZ 183-1, again washed in 0.1 x SSC, 0.1% SDS at 50°C, and again exposed to X-ray film.

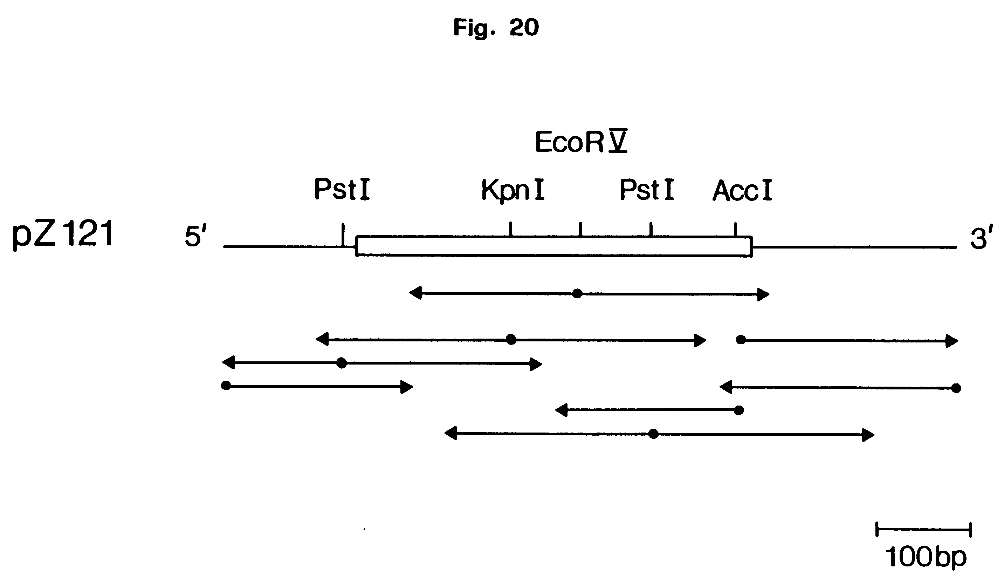

Fig. 20  Restriction enzyme map of the pZ 121 cDNA clone. The arrows indicate the length of the fragments used for subcloning into M13 and the direction in which the given fragment was sequenced.

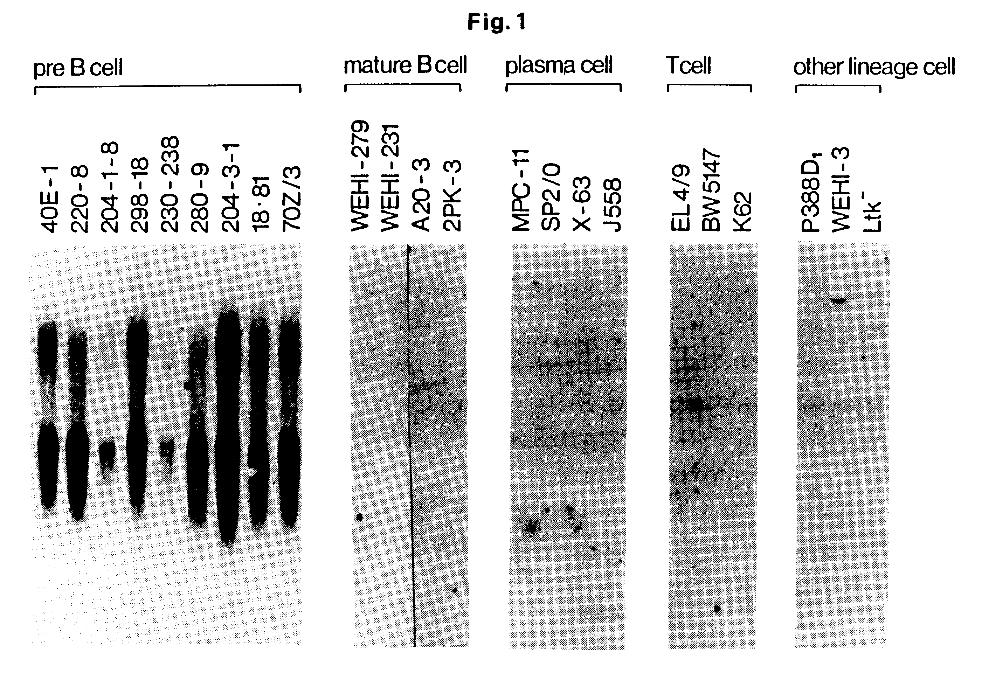

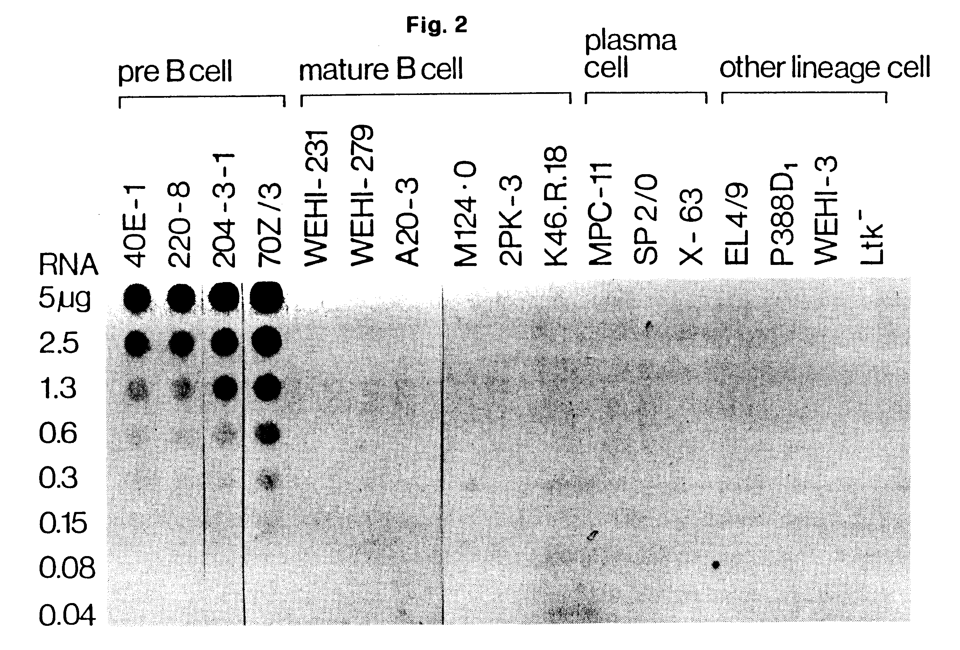

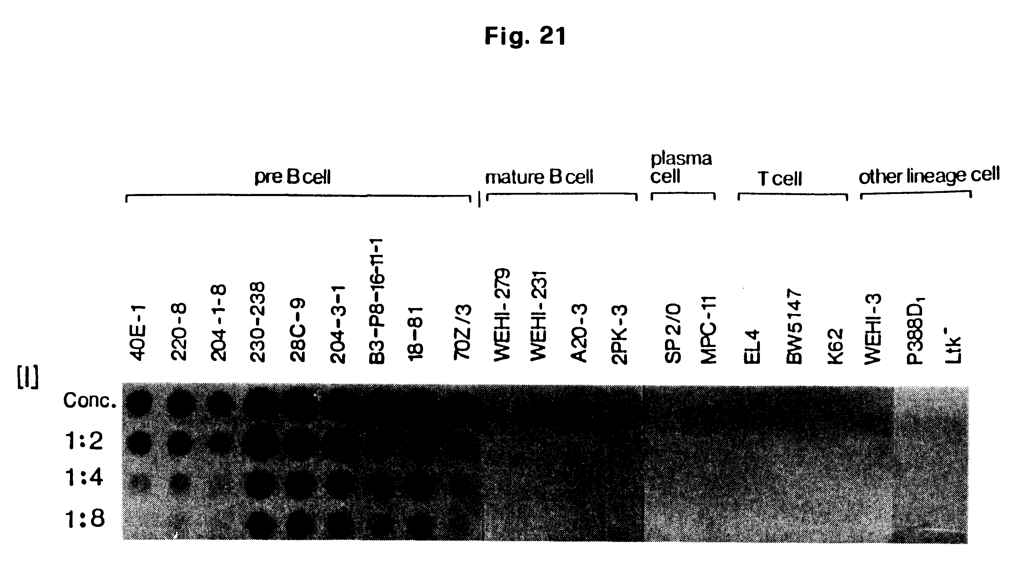

Fig. 21  Quantitative Northern dot blot analysis of pZ 121 ($V_{preB}1$) transcripts in different cell lines. Serial two fold dilutions were blotted on nitrocellulose filters for hybridization with the oligolabelled EcoRI-AccI fragment of pZ 121. The cell lines used to show the pre-B cell specificity have already been used before (see Figure 1 and 2 and Table I) with the exception of B3-P8-16-11-1 which is an Abelson virus transformed pre-B cell and which was obtained as described below. Prehybridization and hybridization were performed with 1.5 x SSPE, 1.0% SDS, 0.5% nonfat powdered milk, 0.5 mg/ml carrier salmon sperm DNA. Filters were washed finally with 0.1 x SSC, 0.1% SDS at 60°C. Filters were probed with radiolabelled pZ 121 cDNA. "Conc." indicates RNA amounts between 1 and 5 μg per dot.

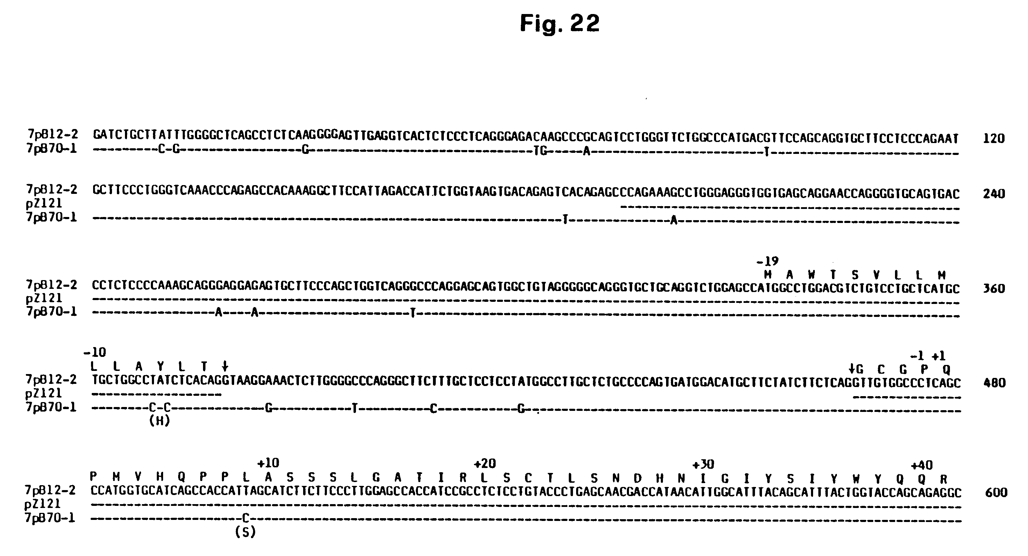

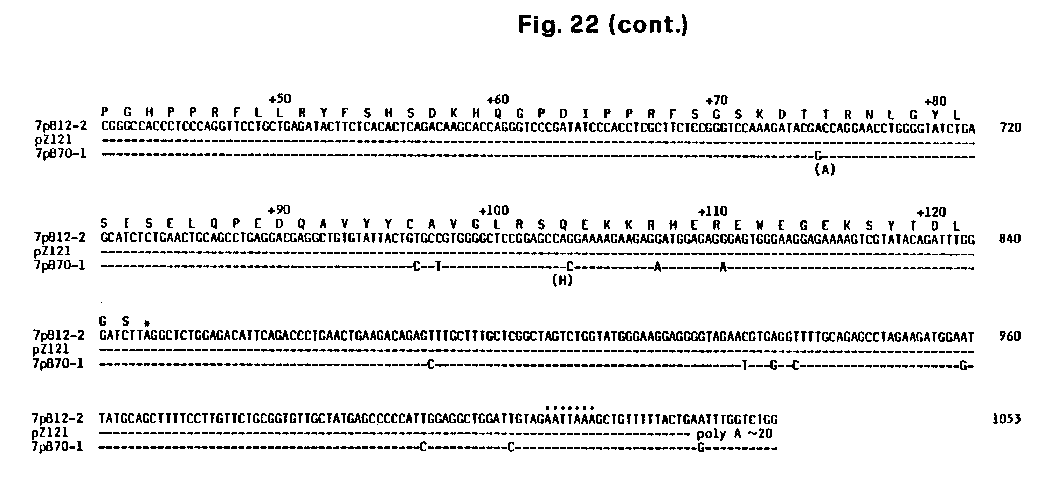

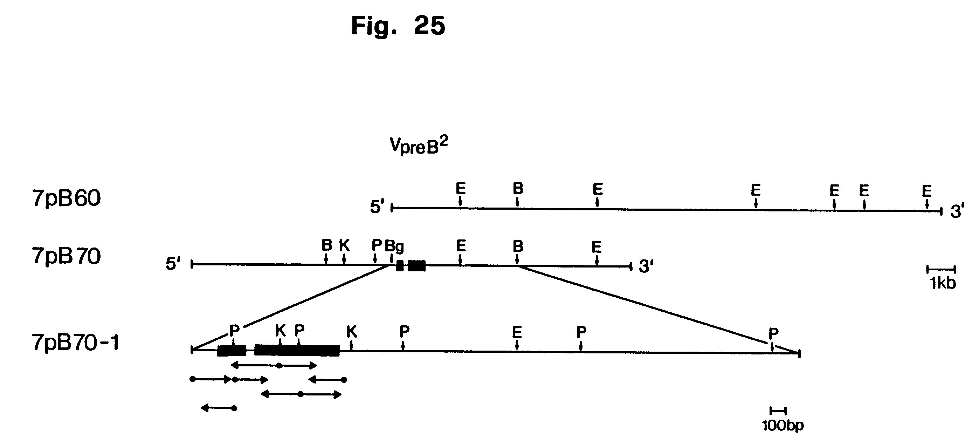

Fig. 22    Nucleotide sequence and deduced amino acid sequence of the $V_{preB}$1 and $V_{preB}$2 gene. For $V_{preB}$1 nucleotide sequences of both the genomic form (7pB12-2) as well as of the cDNA (pZ121) are given. The cDNA sequences are identical with the genomic sequences and are, therefore, only indicated by dashes (-) and follows the genomic sequence in numbering. Numbering of amino acid residues starts with -19 as the first position of the leader and proceeds to +1 as the first position of the mature protein. The sequence marked by closed circles (......) shows the poly A addition signal sequence. Arrows (↓) indicate potential splice sites. The asterisk (*) points to the termination codon TAG. DNA sequencing was carried out using the dideoxy chain termination method by subcloning of fragments into M13mp18 and M13mp19 vectors, using a 17-mer universal M13 primer (Amersham). The $V_{preB}$2 nucleotide sequence of the genomic form (7pB70-1) is given. (For a restriction map of 7pB701, see Fig. 25). $V_{preB}$2 sequences identical to $V_{preB}$1 sequences are indicated by dashes (-). Wherever the deduced amino acid sequence of $V_{preB}$2 differs from that of $V_{preB}$1 the changed amino acid is given in brackets ( ).

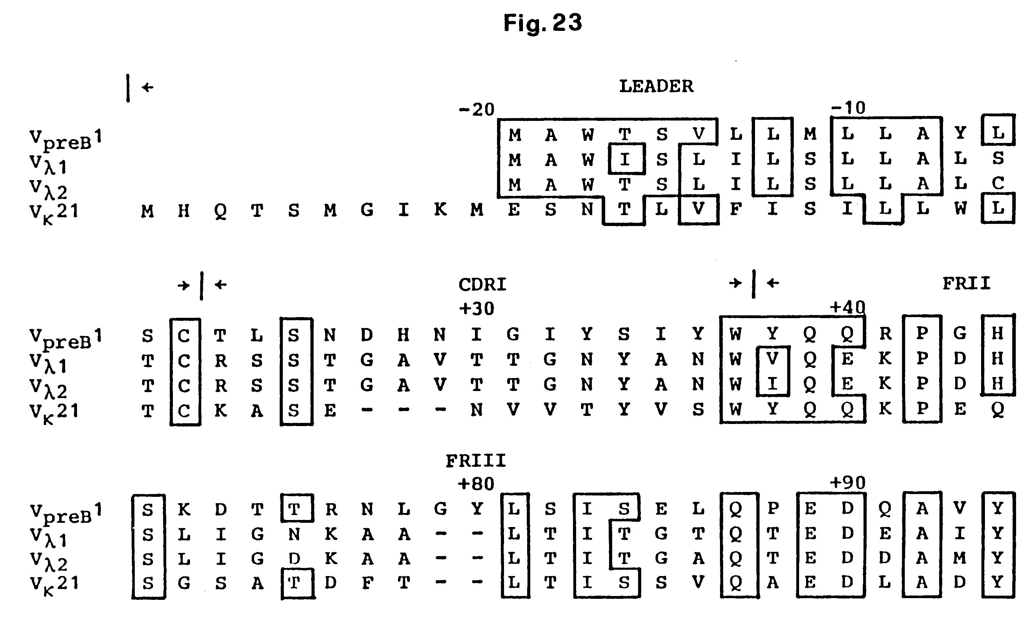

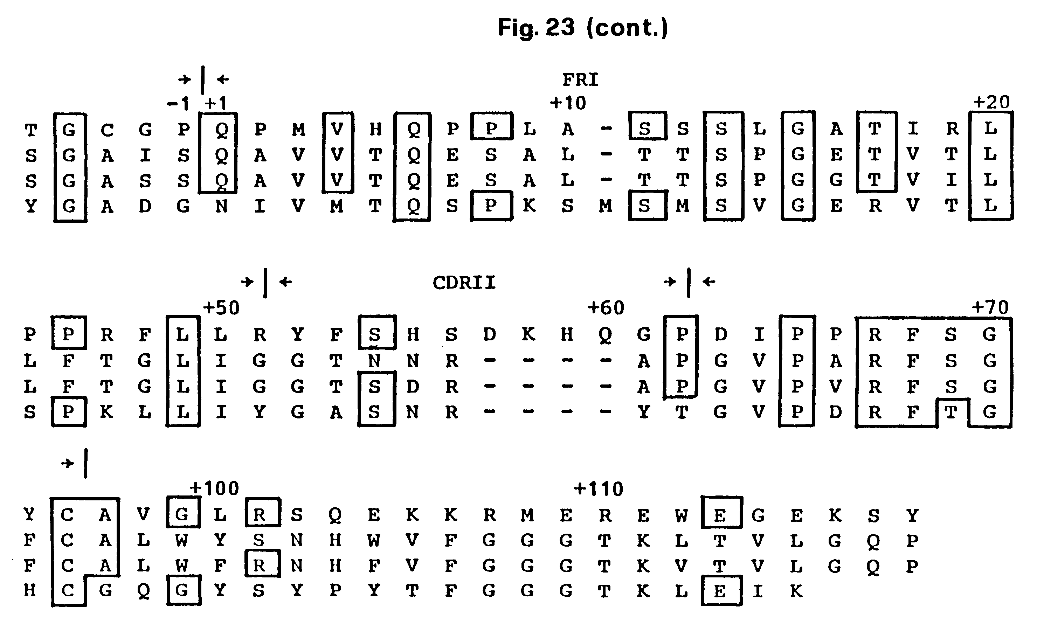

Fig. 23    Alignment of the deduced amino acid sequence of $V_{preB}$1 with sequences of $V_{\lambda 1}$, $V_{\lambda 2}$ - (Tonegawa, et al., Proc. Natl. Acad. Sci. USA, 75 1485-1489, [1978]) and $V_x$21 (Fong, et al., Biochem. Biophys. Res. Commun. 90, 832-841, [1979] and Hamlyn, et al., Nucl. Acids, Res. 9, 4485-4494, [1981]). Those residues homologous to $V_{preB}$1 and any of the other sequences are boxed. The numbering of positions follows that given for $V_{preB}$1 in Fig. 22. Leader, CDR I and II and FR I, II and III indicate the locations of the leader, the complementarity-determining and of framework residues found in variable response of L chain (Kabat, et al., supra).

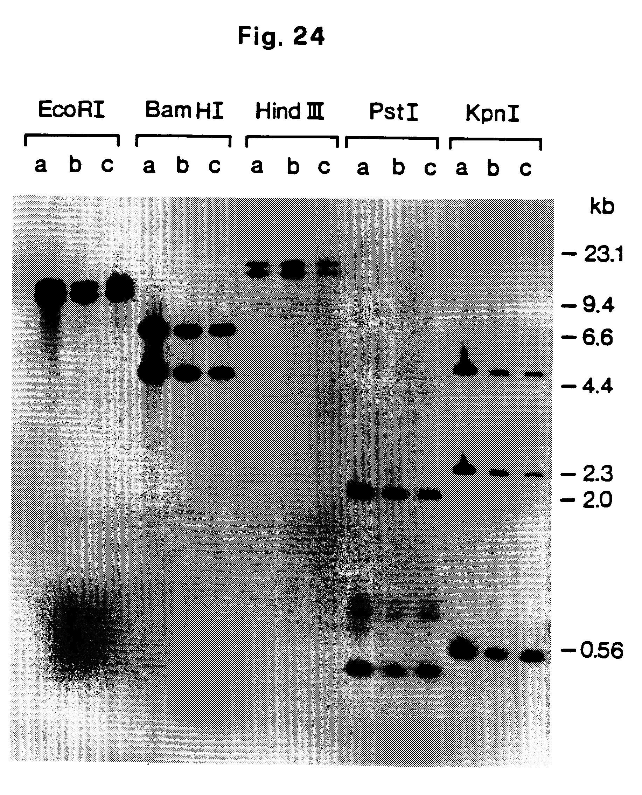

Fig. 24    Southern blot analyses of DNA from DBA/2 liver (a), C57BL/6 liver (b) and from the 70Z/3 (DBA/2 x C57BL/6) pre-B lymphoma cell line (c). DNA was digested with the indicated enzymes. 10 μg of DNA fragments were separated by electrophoresis on agarose gels and transferred to the Zeta probe membrane (BioRad) using 0.4N NaOH as transfer buffer (Reed, et al., Nucl. Acids Res. 13, 7207-7221, [1985]). After transfer, the filter was rinsed twice in 2 x SSC and dried at 80°C under vacuum. Prehybridization and hybridization were carried out with 1.5 x SSPE (0.27 M NaCl, 0.015 M sodium phosphate, 0.015 M EDTA), 1.0% SDS, 0.5% nonfat powdered milk, 0.5 mg/ml carrier salmon sperm DNA and 10% dextran sulfate at 65°C by using $^{32}$P-oligo labelled insert DNA of cDNA clone pZ 121 as a probe. The filter was washed finally with 0.1 x SSC, 0.1% SDS at 65°C.

Fig. 25    Restriction map and sequencing strategy of $V_{preB}$2 genomic clones. The restriction maps of two overlapping $V_{preB}$2 genomic clones, 7pB60 and 7pB70 are shown. The 4.4 kb BglII-BamHI fragment (7pB70-1) of 7pB70 was subcloned into pUC-18 and a more detailed restriction map was determined. Restriction sites are abbreviated as in Fig. 13. The sequence is shown in Fig. 22.

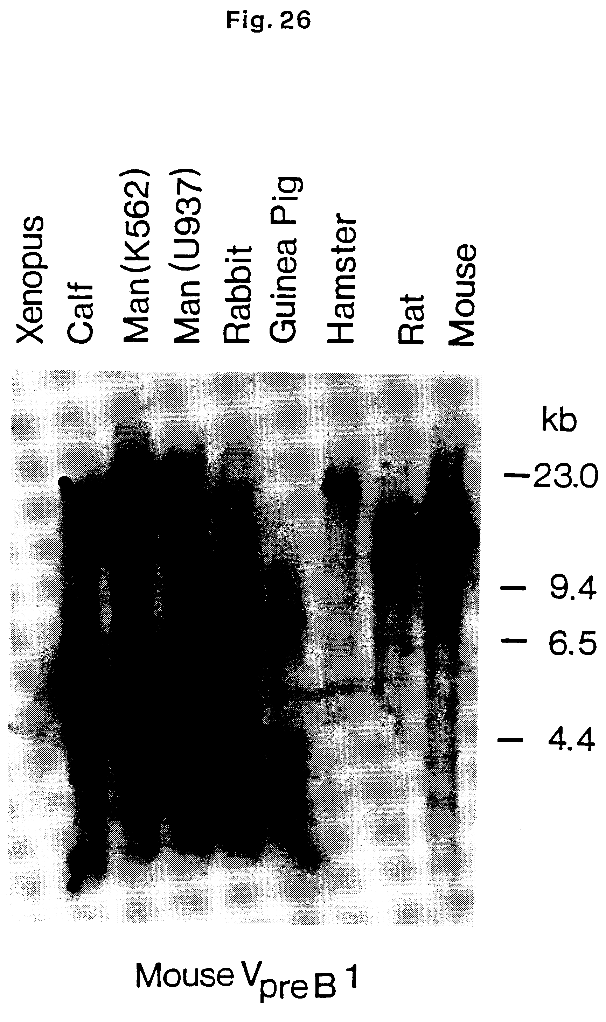

Fig. 26    Southern blot analysis of EcoRI digested DNA from different species.
            7 μg digested DNA from liver of rat, hamster, mouse, guinea pig and rabbit, from red blood cells of frog, from calf thymus, and from human cell lines were electrophoretically separated, blotted onto nitrocellulose and hybridized with a $^{32}$P-labelled mouse VpreB1 probe.

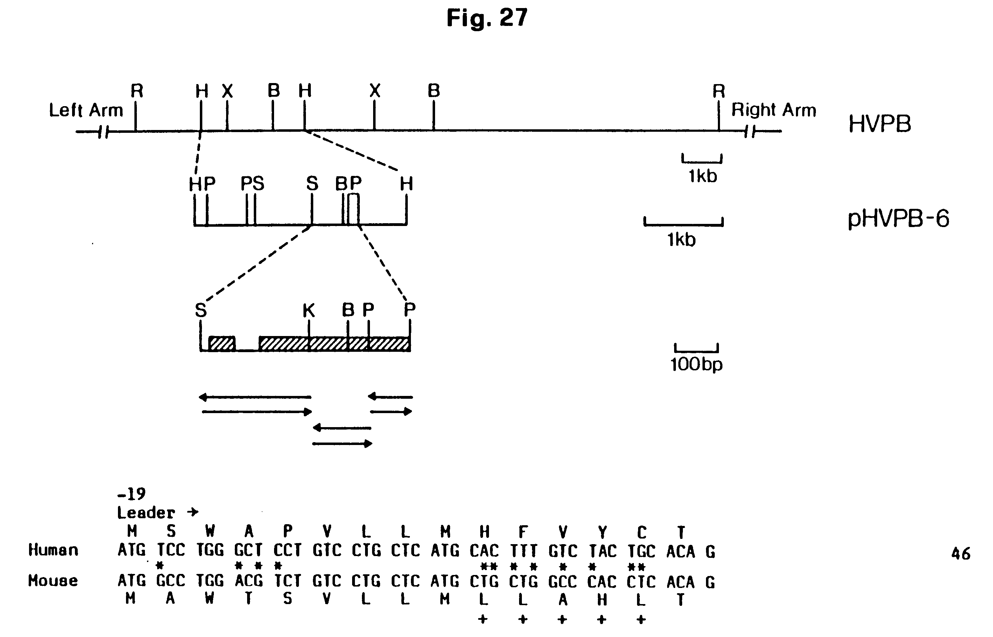

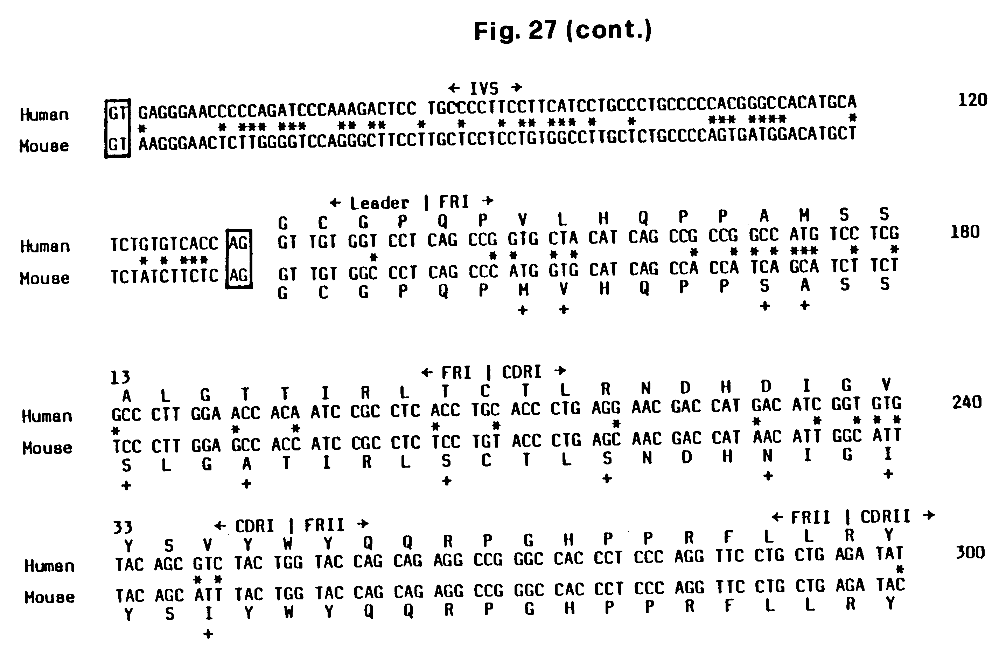

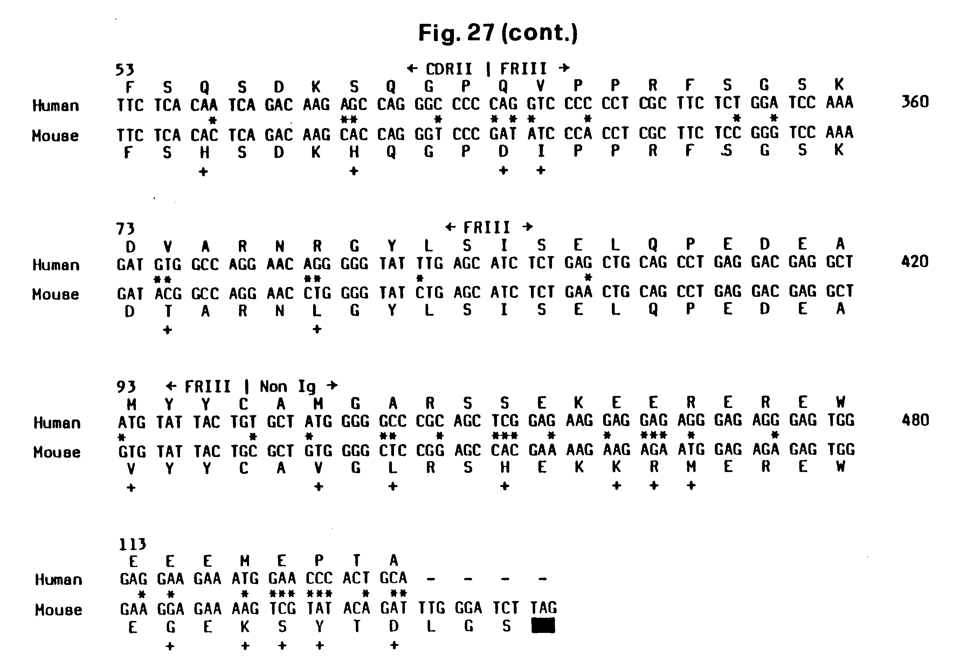

Fig. 27    Restriction map and sequencing strategy for HVPB phage clone and plasmid subclone pHVPB-6 and nucleotide sequence comparison of human VpreB and mouse VpreB1 genes with their deduced amino acid sequences. Restriction enzyme sites are symbolized as: R = EcoRI; B = BamHI; P = PstI; K = KpnI; S = SacI; X = XbaI. Exons I and II are shown as rectangles enclosing slanted lines with the 5′ end at the left. Arrows show the direction and length of DNA sequences derived from indicated restriction fragments subcloned into M13. Putative exon structures are based on comparison with the sequence of mouse VpreB1 cDNA. Potential splice sites are enclosed in boxes. An asterik (*) indicates nucleotide differences while a plus(+) below the mouse amino acid residues indicates a putative protein difference. A black rectangle indicates the mouse termination codon. Dashes (-) indicate human residues not yet determined. Numbering of deduced amino acid residues starts with -19 as the first position of the leader and proceeds from +1 as the first position of the putative mature protein (top left of each row). Nucleotide residues (right of each sequence row) begin with the A of the initiation codon. Note the 1 base gap in the IVS of the human sequence. Leader, CDRI and II and FRI, II and III indicate the locations of the leader, complementarity determining region, and framework regions typically found in immunoglobulin light chain variable regions (Kabat et al., supra) and following the alignment with V region genes of $V_{preB}1$ and $V_{preB}1$.

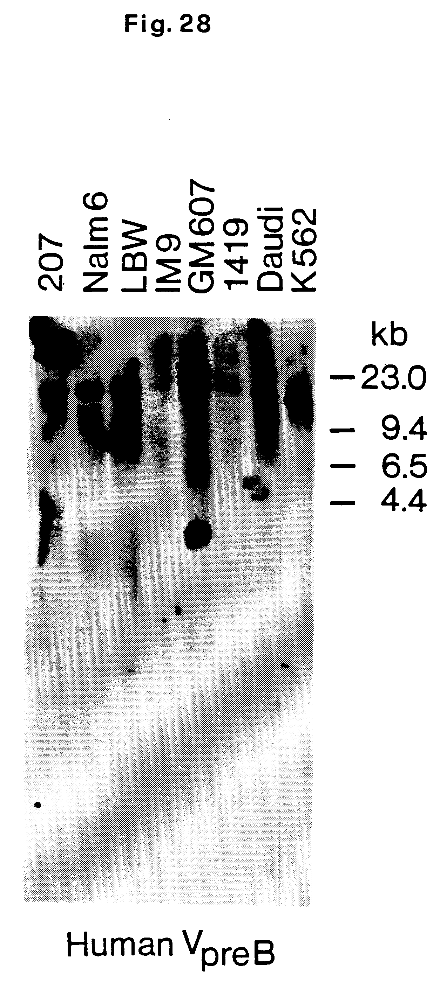

Fig. 28 Southern blot analysis of EcoRI digested DNA from human cell lines.

7 µg digested DNA was applied to a 0.8% agarose gel, electrophoretically separated, then transferred onto nitrocellulose filter. The filter was hybridized with a $^{32}$P-labelled 1.2 kb PstI fragment of pHVPB-6, a 2.7 kb HindIII pUC18 subclone of phage clone HVPB. Washing of the filters was done as described below for the Southern blot analysis shown in Fig. 26. Sizes of hybridizing bands were calculated using HindIII digested phage λ DNA as standards.

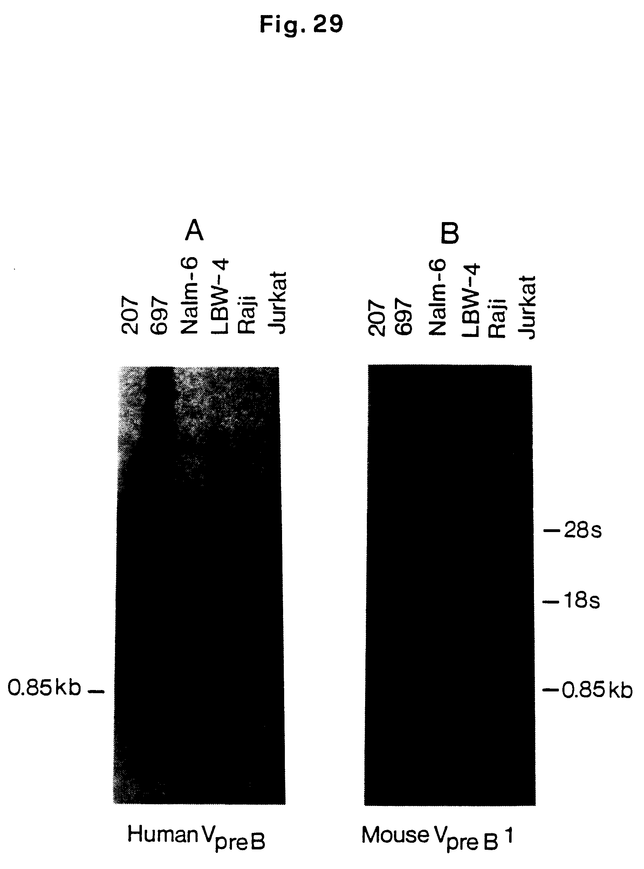

Fig. 29 Northern blot analysis of poly(A)-selected RNA from lymphoid cells.

5 µg poly(A)[+] RNA was applied to each lane, electrophoresed and blotted onto activated DPT paper. Identical filters were probed with: (A) a $^{32}$P-labelled 1.2 kb PstI fragment of pHVPB-6 or (B) a $^{32}$P-labelled 560 bp EcoRI-AccI fragment from pZ121, a mouse $V_{preB}1$ cDNA clone. The filter in panel (A) was washed finally in 0.2 x SSC, 0.1% SDS at 65°C, then exposed to x-ray film overnight at -80°C with intensifying screens. The filter in panel (B) was washed finally in 0.2 x SSC, 0.1% SDS at 37°C and exposed as described above. Sizes of hybridizing bands were calculated using RNA molecular weight standards purchased from BRL (Bethesda, MD).

The nucleotide sequences which are selectively expressed in pre-B cells may be identified by subtraction hybridization, that means by a method in which nucleotide sequences which are expressed in pre-B cells and in other cells are eliminated and only those sequences are selected which are solely expressed in pre-B cells. More specifically the nucleotide sequence is identified by preparing a cDNA library from poly A containing RNA from a pre-B cell and selecting from that library cDNA clones which hybridize to polysomal poly A containing RNA from a pre-B cell and not to polysomal poly A containing RNA from a different cell, that means a cell which is not a pre-B cell. Alternatively a nucleotide sequence which is selectively expressed in pre-B cells may be identified by screening a cDNA library with a homologous nucleotide sequence from another species, which nucleotide sequence has been identified by subtraction hybridization as described above. Suitable cDNA libraries are described in the example. Homologous nucleotide sequences are nucleotide sequences with the same function but originating from different species and with practically an identical nucleotide sequence.

For identifiying a first nucleotide sequence selectively expressed in pre-B cells mRNA, preferably microsome-bound polysomal poly A containing RNA can be isolated from pre-B cells by methods known in the art (e.g. Maniatis, et al., supra, pp. 188-209) or as described in the Example. Since it is difficult to isolate a sufficient number of such cells from a mammalian organism especially from a human organism, a cell line derived from such a subset of the lymphoid cell population may be chosen. Pre-B cell lines may be easily prepared as described by Rosenberg, et al., Proc. Natl. Acad. Sci. USA 72, 1932-1936, [1975]. The pre-B cell line used in the present invention is the pre-B lymphoma cell line 70Z/3. This cell line is derived from a chemically induced pre-B cell tumor (Paige, et al., J. Immunol. 121, 641-647, [1978]). The cell line has been shown to express µ-heavy (H) chain, but no light (L) chain and, therefore, was identified as a surface Ig-negative pre-B cell (Paige, et al., supra; Sakaguchi, et al., J. Immunol. 125, 2654-2659, [1980]; Perry, et al., Cell 18, 1333-1339, [1979]). A cDNA library, that means a collection of DNA's complementary to the poly A containing RNA from pre-B cells can be prepared by methods known in the art (e.g. Maniatis, et al., supra, pp. 211-246) or as described in the Example. Repeated subtraction hybridization using polysomal poly A containing RNA from a pre-B cell and from a cell which is not a pre-B cell is used to isolate a cDNA clone comprising a nucleotide sequence which is selectively expressed in pre-B cells. Suitable cells which are not pre-B cells are those distinctly different from cells of the early stages of the B cell lineage but on the other hand related to the latter cells, so that they both express a similar subset of genes. Examples of such cells are lymphoid cells e.g. T cells, preferably a T cell hybridoma. In the present invention the T cell hybridoma K62 was used. This T cell line was obtained in the fusion of the thymic lymphoma BW 5147 with an allo-reactive, H-2[k]-specific helper T cell clone. It is within the scope of the invention to use other T cell lines or any other suitable cell which is not a pre-B cell.

In a preferred embodiment of the present invention the hybridization reactions may be performed at 68°C for 16-20 hours in order to achieve a Cot of about 2000 (Britten, et al., Science, 161, 529-540, [1968]). The nonhybridized fraction of cDNA molecules can then be used to construct a phage library according to the method described by Davis, et al., Proc. Natl. Acad. Sci. U.S.A. 81, 2194-2198, [1984]. For a first screening this selected cDNA library may then be tested either with radioactive pre-B cDNA or with radioactive K62 T cell hybridoma cDNA. In the present invention the frequency of pre-B cell-specific cDNA clones in the T cell cDNA-subtracted library was found to be 14 in 5'000, while there were 10 in 50'000 in the unsubtracted, original 70Z/3 pre-B cDNA library. Clones positive with only the pre-B cDNA but not with the K62 T cell cDNA can be selected and used for a second screening. For that purpose the selected clones are individually grown up. The phage DNA containing the pre-B cell-specific sequences is isolated and after radiolabeling used for hybridization with a panel of RNA preparations from different cell lines of different differentiation lineages at different stages of differentiation. In the present invention clone pZ 183 was selected from two hundred 70Z/3 specific cDNA clones. The clone contained a 380 nucleotide pair long insert of 70Z/2 cDNA. The selected nucleotide sequence was found to be expressed 1.5 times more than μ-heavy chain-specific mRNA in the 70Z/3 tumor cells. This level of expression is in the range of medium abundance and, thus, is estimated to represent 100-300 mRNA copies per cell (Hastie, et al., Cell 9, 761-774, [1976]). Mature B cells, plasma cells, T cells, macrophages and a variety of cells from other lineages and tissues were found not to express the corresponding gene. The selected 70Z/3 cDNA sequences or fragments thereof can therefore be used as a probe for the detection of transcripts of genes which are selectively expressed in pre-B cells that means for the determination of pre-B cells in a diagnostic test. Such a probe (= negative strand probe) comprises a polynucleotide hybridizing specifically to a transcript wich is selectively expressed in pre-B cells. Said polynucleotide is preferably single stranded and has a nucleotide sequence substantially complementary to that of said transcript. The length of said complementary nucleotide sequence determines the specificity of the probe. A length of at least about 20 nucleotides is in most cases required for a high enough specificity. Said polynucleotide may be in linear or circular from and may be comprised of two or more individual segments interrupted by non complementary sequences. These non-hybridizable sequences can be linear, or they can be self-complementary and form hairpin loops. In addition, the complementary region of the probe can be flanked at the 3′- and the 5′-termini by non hybridizable sequences, such as those comprising the DNA or RNA of a vector into which the complementary nucleotide sequence had been inserted for amplification. A polynucleotide having the same nucleotide sequence as the transcript (= positive strand probe) may be used as negative control.

Large amounts of the polynucleotide used for the probe can be obtained by chemical synthesis using conventional polynucleotide synthesis procedures preferably on a solid phase by the triester or the phosphite method (for a review see e.g. Kiefer, et al., Immunol. Methods 3, 69-83, [1985]) or by cloning said polynucleotide into a suitable replicable microbial vector, transforming said vector into a suitable host and culturing the host under conditions permitting production of multiple copies of the cloned polynucleotide. Suitable vector/host-systems are described in the Example. Preferably the bacteriophage M13 vector system (Messing, Methods in Enzymol. 101, 20-78, [1983] is used since in that system directly single stranded polynucleotides can be prepared. Alternatively large amounts of the polynucleotide used for the probe can be obtained by inserting a complementary polynucleotide into a vector containing a very active transcription promoter e.g. the Salmonella typhimurium bacteriophage SP6 transcription promoter (Green, et al., Cell 32, 681-694, [1983]) and producing multiple RNA copies which can be used for the probe. The polynucleotide may contain modified nucleotides.

For use in a diagnostic test a suitable label has to be incorporated into the probe. Suitable labels are e.g. radioactive labels, fluorescent labels, enzyme labels and other labels generally known in the art. Such labels may be linked covalently or noncovalently to the polynucleotide or to the hybrid formed after contact of the polynucleotide with a transcript which is selectively expressed in pre-B cells. Typical labels and methods which may be used in a diagnostic test to determine whether a cell is a pre-B cell or not are described e.g. in the following European Patent Applications with the Publication Nos. 139 489 (published May 2, 1985), 144 913 (published June 19, 1985), 144 914 (published June 19, 1985), 146 039 (published June 26, 1985), 146 815 (published July 3, 1985), 149 339 (published July 24, 1985) and 163 220 (published December 12, 1985). Such a diagnostic test may be useful for the classification of leukemias which may guide therapeutic decisions (Foon, et al., Blood 68, 1-31, [1986]). Further diagnostic test methods are described in the Example (e.g. "in situ" hybridization, Northern-blot).

In the present invention the selected 70Z/3 cDNA sequence hybridized specifically to a 1.2 kb size transcript present in a variety of pre-B cells. No hybridization was found under the same conditions using poly A containing RNA from mature B cells, plasma cells, T cells and other cells which are not from the B cell lineage. The selected 70Z/3 cDNA sequence is 380 nucleotide pairs long. It represents therefore only a partial cDNA of the 1,2 kb long transcript of the gene selectively expressed in pre-B cells.

The selected 70Z/3 cDNA can be used to prepare a full-length cDNA of said pre-B cell transcript. Such a full-length cDNA can again be used as a probe as described above. Preferably a polynucleotide corresponding to a part of the full-length cDNA sequence is used as a probe. The polynucleotide used as a probe is selected in a way that it recognizes sequences or portions thereof which are unrelated to any other nucleotide sequence known to be expressed by B-cells. Such a selection can be done by comparing the sequence of the full-length 70Z/3 cDNA with sequences stored in a gene sequence data bank e.g. the GenBank™ (supra). The selected polynucleotide can then be amplified in a manner known in the art e.g. by cloning said polynucleotide into a replicable microbial vector. Suitable microbial vectors are described by Maniatis et al. (supra, pp. 1-54) and include plasmids and single- as well as double-stranded bacteriophages. After transformation of the replicable microbial vector into a suitable host organisms, said host can be cultured under conditions permitting production of multiple copies of said vector. Upon incorporation of a label, said vector or a part thereof containing the selected polynucleotide can be used as a probe as described above. Such a probe can also be used to isolate the genomic sequence λ5 encoding the transcript selectively expressed in pre-B cells. The full-length cDNA of the transcript selectively expressed in pre-B cells or fragment thereof can be used to prepare a recombinant polypeptide having an amino acid sequence encoded by said transcript. For this purpose the nucleotide sequence of the cloned cDNA is determined by methods known in the art (e.g. Sanger, et al., Proc. Natl. Acad. Sci. USA 74, 5463-5467, [1977]). The nucleotide sequence is then searched for initiation and termination signals. Any long open reading frame may represent a polypeptide which is selectively expressed in pre-B cells. It is known that extracellular polypeptides are synthesized in a precursor form comprising a signal peptide sequence. Specific peptidases cleave the precursor polypeptide at the signal peptidase recognition site (Perlman, et al., J. Mol. Biol. 167, 391-409, [1983]) to yield the mature polypeptide. Such a polypeptide or a fragment thereof may be produced by methods known in the art of recombinant DNA technology in that in a first step a DNA sequence coding for the desired polypeptide is brought under the control of a microbial expression control sequence contained on a replicable microbial vector. The resulting expression vector for said polypeptide is inserted into a suitable host organism capable of expressing said polypeptide. Said host organism may be cultured under conditions permitting production of large amounts of polypeptide. The recombinant polypeptide may then be isolated and purified by methods known in the art. Suitable expression control sequences are described in European Patent Application, Publication No. 186 069, published July 2, 1986. Those of skill in the art may select from these expression control sequences those that are most effective for expressing the desired polypeptide without departing from the scope of this invention.

The selection of a particular host for use in this invention is dependent upon a number of factors recognized by the art. These include, for example, compatibility with the chosen expression vector, toxicity of the polypeptide encoded by the hybrid plasmid, ease of recovery of the desired polypeptide, expression characteristics, biosafety and costs. Within these general guidelines, examples of useful bacterial hosts are gram-negative and gram-positive bacteria, especially strains of E. coli and B. subtilis.

Once the organisms capable of carrying out the expression of the polypeptides of the present invention have been prepared, the process of the invention can be carried out in a variety of ways, depending upon the nature of the construction of the expression vector and upon the growth characteristics of the host. Typically, the host organism will be grown under conditions which are favorable for the production of large quantities of cells. When a large number of cells have accumulated, suitable inducers or derepressors in the growth medium or a temperature-shift cause the control sequence supplied with such DNA sequence to become active, permitting the transcription and translation of the coding sequence. In the present invention the expression of the DNA fragment encoding the polypeptide is inhibited by the lac repressor. When a large number of cells have accumulated, the gene is derepressed by the addition of isopropyl-$\beta$-D-thiogalactopyranoside (IPTG). The polypeptide produced by the recombinant cell can be released by lysing the cell by conventional means well known in the art. The particular means of lysing will depend upon the host cell utilized.

Alternatively a polypeptide encoded by the nucleotide sequence which is selectively expressed in pre-B cells or preferably by fragments thereof can be synthesized by any known conventional procedure for the formation of a peptide linkage between amino acids. Such conventional procedure includes, for example, any solution phase procedure permitting a condensation reaction between the free alpha amino group of an amino acid or residue thereof having its carboxylic group or other reactive groups protected and the free primary carboxylic group of another amino acid or residue thereof having its amino group or other reactive groups protected.

The process for synthesizing the polypeptides may be carried out by a procedure whereby each amino acid in the desired sequence is added one at a time in succession to another amino acid or residue thereof or by a procedure whereby peptide fragments with the desired amino acid sequence are first synthesized conventionally and then condensed to provide the desired peptide.

Such conventional procedures for synthesizing the polypeptides of the present invention include for example any solid phase peptide synthesis method. In such a method the synthesis can be carried out by sequentially incorporating the desired amino acid residues one at a time into the growing peptide chain according to the general principles of solid phase methods (Merrifield, J. Am. Chem. Soc. 85, 2149-2154, [1963]; Barany, et al., The Peptides, Analysis, Synthesis and Biology, 2, Gross, E. and Meienhofer, J., Eds., Academic Press, 1-284, [1980]).

The polypeptide of the present invention can be purified by known methods, such as precipitation with ammonium sulfate, dialysis to remove salts (under normal or reduced pressure), gel filtration, preparative flat-bed iso-electric focusing, gel electrophoresis, various chromatographical methods e.g. high performance liquid chromatography ion exchange chromatography, reverse phase chromatography and affinity chromatography, (e.g., on dye bound carrier or on Sepharose coupled with monoclonal antibodies against said polypeptide) and the like.

The polypeptide of the present invention may form multimers e.g. dimers, trimers or tetramers or may be present as a fusion polypeptide. Such a fusion polypeptide may be obtained by linking the above mentioned DNA fragment with a DNA sequence selected from a large variety of DNA sequences that encode procaryotic or eucaryotic polypeptides. After expression of the fusion polypeptide encoded by the combined DNA sequences in a suitable host, the fusion polypeptide may be purified by affinity chromatography using a ligand specific for said procaryotic or eucaryotic polypeptide. In a preferred embodiment the polypeptides of the present invention can be synthesized as a fusion protein of active $\beta$-galactosidase and the peptide encoded by the nucleotide sequence selectively expressed in pre-B cells or by a fragment thereof. Such fusion proteins can be prepared and purified as describe by Rüther, et al., EMBO J., 2, 1791-1794, [1983].

Moreover the polypeptide of the present invention may form heterodimers via cysteine residues with possibly immunoglobulin H chains as partner.

Furthermore antibodies, in particular monoclonal antibodies can be raised against the polypeptides of the present invention in a manner known per se. Suitable host animals for eliciting antibodies include mammals such as rabbits, horses, goats, ginea-pigs, rats, mice, cows, sheep, etc. The resulting antiserum will contain antibodies which will selectively react with and bind to said polypeptides. The antiserum will either be used directly or the specific antibodies will be purified by methods known in the art e.g. by ammonium sulfate precipitation. The antiserum or the specific antibodies can be used in a well known manner for diagnostic or therapeutic purposes e.g. antibody mediated cytotoxic reactions as well as for purification purposes (e.g. affinity chromatography).

In a diagnostic assay various methods which are generally known may be employed for the determination if pre-B cells express the above mentioned polypeptides.

In one such procedure a lymphocyte preparation is first treated with unlabeled antibody against the polypeptide of the present invention and after washing treated with a labeled antibody against said antibody. After a further washing step the presence or absence of the label is determined. Only pre-B cells are labeled.

Suitable labels are enzymes, e.g. peroxidase, radiolabels of fluorescence-labels. In case that peroxidase is used as label the determination is performed with the substrate, e.g. with o-phenylene diamine or with tetramethyl benzydine.

Alternatively monoclonal antibodies may be used. Such monoclonal antibodies may be prepared based on the method developed by Köhler and Milstein (Nature, 256, 495-497, [1975]).

The methods for the determination of the polypeptides of the present invention described above can be conducted in suitable test kits comprising in a container antibodies elicited by a polypeptide of the present invention and one or more reagents needed to perform the diagnostic assay procedures described above.

Another method for the determination of a polypeptide of the present invention is the so-called "Western Blotting"-technique (Towbin, et al., Proc. Nat. Acad. Sci. U.S.A. 76, 4350-4354, [1979]). According to this technique polypeptides isolated from a lymphocyte preparation are separated on an SDS-polyacrylamide gel and then transferred from the gel electrophoretically on to nitrocellulose paper. This nitrocellulose paper is then treated with antibodies to the polypeptide to be tested. After washing, the nitrocellulose paper is then treated with an anti-antibody labeled with peroxidase. The peroxidase is then determined by a suitable substrate, e.g. with o-phenylene diamine. Of course other labels like radioactive or fluorescence labels may be used.

Alternatively the polypeptides of the present invention can be used to titrate antibodies against said polypeptide. Such antibodies may be present in serum from patients having an abnormal proliferation of lymphocytes of the B cell lineage (e.g. a non-T ALL).

A convenient technique for the determination of such antibodies is an enzyme linked immunosorbent assay (ELISA). According to this test a polypeptide of the present invention is adsorbed to a solid phase. Suitable solid phases are organic and inorganic polymers [amyloses, dextrans, natural or modified celluloses, polyacrylamides, agaroses, magnetite, porous glass powder, polyvinylidene fluoride (Kynar™) and latex], the inner wall of test vessels (test tube, titer plates or cuvettes of glass or artificial material) as well as the surface of solid bodies (rods of glass and artificial material, rods with terminal thickening, rods with terminal lobes or lamellae). Preferably the test is performed in the wells of a microtiterplate. The solid phase is then treated with the sera to be tested. After washing, anti-human IgG labeled with peroxidase is added. The determination of the peroxidase is performed with a corresponding substrate, e.g. with o-phenylene diamine. Also in this procedure the peroxidase can be exchanged by another label, e.g. by a radioactive or fluorescence label.

Furthermore the "Western-Blotting"-technique described above may also be used for the determination of antibodies against said polypeptides. In this case a sample of the polypeptide of the present invention is transferred onto nitrocellulose paper. This nitrocellulose paper is then treated with a serum sample to be tested. After washing and treatment with an anti-antibody labeled with peroxidase, the peroxidase is determined by a suitable substrate, e.g. with o-phenylene diamine. As mentioned above other labels like radioactive or fluorescence labels may be used.

Having now generally described this invention, the same will become even better understood by reference to the following Example which is included herein for purpose of illustration only and is not intended to limit the invention.

## Example

### Microsome-bound polysomal RNA preparation

Pre-B cells, e.g. 70Z/3 cells (ATCC No. TIB 158), were grown in RPMI-1640 medium containg 2mM glutamine, 5% heat inactivated fetal calf serum (Gibco), $\beta$-mercaptoethanol ($5 \times 10^{-5}$ M), streptomycin (100 $\mu$g/ml) and penicillin (100 units/ml) in a 10% $CO_2$ atmosphere to a density of $1 \times 10^6$ cells/ml. Before harvesting the cells were incubated for 15 min with cycloheximide (20 $\mu$g/ml). Microsome-bound polysomes were prepared as described (Mechler, et al., J. Cell Biol. 88, 29-36, [1981]. Briefly, cells were resuspended in 20 volumes of hypotonic buffer, 10mM Tris-HCl pH 7.4, 10mM KCl, 1.5mM $MgCl_2$, 10mM vanadium ribonucleoside complex, 20 $\mu$g/ml cycloheximide and allowed to swell for 15 min on ice. After homogenization by 30 strokes of a tight-fitting (B) Dounce glass homogenizer and centrifugation at 1000xg for 5 min, the cytoplasmic extract was diluted five fold in 2.5M sucrose with TKM buffer (80 mM KCl, 5mM $MgCl_2$, 50mM Tris-HCl pH 7.4) and layered over 2 volumes of 2.5M sucrose TKM buffer. Two layers of sucrose TKM buffer were successively added, one with 2.05M sucrose and a second with 1.3M sucrose. The gradients were centrifuged for 5 hr at 4°C in a SW27 rotor (Beckman Instruments, Inc.) at 25,000 rpm. Microsomes floating at the interface between the 2.05 and 1.3M sucrose layers were collected, dissolved in 0.5% NP-40 and layered over two layers of 2M sucrose-TKM and 0.5M sucrose-TKM. After centrifugation at 35,000 rpm for 4 hr with a Beckman 60 Ti rotor, the polysomal pellet was dissolved in 10mM Tris-HCl pH 7.5, 5mM EDTA, 0.2% SDS and then extracted with an equal volume of phenol:chloroform:isoamylalcohol (24:24:1).

Poly A containing RNA was prepared by repeated oligo(dT) cellulose chromatography (P-L Pharmacia, Uppsala, Sweden) in the presence of dimethyl sulfoxide as described (Bantle, Anal. Biochem. 72, 413-427, [1976]).

### Construction of the selected cDNA library

A subtracted cDNA library for pre-B cell specific clones was constructed essentially according to Davis et al. (supra). The first cDNA strand was synthesized with 10 $\mu$g of microsome-bound polysomal poly A containing RNA from 70Z/3 in 50mM Tris-HCl pH 8.3, 6mM $MgCl_2$, 60mM NaCl, 20mM dithiothreitol (DTT), 10 $\mu$g/ml of oligo ($dT_{12-18}$), 1mM of each four deoxyribonucleotides, 100 $\mu$Ci of $^{32}$P-dCTP (~3000 Ci/mmol), 60 units/ml of placental ribonuclease inhibitor, 100 $\mu$g/ml of actinomycin D, and 100 units of AMV reverse transcriptase (Stehlin, Co., Basle, Switzerland) at 40°C for 2 hr. After RNA hydrolysis, hybridization reactions were performed in 0.5M phosphate buffer, 5mM EDTA, 0.1% lithium laurylsulfate with twenty times excess amount of polysomal poly A containing RNA from a T cell hybridoma e.g. from the T cell hybridoma K62 at 68°C for 16-20 hr to achieve a Cot of 2000. The single-stranded fraction after hydroxylapatite (Bio-Rad Laboratories, Richmond, CA, USA) chromatography in 0.12M phosphate buffer, 0.1% lithium laurylsulfate, 5mM EDTA at 65°C was re-subtracted in the same conditions as above. The enriched single stranded cDNA obtained by these procedures (1.75% of the starting amount of cDNA) was made double stranded by the Klenow reaction in 50mM Hepes-KOH pH 6.9, 70mM KCl, 20mM $MgCl_2$, 10mM DTT, 50 $\mu$g/ml bovine serum albumin, 0.5mM of each deoxyribonucleotides, and 300 units/ml of Klenow fragment of DNA polymerase I (Stehlin) at 12°C for 20 hr and followed by the reverse transcriptase reaction in 0.1M Tris-HCl pH 8.3, 140mM KCl, 10mM $MgCl_2$ 30mM $\beta$-mercaptoethanol, 1mM of each of the four deoxyribonucleotides and 800 units of AMV reverse transcriptase at 42°C for 1 hr.

After removal of the hairpin loop with S1 nuclease (P-L Pharmacia), the cDNA was methylated with EcoRI methylase (New England Bio-Labs) and the ends were filled in with T4 polymerase (P-L Pharmacia). EcoRI linkers (New England Bio-Labs) were ligated to both ends by the T4 ligase (Stehlin) reaction with 15% polyethyleneglycol 6000 at 12°C for 16 h and were digested with EcoRI. After the digestion, the cDNA preparation was purified from the digestion product by passage through a column of Bio-Gel A15M, 100-200 mesh (Bio-Rad). Samples (~30 ng) were ligated to 3 $\mu$g $\lambda$gt11 DNA (ATCC No. 37194) that had been cut with EcoRI and treated with calf intestinal phosphatase. Ligated samples were packaged into phage (Young, et al., Proc. Natl. Acad. Sci. USA 80, 1194-1198, [1983]) and plated onto E. coli Y 1088 (ATCC No. 37195). Approximately 5 x $10^4$ total recombinants were obtained.

Differential hybridization of recombinant clones with radioactive B cell and T cell cDNAs

Recombinant phage clones were plated onto agar plates (Maniatis, et al., pp 68-73) with ampicillin (25 $\mu$g/ml) and transferred to nitrocellulose filter as described (Benton, et al., Science 196, 180-182, [1977]). The duplicate filters were screened with radioactive cDNA probes from the mRNA of either the pre-B cell or the T cell hybridoma used to construct the selected cDNA library. Radioactive cDNA probes were synthesized from each mRNA preparation by the AMV reverse transcriptase reaction with the random primer of oligodeoxyribonucleotide mixtures produced from calf thymus DNA (P-L Pharmacia).

Prehybridization and hybridization were performed in 5 x SSPE (750 mM NaCl, 50 mM NaH$_2$PO$_4$, 5 mM EDTA; pH 7,4), 5 x Denhardt's solution (Maniatis et al., supra, p. 327), 1% SDS, 1 $\mu$g/ml tRNA from E. coli, 1$\mu$g/ml poly(A), 100 $\mu$g/ml of denatured salmon sperm DNA at 68°C and the filters were washed finally with 0.1 x SSC (1 x SSC = standard saline citrate = 150 mM NaCl, 15 mM trisodium citrate, pH 7,0), 1% SDS at 65°C. The clones which showed positive after 7 days of exposure with intensifying screen at -70°C were rescreened with both probes. 200 individual phage clones were selected by this procedure out of 50,000 clones. For a second screening these clones were then individually grown up and the DNA was extracted by a method described by Yamamoto, et al., Virology 40, 734-744, [1970]. EcoRI digested insert fragments from phage clone DNA were separated by electrophoresis through a 1% low melting point agarose gel, and radiolabeled with $^{32}$P-dATP by the Klenow fragment reaction with the random hexanucleotide primers from calf thymus DNA (Feinberg, et al., Anal. Biochem. 137, 266-267, [1984]).

One radiolabeled insert DNA fragment designated pZ 183 was selected and used for hybridization with a panel of RNA preparations from various cells in order to show that the insert DNA fragment hybridizes selectively to poly A containing RNA from pre-B cells. Preferably different cell lines of different lineages at different stages of differentiation are used. These cell lines are considered to be "frozen" at a certain stage of normal cell development and, thus, represent the phenotype of these normal counterparts. A large variety of such cell lines are known to one skilled in the art and include the cell lines indicated in Figure 1 and Figure 2. Besides the desirability that at least one cell line selected is a pre-B cell line, a mature B cell line, a plasma cell line, a T cell line and a non-T/non B-cell line, none of the specific cell lines mentioned is indispensable. Suitable cell lines are e.g. the pre-B cell line 70Z/3 (ATCC No. TIB 158) or any other pre-B cell line e.g. as obtained by in vitro transformation of lymphoid cells by Abelson murine leukemia virus (Rosenberg, et al., supra). Examples for mature B cell lines are WEHI-231 (deposited at the European Collection of Animal Cell Cultures [ECACC], PHLS Centre for Applied Microbiology and Research, Porton Down, Salisbury, SP4 0JG. U.K. its accession number being ECACC No. 85022107), A 20-3 (ATCC No. TIB 208) and 2PK-3 (ATCC No. TIB 203). Examples for plasma cells are MPC-11 (ATCC No. CCL 167), SP2/0 (identical with SP2-0-Ag14, ECACC No. 85072401), X63 (identical with P3X63Ag8, ATCC No. TIB 9) and J558 (ATCC No. TIB 6). Examples for T cell lines are EL 4/9 (identical with EL4, ATCC No. TIB 39), BW5147 (ATCC No. TIB 47) and K62. Examples for other lineage cells are p388D1 (identical with P388.DI, ECACC No. 85011439) and WEHI-3 (ATCC No. TIB 68). Again none of the above listed cell lines is indispensable for performing the invention. Those of skill in the art may select other suitable cell lines from the large number of lymphoid cell lines which are known or which may be produced and characterized (e.g. by surface markers or by the rearrangement pattern of their immunoglobulin genes) by methods known in the art. Cell lines are preferred since they provide large amounts of homogenous cells as a source of genes expressed in a lineage- and differentiation-specific manner. Total cytoplasmic RNA (30 $\mu$g) from the cell lines selected was electrophoresed on 1.2% agarose/formaldehyde gel, transferred to nitrocellulose filter, and hybridized with the radioactive probe pZ 183. After hybridization the filters are washed with 0,2 x SSC, 1% SDS at 65°C and then exposed to film in the presence of intensifying screen at -70°C for 72 hours (so-called Northern blot, Alwine, et al., Proc. Natl. Acad. Sci 74, 5350-5354, [1977]).

The results in Figure 1 show that pZ 183 sequences are transcribed preferentially in the cell lines of the precursor B cell stages, including c$\mu^+$, sIg$^-$ pre-B, 28C-9, 204-3-1, 18-81, 70Z/3 as well as c$\mu^-$, sIg$^-$

progenitor cells, 40E-1, 220-8, 204-1-8, 230-238. All of such cells tested proved to be pZ 183 positive (Fig. 1 and Table I). On the other hand pZ 183 transcripts are not detected in early mature B cells (sIgM$^+$) e.g. WEHI-231, intermediate mature B cells (sIgM$^+$, sIgD$^+$) e.g. WEHI-279, pre-secreting B cells (sIgD$^+$) e.g. 2PK-3 or plasma blasts e.g. SP2/0, MPC-11, X-63 or J558. pZ 183 transcripts are also not detected in cells of T lineages e.g. BW5147, EL4 or K62, in monocytes, P388D1, WEHI-3 and in a fibroblast cell line, Ltk$^-$.

These data further suggest that pZ 183 sequences are only transcribed at the pre-B cell stage of B cell differentiation. In this pattern of differentiation expression of pZ 183 is similar, but not identical to the expression of the surface antigen detected by the monoclonal antibody AA4 (McKearn, et al., supra). The exceptions from this rule are the cell lines 2PK-3 and P388.DI which are pZ 183 negative, but AA4 positive.

Expression of pZ 183 sequences in pre-B cells at various stages of Ig-gene rearrangements

A set of Abelson-virus (A-MuLV) transformed pre-B cell lines exists that are "frozen" at different stages of Ig gene rearrangements (Alt, et al., supra; Yancopoulos, et al., Nature 311, 727-733, [1984]). Differential expression of pre-B cell-associated surface antigens characterized by the monoclonal antibodies 14.8, AA4 and GF1 has been observed with these cell lines (McKearn, et al., Eur. J. Immunol. 15, 295-298, [1985]). B cell precursors with fully rearranged ($V_H \rightarrow D_H \rightarrow J_H$) or with incompletely rearranged ($D_H \rightarrow J_H$) IgH genes both express in general the B 220 surface glycoprotein and the antigen recognized by the monoclonal antibody AA4, whereas some of these cells do not express an antigen recognized by the monoclonal antibody GF1. Therefore, pZ 183 expression was probed with RNA extracted from A-MuLV transformed pre-B cell clones obtained from different strains of mice, from different organs at different times of gestation, characterized by different stages of rearrangements of IgH and L chain genes and by other surface markers (listed in Table I).

Table I. Cell lines tested for the expression of pZ 183 transcripts

| Differentiation type | Name of clone | Transformation (induction) | Tissue source | Strain | IgH-IgL genes protein | IgH-IgL chain | Phenotype[e] B220 | sIgM | AA4 | GFI |
|---|---|---|---|---|---|---|---|---|---|---|
| Pre-B cell | 40E-1 | A-MuLV[a] | d 13-14 FL[b] | BALB/c | R[c] E[d] | – | + | – | + | + |
| | 220-8 | A-MuLV | Adult BM | BALB/c | R E | – | + | – | + | + |
| | 204-1-8 | A-MuLV | Adult BM | BALB/c | R E | – | + | – | + | . |
| | 298-18 | A-MuLV | Adult BM | BALB/c | R E | – | + | – | + | – |
| | 230-238 | A-MuLV | Adult BM | C57Bl/6 | R E | – | + | – | + | – |
| | 28C-9 | A-MuLV | d 17-19 FL | BALB/c | R E | + | + | – | + | + |
| | 204-3-1 | A-MuLV | Adult BM | BALB/c | R E | + | + | – | + | + |
| | 18-81 | A-MuLV | Adult BM | BALB/c | R R | + | + | – | + | + |
| | 70Z/3 | Nitrosourea | | (C57Bl/6 × DBA/2)F₁ | R R | + | + | – | + | – |
| Mature B cell | WEHI-279 | Radiation | | NZC | | | + | sIgM sIgD | + | + |
| | WEHI-231 | Mineral oil | | (BALB/c × NZB)F₁ | | | + | sIgM | – | – |
| | A20-3 | Spontaneous | | BALB/c | | | N.T.[f] | sIg | N.T. | N.T. |
| | M124.0 | Spontaneous | | BALB/c | | | N.T. | sIg | N.T. | N.T. |
| | K46.R.18 | Spontaneous | | BALB/c | | | + | sIgM sIgD | N.T. | N.T. |
| | 2PK-3 | Mineral oil | | BALB/c | | | – | sIgD | + | + |
| Plasma cell | MPC-11 | Mineral oil | | BALB/c | | | – | – | – | – |
| | SP2/0 | Hybridoma | | BALB/c | | | N.T. | N.T. | N.T. | N.T. |
| | X63 | Mineral oil | | BALB/c | | | N.T. | N.T. | N.T. | N.T. |
| | J558 | Mineral oil | | BALB/c | | | – | – | + | – |
| T cell | EL4/9 | | | BALB/c | | | N.T. | N.T. | N.T. | N.T. |
| | BW 5147 | Spontaneous | | AKR/J | | | N.T. | – | – | – |
| | K62 | Hybridoma | | AKR/J × C57Bl/6 | | | N.T. | N.T. | N.T. | N.T. |
| Other lineage cell | p388D1 | | | DBA/2 | | | | | | |
| | WEHI-3 | | | BALB/c | | | | | | |
| | Ltk⁻ | | | C3H | | | | | | |

[a] A-MuLV, Abelson murine leukemia virus.
[b] Day 13-14 fetal liver.
[c] R=rearranged in immunoglobulin genes.
[d] E=embryonic configuration in immunoglobulin genes.
[e] Referred from McKearn and Rosenberg, Eur. J. Immunol. 15, 295-298, [1985]
[f] N.T., not tested.

Results in Fig. 1 show that pZ 183 is expressed in all pre-B cell lines, as they originate either from pre-B cells of day 13-14 (40E-1), day 17-19 of gestation in fetal liver (28C-9), and from adult bone marrow (220-8, 204-1-8, 230-238, 204-3-1, 18-81). It is, of course, also expressed in the 70Z-3 pre-B cell line obtained by chemical carcinogenesis from which the cDNA library was made. pZ 183 is expressed in lines which have only $D_H \rightarrow J_H$ rearranged, clones which have $V_H \rightarrow D_H \rightarrow J_H$ rearranged (28C-9, 220-8, 18-81, 70Z/3, 204-1-8, 230-238), clones which either express $(c\mu^+)$ (28C-9, 204-3-1, 18-81, 70Z/3) or do not express $(c\mu^-)$ $\mu$ heavy

16

chain proteins (40E-1, 220-8, 204-1-8, 230-238), and clones which either express (40E-1, 220-8, 230-238, 28C-9, 204-3-1, 70Z/3) or do not express the GF1 surface antigen (204-18, 18-81). Again the cell lines mentioned are a selection of a wide variety of other cells which may be used for the same purpose.

RNA dot blot and Northern blot analysis

The level of expression of pZ 183-specific transcripts was estimated by RNA dot blot analysis of serially diluted cytoplasmic RNA samples from different cell lines (Fig. 2).

Preparation of cytoplasmic RNA for RNA dot blot analysis and transferring to nitrocellulose filters were done as described (White, et al., J. Biol. Chem. 257, 8569-8572, [1982]). pZ 183 DNA was used as a hybridization probe. Prehybridization, hybridization and washing were the same as in the experiments of Figure 1. Only pre-B cells show expression of pZ 183 transcripts. The level of expression was similar in all pre-B cells tested.

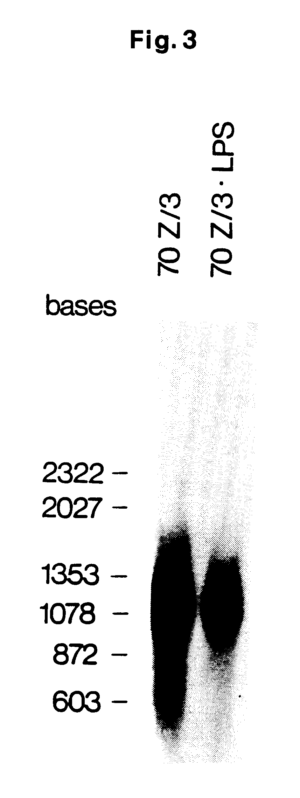

The size of the pZ 183-specific transcript(s) was analyzed in RNA prepared from either unstimulated or LPS-stimulated 70Z/3 cells by Northern blot analysis (Figure 3). Cytoplasmic RNA was isolated (Chirgwin, et al., Biochemistry 18, 5294-5299, [1979]) from 70Z/3 cells cultured with or without LPS (10 $\mu$g/ml) for 12 hours. The RNA was enriched for poly A containing RNA by oligo (dT) cellulose chromatography. One to forty micrograms of a RNA sample were electrophoretically separated on an agarose/formaldehyde gel and transferred to nitrocellulose filters as described (Maniatis et al., supra, pp 382-389). Prehybridization and hybridization were performed as described in the sections of differential hybridization but with 50% formamide at 42°C. The filters were finally washed with 0.2 x SSC, 1% SDS at 65°C.

pZ 183-specific RNA from both types of cells was found to be 1.2 kb in size. LPS stimulation of 70Z/3 cells which induces further maturation of these cells so that they express kL chains (Paige, et al., supra; Sakaguchi, et al., supra) does not influence the level of transcription or the size of the pZ 183-specific transcript.

Expression of pZ 183 sequences in the cells of normal organs and tissues.

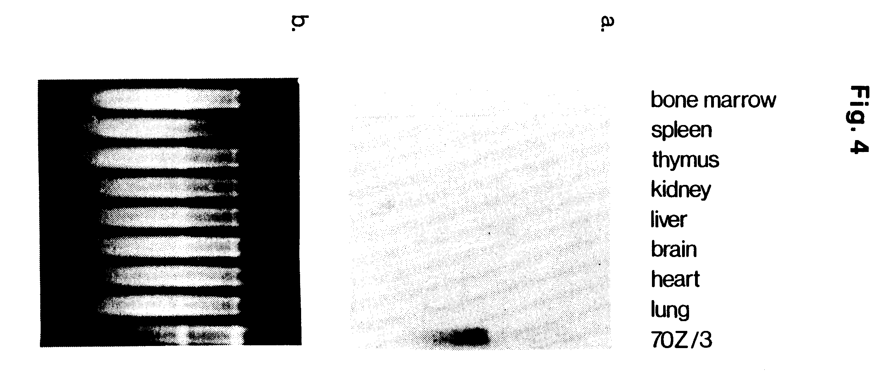

RNA was prepared from cells of spleen, thymus, kidney, bone marrow, lung, heart, brain and liver. Poly A containing RNA isolated from these organs, (5 $\mu$g each) and poly A containing RNA from 70Z/3 cells (2 $\mu$g) were electrophoretically separated, stained with ethidium bromide (Fig. 4b), transferred to nitrocellulose filter and hybridized to radioactive pZ 183 probe (Fig. 4a). Blots were exposed to X-ray film for 7 days at -70°C in the presence of an intensifying screen. It is clear from the data presented in Figure 4 that this analysis yielded no positive signals from RNA of all the organs tested. This indicated that the relative contribution of pre-B cells expressing pZ 183 in all of these organs must be too low in the total mixture of all other cells to be detectable by Northern gel analysis.

Therefore an "in situ" hybridization technique developed for tissue sections and single cells of lymphoid organs (Berger, EMBO J. 5, 85-93, [1986]) was used to probe for expression of pZ 183 sequences in these organs. For this, an insert fragment of pZ 183 of 380 bp was introduced into the M13 phage mp11 EcoRI site to obtain a single stranded pZ 183-specific probe which could be used in radiolabeled form in the "in situ" hybridization technique. The M13 vectors are described by Messing, et al., (supra) and by Yanisch-Perron et al. (Gene 33, 103-119, [1985]). The pZ 183-specific sequence was radiolabeled by primer extension DNA synthesis using $^3$H-labeled nucleotides and the labeled pZ 183 sequences, separated from the adjacent M13 sequences, were purified as the single strand probe molecules were, by gel electrophoresis. The positive strand phage DNA and the opposite strand phage DNA were tested by RNA dot hybridization on RNA samples of 70Z/3.

Ten thousand cells were attached to the glass slide, fixed, treated with pronase, prehybridized and hybridized with 10 $\mu$l of single strand radiolabeled DNA probe (6500 d.p.m./$\mu$l) in prehybridization solution (50% formamide, 0.6M NaCl, 1mM EDTA, 10mM Tris-HCl pH 7.5, 1 x Denhardt's solution, 150 $\mu$g/ml sonicated salmon sperm DNA, 500 $\mu$g/ml tRNA) for 3 days at 35°C. The slides were washed within 24 hours with three to four changes of 50% formamide, 0.6M NaCl, 1mM EDTA, 10mM Tris-HCl pH 7.5. Photographic development of the slides and counting of the grains were done as described (Berger, supra).

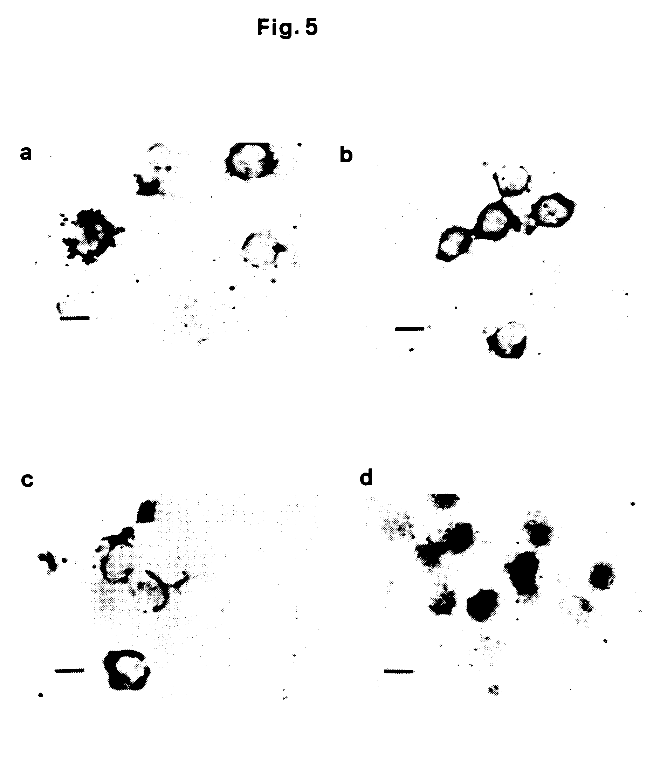

As can be seen in Figure 5, the radiolabeled pZ 183 probe readily detected specifically hybridizable cells expressing pZ 183-specific RNA within 15 day fetal liver cells (Figure 5a), and in total adult bone marrow cells (Figure 5c), but not in small resting spleen cells (Figure 5d) (more than a thousand cells counted). As one control, 70Z/3 cells were found to be positive. As another control the positive strand probe, as expected, did not label any cells of the fetal liver (Figure 5b) (more than a thousand cells counted). In fetal liver cells from day 15 of gestation, 2.5% of all cells (13 of 485 counted cells) were found to be pZ 183-positive while, at day 16, 4% (8 of 216 counted cells) of all cells were positive. In the adult bone marrow, 1% of cells (6 of 619 counted cells) expressed pZ 183 transcripts.

Finally, the expression of pZ 183 sequences was studied in tissue sections on day 16 fetal liver. Fetal liver organs were snap frozen in liquid nitrogen and prepared as 5 $\mu$m thick sections on glass slides. The in situ hybridization was carried out as described above. Figure 6 shows that the pZ 183 positive cells in fetal liver do not form foci, but are scattered in a diffuse way over large areas of the fetal liver.

## Construction of clone pZ 183-1

In order to obtain longer cDNA clones corresponding to the 1,2 kb transcript found in all pre-B cell lines a new cDNA library was constructed. This time a poly A containing RNA preparation obtained from 70Z/3 cells by the tailing method (Maniatis, et al., supra, pp. 230-242) was inserted into the pUC-13 vector (Messing, et al., supra). The new cDNA library was screened with the 380 nucleotide pair long insert of the cDNA clone pZ 183. Nine cDNA clone with the longest, i.e. a 0.7 kb insert, called pZ 183-1, was sequenced by the dideoxy chain termination method (Sanger, et al., supra). Figure 7 shows the restriction enzyme sites used for generating the fragments which were used for cloning into M13 phage vector. The arrows indicate the length of the fragments generated and the direction in which the fragments were sequenced.

The nucleotide sequence of the cDNA clone pZ 183-1 is shown in Figure 8. The 5$'$ to 3$'$ orientation was deduced from the location of the poly A tail and the poly A attachment site (underlined). Nucleotide positions are numbered from the most 5$'$ position of the insert cDNA fragment.

## Deduction of the amino acid sequence of the insert in pZ 183-1

A DNA sequence data bank (GenBank™, supra) was searched for possible evolutionary relationships of the pZ 183-1 sequence to known DNA sequences. Strong homology was detected for positions 240-554 to the C region nucleotide sequence of $\lambda_1$ L-chains. Equally strong homology was found for the adjacent 5$'$ positions 201-239 to the J segment sequence of $\lambda_1$ L chains (Table II). The reading frame of the $\lambda_1$ L chain J plus C nucleotide sequence was then imposed on the pZ 183-1 sequence as a reading frame. This happened to be reading frame 1 (starting with nucleotide 1) of the sequence shown in Figure 8. In this reading frame only two stop codons are found in positions 554 and 594, while reading frame 2 contains eleven (the first in position 9) and reading frame 3 nine (the first in position 101) stop codons. Comparison with the $\lambda_1$ L chain nucleotide sequence shows that the first stop codon in frame 1 of the pZ 183-1 sequence is found at the same position where the first, i.e. the functional stop codon of $\lambda_1$ L chains is positioned. Reading frame 1 is, therefore, the most likely candidate for a translatable sequence.

The amino acid sequence of the J-like and C-like regions of the pZ 183-1 gene deduced from reading frame 1 is shown in Figure 9 and compared to corresponding sequences of Ig and T cell receptor proteins. The strongest homology, again, is detectable with the J-segment and the C domain of $\lambda_1$ L chains (Table (II).

18

## Table II
## Homologies of pZ 183-1 sequences with immunoglobulin light chain genes and proteins

| Sequence position of pZ 183-1 | L-chain | literature reference | Homology with | |
|---|---|---|---|---|
| | | | nucleotide sequence* | deduced amino acid sequence+ |
| | $C\lambda_1$ | (a, d) | 77.9% | 66.7% |
| | $C\lambda_2$ | (a, d) | 70.2% | 61.1% |
| 240 to 554 | $C\lambda_3$ | (b, d) | 70.9% | 61.1% |
| | $C\lambda_4$ | (b) | 73.5% | – |
| | $Cx$ | (c, d) | 22.5% | 30.8% |
| | $J\lambda_1$ | (a, d) | 67.5% | 66.0% |
| | $J\lambda_2$ | (a, d) | 70.2% | 75.0% |
| 201 to 239 | $J\lambda_3$ | (b, d) | 67.6% | 50.0% |
| | $J\lambda_4$ | (b) | 70.2% | – |
| | $J_1x$ | (c, d) | 59.4% | 58.0% |

\* Nucleotide sequence comparisons were done by a computer program and are shown as percent matches per length.

+ Alignment of the deduced amino acid sequence of pZ 183-1 with mouse $\lambda_1$, $\lambda_2$, $\lambda_3$, and $\lambda_4$ L chains, as well as with κ L was done in order to maximize identities and structure homologies with pZ 183-1, with attention paid to the alignment of certain key conserved residues.

References:

a) Bothwell, et al., supra

b) Selsing, et al., Proc. Natl. Acad. Sci. USA 79, 4681-4685, [1982]

c) Hamlyn, et al., Cell 15, 1067-1075, [1978]

d) Kabat, et al., supra.

Furthermore, pZ 183-1 shows cysteine residues at the consensus positions characteristic of all Ig domains, and has the consensus sequence WVFGGGTKVTVLG characteristic of J-segments of the Ig genes. These results suggest that the 3′ portion of the pZ 183-1 cDNA clone belongs to a λ L chain-related gene locus which we call $\lambda_5$ ($J\lambda_5$, $C\lambda_5$).

19

In contrast, the $5'$ sequence adjacent to the $J\lambda_1$-like sequence of pZ 183-1 does not show strong homology to $V\lambda_1$, to any other known V-segment, or to any other gene known to us. It does not contain a cysteine at the position where a consensus cysteine residue would be expected in V-region domains of Ig. The $5'$ sequence adjacent to the J-like sequence of pZ 183-1 also does not show any detectable homologies to intron nucleotide sequences either $5'$ of $J\lambda_1$ or $3'$ of $V\lambda_1$.

Southern blot analyses of DNA from 70Z/3 cells. (Figure 10)

DNA was digested with the restriction enzyme HindIII, electrophoresed through a 1.0% agarose gel, blotted onto nitrocellulose filter and hybridized with the $^{32}$P-labelled insert of the clone pZ 183-1 (lane 1). Hybridization was carried out at 42° in 50% formamide and 5xSSC. The filters were washed at 65°C in 0.2xSSC. After exposure to X-ray film, the same filter was washed in distilled water containing 0.1% SDS at 95°C for 10 min. and repeatedly used to probe with $C\lambda_1$ (XhoI- PstI fragment of pAB 1-1, Bothwell, et al., supra) (lane 2), both pZ 183-1 and $C\lambda_1$ (lane 3), and $C\lambda_2$ (450 bp SacI fragment of Weiss, et al., Eur. J. Immunol. 15, 765-768, [1985]) (lane 4) successively.

Figure 10, lane 1 shows that the pZ 183-1 gene is carried in the genome of 70Z/3 cells in a HindIII fragment of over 9.2 kb. Furthermore the Southern blot analysis shows that it is different in context from $C\lambda_1$ (Figure 10, lane 2) and $C\lambda_2$ (Figure 10, lane 4). The unknown $5'$ sequences as well as the $J\lambda_1$- and $C\lambda_1$-like $3'$ sequences of pZ 183-1 appear to be on the Pst 1-fragment of 4.5 kb. It is on one PstI/BamHI, and on one PstI/HindIII genomic fragment, each 4.2 kb in length. Double digestion with PstI and EcoRI yields two fragments of 1.3 and 2.0 kb (Figure 11). No differences in the lengths of restriction enzyme fragments of DNA from liver (germ line) and from 70Z/3 cells (pre-B cell) are detectable (Figure 11). This indicated that the $5'$ and $3'$ regions of the pZ 183-1 gene are near to each other, i.e. on one 4.2 kb fragment in germ line DNA.

Construction of clone pZ 183-1a

From the above results it was clear that the clone pZ 183-1 did not correspond to the full-length 1.2 kb long transcripts which is selectively expressed in pre-B cells. Therefore a cDNA library was constructed from poly A containing RNA of the uninduced murine pre-B lymphoma cell line 70Z/3 by the method described by Okayama et al. (Mol. Cell Biol. 2, 161-170, [1982]). $5 \times 10^4$ individual recombinant clones were screened with the radioactive insert of pZ 183-1 as described above. Nine positive clones were identified. After restriction map analysis one clone was chosen which appeared to have the longest insert. This insert was cloned into the PstI site of the polylinker of pUC13 (Messing, et al., supra). The plasmid pZ 183-1a obtained was transfected into E. coli DH1 (ATCC No. 33849). The resulting DH1 (pZ 183-1a) was deposited on November 20, 1986 at the Deutsche Sammlung von Mikroorganismen (DSM) its accession number being DSM 3902. DNA sequencing was carried out by the dideoxy chain termination method by subcloning the fragments indicated in Fig. 12 into M13 mp18 and M13 mp19 vectors (Messing, et al., supra; also commercially available at New England Biolabs, Beverly MA, U.S.A. under catalogue No. 408 and at GIBCO BRL GmbH, Eggenstein, West Germany, catalogue Nos. 520-8227 SA and 520-8229 SA), using a 17-mer universal M13 primer (Amersham). Plasmid pZ 183-1a comprises the nucleotide sequence selectively expressed in pre-B cells which is shown on page 4.

Isolation and characterization of λ5 genomic clones

DNA from the 70Z/3 murine pre-B lymphoma, partially digested with MboI to an average size of 20 kb, was used to construct a genomic phage library in the vector EMBL-3 (Frischauf, et al., J. Mol. Biol. 170, 827-842, [1983]; Uematsu, et al., EMBO J. 5 2123-2129, [1986]). The genomic library was screened successively with three kinds of $^{32}$P-labelled probes: with $5'$-side cDNA (260 bp HindIII/StuI fragment of pZ 183-1a insert), $3'$-side cDNA (150 bp PvuII/EcoRI fragment of pZ 183), and $C\lambda 1$ (420 bp XhoI/PstI fragment of pAB-1-1, Bothwell, et al., supra). After hybridization, filters were washed under two stringencies, in an attempt to avoid cross hybridization between λ1 and λ5. Condition 1: 1xSSC, 0.1% SDS at 65°C, condition 2: 0.2xSSC, 0.1% SDS at 65°C. After screening of one million recombinant clones, four independent clones, 7pB5, 7pB12, 7pB18, and 7pB28 were found to be strongly positive in hybridization with both the $5'$-probe as well as the $3'$-probe of pZ 183. They were further examined by restriction map analysis. The PstI fragment of clone 7pB12 was subcloned into the PstI site of pUC-18 (Messing, et al., supra; Gibco BRL catalogue No. 520-5363 SA) and the resulting subclone 7pB12-1 was analyzed by restriction enzyme mapping (Fig. 13).

DNA sequencing was performed by the dideoxy chain termination method (Sanger, et al., supra) using M13 mp18 and M13 mp19 vectors with a 17-mer universal M13 primer and 17-18mer synthesized oligonucleotides (Messing, et al., supra). The fragments sequenced are indicated by arrows in Figure 13.

Figure 14 shows partial nucleotide sequence of the genomic form of λ5. Altogether 2.1 kb of DNA sequences were determined within a stretch of 3.75 kb. Comparison of these genomic sequences to the cDNA sequence of pZ 183-1a shows that λ5 is encoded by three exons (boxed in Figure 14). Exon I, 323 bp long is separated from Exon II, 116 bp in length, by an intron of approximately 1.2 kb. Exon II is separated from exon III, 465 bp in size, by an intron of approximately 1.35 kb. No difference was found between the nucleotide sequence of the pZ 183-1a cDNA clone and the corresponding portion of the gene.

The exon-intron boundaries are characterized by splice signal sequences corresponding to the consensus sequences described by Breathnach, et al., (Ann, Rev. Biochem. 50, 349-383, [1981]). It thus appears that the λ5 gene codes for a protein with 209 amino acids including a 30 amino acid-long signal peptide which are shown in Figure 14 in the one letter code described by Dayhoff et al., ("Atlas of Protein Sequence and Structure", M.O. Dayhoff, ed., Vol. 5, p. 17, Natl. Biomed. Res. Found., Silver Spring, Maryland, U.S.A., [1979]).

The structure of exon III and its surrounding introns

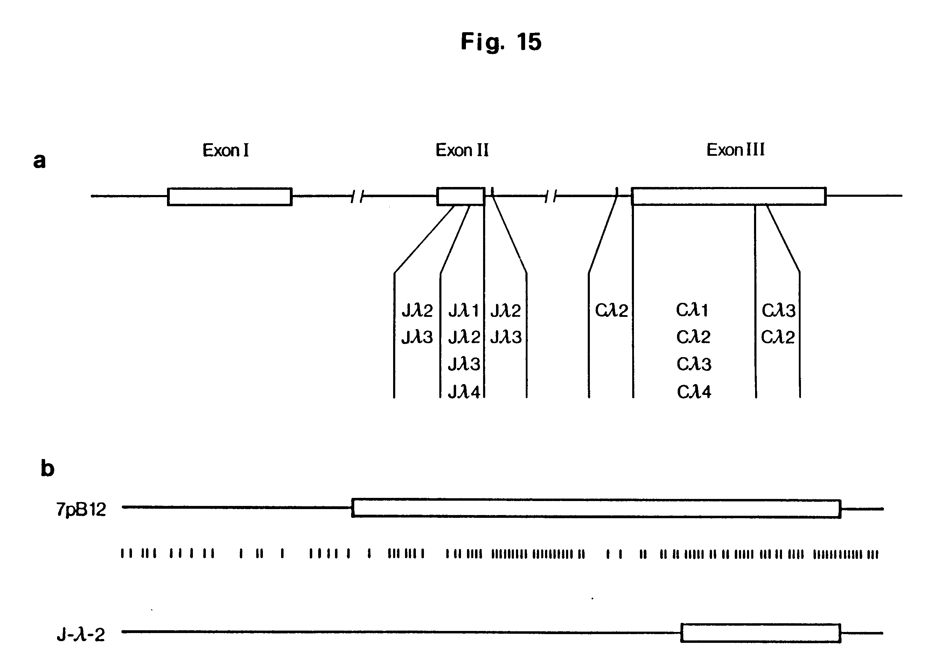

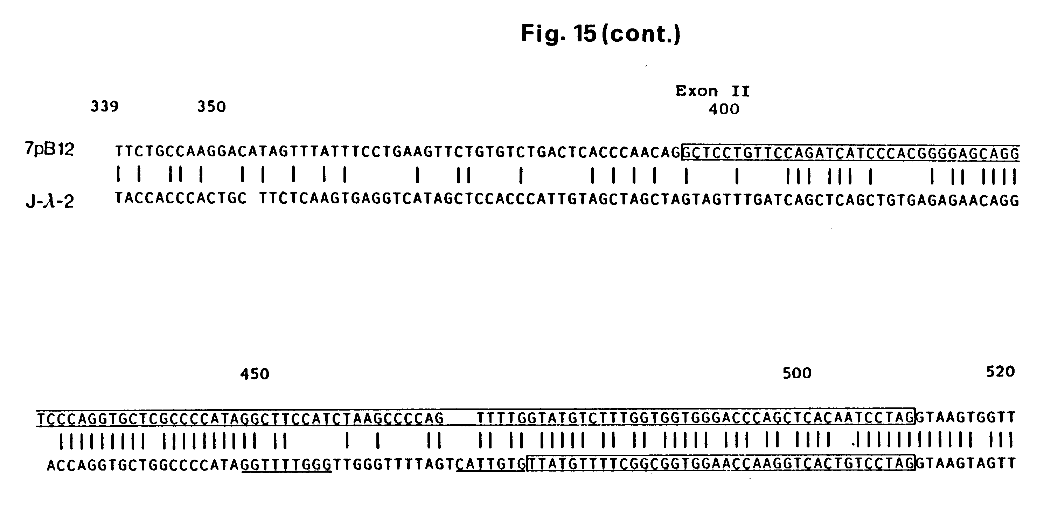

Exon III represents the portion of the $\lambda_5$ cDNA that has been shown to be highly homologous to $C\lambda_1$, $C\lambda_2$, $C\lambda_3$ and $C\lambda_4$ (Selsing, et al., supra). Moreover the intron sequences adjacent to the 5$'$ end of exon III are highly homologous to the 5$'$ flanking intron sequences of $C\lambda_2$. Sequences 3$'$ of the coding region of exon III show strong homologies to sequences 3$'$ of the coding region of the exons for $C\lambda_2$ and $C\lambda_3$ (Fig. 15). This shows that this entire region of the $\lambda_5$ gene is closely related to λL-chain constant regions.

The structure of exon II and its surrounding introns

Exon II is separated from exon III by an intron of approximately 1.35 kb in length, a distance very similar to that observed between $C\lambda_{1,2,3 \text{ or } 4}$ and $J\lambda_{1,2,3, \text{ or } 4}$ gene segments (Miller, et al., Nature 295, 428-430, [1982]; Blomberg, et al., Proc. Natl. Acad. Sci. USA 79, 530-533, [1982]). The 39 nucleotides at the 3$'$ end of exon II show strong homology to $J\lambda_1$, $J\lambda_2$ and $J\lambda_3$ exons (Miller, et al., supra). Intron sequences 3$'$ of exon II show strong homologies to the corresponding intron sequences 3$'$ of the exons encoding $J\lambda_2$ and $J\lambda_3$ (Fig. 15 and Table III).

In contrast to classical J segments of Ig genes, and, therefore, also of $\lambda_L$ chains, however, exon II extends its coding region at the 5$'$ end for another 78 nucleotides. This coding sequence within exon II of $\lambda_5$ shows partially strong, partially weak homologies to corresponding intron sequences 5$'$ or the exons of $J_{\lambda 2}$ and $J_{\lambda 3}$ (strong at positions 418-449, and weak at position 395-417 and 449-472). At the 5$'$ end of exon II, in the intron adjacent 5$'$ to this exon and for the rest of the 5$'$ region of the genomic structure of $\lambda_5$ no further homologies can be detected to λL-chain sequences (Fig. 15 and Table III).

## Table III

### Homologies of λ5 genomic sequences with genomic λL-chain sequences

In detail the following homologies were found (for numbering of positions see Fig. 14)

#### 7pB12 - area of Exon II

| | |
|---|---|
| position 340 to 394: | 33% to Jλ2 |
| position 395 to 418: | 38% to Jλ2 |
| position 419 to 449: | 84% to Jλ2 |
| position 450 to 472: | 38% to Jλ2 |
| position 473 to 510 (J-like part of Exon II): | |
| | 74% to Jλ1; 79% to Jλ2, |
| | 72% to Jλ3; 62% to Jλ4, |
| position 511 to 527: | 44% to Jλ1; 65% to Jλ2, |
| | 76% to Jλ3; 44% to Jλ4, |

#### 7pB12-area of Exon III

| | |
|---|---|
| position 233 to 258: | 38% to Cλ1; 58% to Cλ2, |
| | 46% to Cλ3; 35% to Cλ4, |
| position 259 to 578 (coding region of Exon III): | |
| | 78% to Cλ1; 70% to Cλ2, |
| | 71% to Cλ3; 74% to Cλ4, |
| position 579 to 608: | 50% to Cλ1; 53% to Cλ2, |
| | 53% to Cλ3; 43% to Cλ4, |

The structure of exon I and its flanking regions

Exon I does not have strong sequence homologies to the corresponding 5′ sequences of $\lambda_L$ chains, i.e. to $V_\lambda$ sequences. Sequences comparisons of exon I to other known sequences detected only short but significant homologies to leader sequences of T cell receptor λ chains, between positions 458 and 483. Nucleotide sequence homologies were also detectable to parts of known variable regions of Ig genes, i.e. to framework 1 of $V_{H11}$ (Fig. 16). Exon I contains an ATG as potential initiation site for protein synthesis at

22

positions 407 to 409. The deduced amino acid sequence in Fig. 14 shows an amino terminal signal peptide sequence which could be cleaved by a signal peptidase either at the consensus sequence V D G (nucleotide positions 488-496 in exon I) or I L S (nucleotide positions 506-514 in exon I) (Perlman, et al., supra).

Transcription initiation in pre B cells

The $5'$ end of mature $\lambda_5$ mRNA in pre-B cells was determined by primer extension. A synthetic oligonucleotide $5'$-CAGAGTCTGTCCTACTCT-$3'$ complementary to a part of Exon I was labeled with $\gamma$-$^{32}$P ATP using T4 polynucleotide kinase (Ingraham, et al., Mol. Cell. Biol. 6, 2923-2931, [1986]). Poly A containing RNA of the murine pre-B cell line 40E-1 was purified as described above. 500 ng of labeled oligonucleotides were annealed to either 10 $\mu$g of 40E-1 poly A containing RNA or yeast transfer RNA in 50 mM Tris/HCl pH 7.5, 75 mM KCl, 3 mM MgCl. Cloned Moloney murine leukemia virus reverse transcriptase (600 units) (Bethesda Research Laboratories) was added to each mixture and the reaction was carried out for 1 hour at 37°C in the presence of 0.5 mM dATP, dGTP, dCTP and dTTP, 10mM dithiothreitol, 1 mg/ml BSA, 1000 U/ml of ribonuclease inhibitor (Stehelin, Basle) and 100 $\mu$g/ml of actinomycin D.

After the termination of the reaction with EDTA (50 mM), the extended products were precipitated with ethanol. To precisely localize the cap site with respect to the genomic sequences, samples of primer extension reactions were analysed on 6% polyacrylamide DNA sequencing gels in parallel with sequencing reactions (Fig. 17). Two major sites of termination of extension were found. Site a (see Fig. 17) occurs at an A nucleotide and is, thus, the most likely site of transcription initiation and CAP formation (Breathnach, et al., supra).

Isolation of human cDNA homologous to mouse cDNA pZ 183-1a

A human fetal liver cDNA library in the vector $\lambda$gt11 was obtained from Clontech Laboratories, Inc. (4055 Fabian Way, Palo Alto, California 94303, USA - Catalog #HL 1005) 2.5 x $10^6$ recombinant phage clones were plated onto agar plates (Maniatis, et al., pp. 68-73) containing 25 $\mu$g/ml ampicillin and were transferred to nitrocellulose filters as described (Benton, et al., supra). The filters were screened with a $^{32}$P-labelled 700 nucleotide pair fragment generated by cutting the plasmid pZ 183-1a with PvuII and HindIII (mouse probe). Following digestion, the insert was separated by electrophoresis through a 1% low melting point agarose gel and radiolabeled with $^{32}$P-dATP by the Klenow fragment reaction with random hexanucleotide primers from calf thymus DNA (Feinberg, et al., supra). Prehybridization of the filters was done at 37°C in a solution containing 50% formamide, 5 x SSPE, 0,1% SDS, 10x Denhardt's solution. 100 $\mu$g/ml salmon sperm DNA, 1 $\mu$g/ml E.coli RNA, and 1 $\mu$g/ml poly(A). Hybridization of the filters was done in the same conditions with the addition of 1 x $10^6$ cpm/ml of $^{32}$P-labeled mouse probe. In order to detect human cDNAs cross-hybridizing with the mouse probe, the filters were exposed to autoradiographic film following each of a series of increasingly stringent washes. The washes each contained 2 x SSC, 0.1% SDS and were done with three 20 minute rinses at the following temperatures: 22°C (wash 1), 45°C (wash 2) and 65°C (wash 3). 15 primary clones were picked which were positive following the 65°C wash. After a second round of plating and screening of the 15 primary clones, 6 secondary clones were individually grown up and DNA was extracted by the method of Yamamoto et al. (supra). The DNA was cut with EcoRI and separated by electrophoresis through a 1% agarose gel, blotted onto nitrocellulose filter and hybridized with probes specific for the $5'$ or $3'$ regions of pZ 183-1a. Plasmid subclones in pUC13 (pHFL-1) or pUC18 (pHFL-2) were prepared by isolating the EcoRI inserts of $\lambda$gtII clones HFL-1 and HFL-2 from 1% low melting point agarose gels and ligating the inserts into the EcoRI sites of the pUC vectors. Restriction map analysis and DNA sequence analysis are used to characterize the cloned DNA sequence. RNA dot blots and Northern blots are used to show the selective expression of this sequence in human pre-B cells.

In a search for possible noncoding exons upstream of $\lambda_5$ with functions in the regulation of expression of this gene, total RNA preparations of 70Z/3 pre-B lymphoma cells ($\lambda_5$ +) and of EL4 thymic lymphoma cells ($\lambda_5$-) were probed by Northern blot analysis with different radiolabelled segments of the 7pB12 genomic clone containing the $\lambda_5$ gene plus 10 kb upstream of it. The subclone 7pB12-2 containing a BamHI-XbaI fragment of the $5'$ region of 7pB12 (Figure 18) was found to hybridize to 70Z/3, but not to EL4 total RNA (Figure 19). The size of the hybridizing RNAs, however, was not that of $\lambda_5$ mRNA, i.e. not 1.2 kb but 0.85 kb. Therefore a cDNA clone which would hybridize with this BamHI-XbaI fragment of the genomic 7pB12 clone was searched. In a library of $10^6$ once amplified cDNA clones constructed from 70Z/3 pre-B lymphoma poly A+ RNA around 100 positive clones were found. One out of seven strongly hybridizing clones was selected because it appeared to have the longest insert. This clone named pZ121 (Figure 20) contains a 780 base pairs long pre-B specific insert including 20 base pairs of poly A. The clone pZ121 has been deposited on April 23, 1987 at the Deutsche Sammlung von Mikroorganismen (DSM) in form of a sample of E.coli DH1 (pZ121) its accession numbers being DSM 4088. The expression of the gene corresponding to pZ 121 was probed with RNA from a variety of cell lines by Northern dot blot analysis (Figure 21). It was found to be expressed in Abelson virus-transformed cell lines of day 13-14 (40E-1), day 17-18 of gestation in fetal liver (28C-9, B3-P8-16-11-1), and of adult bone marrow (220-8, 204-1-8, 230-238, 204-3-1, 18-81), and in 70Z/3 pre-B cells from where the gene has been isolated. It is not expressed in mature Ig positive B cells (WEHI-279, WEHI-231, A 20-3, 2PK-3), in plasma cells (SP2/0, MPC-11), in T cells (EL4, BW5147, K62), and in macrophages (WEHI-3, P338DI) and Ltk$^-$ cells. The pattern of expression is indistinguishable from that of the $\lambda_5$ gene and indicates selective expression in pre-B cells.

The nucleotide sequence of pZ121 was determined by the strategy outlined in Figure 20 and is shown in Figure 22. A DNA sequence data bank (Gen Bank™, 1986) was searched for possible evolutionary relationships of the pZ121 sequence to known DNA sequences. For positions 477 to 785 homologies were found with sequences encoding murine variable regions $V_x$ and $V_\lambda$ of L chains. This nucleotide sequence shows 48% homology to the corresponding sequence of $V_{\lambda 1}$, and 46% to $V_{x21}$ (43% to framework [FR] II of $\lambda 1$, 57% to FR II of $V_{x21}$, 56% to FR III of $V_{\lambda 1}$, and 53% to FR III of $V_{x21}$). The longest open reading frame of the pZ121 cDNA codes for a protein of 142 amino acids including a 19 amino acid long leader sequence at its amino terminus (Figure 22). Comparison of the deduced amino acid sequence of pZ121 with the published sequences of $V_{x21}$ (Fong, et al., Biochem. Biophys. Res. Commun. 90, 832-841, [1979]), (Hamlyn, et al., Nucleic Acids Res. 9, 4485-4494, [1981]), $V_{\lambda 1}$ and $V_{\lambda 2}$ (Tonegawa, et al., Proc. Natl. Acad. Sci. USA 75, 1485-1489, [1978]) indicates that consensus cysteine residues as well as other consensus amino acids are conserved in the protein encoded by pZ121 which characterize Ig domains (Amzel, et al., Ann. Rev. Biochem. 48, 961-997, [1979]) (Figure 23). The homologies of pZ121 to $V_{x21}$, $V_{\lambda 1}$ and $V_{\lambda 2}$ are in the same ranges as those between $V_x$ and $V_\lambda$. The amino acid sequence of $V_{preB}1$ between position +1 and +103 shows 31% homology to the corresponding sequence of $V_{\lambda 1}$, and 34% to $V_{x21}$, in an area where $V_{\lambda 1}$ and $V_{x21}$ are 39% homologous. With $V_x$, $V_\lambda$ and $V_H$ pZ121, therefore, detects a gene which constitutes a fourth locus of V-related sequences which was called $V_{preB}1$ and which is 4.6 kb upstream from the $\lambda 5$ gene.

The evolutionary relationship of $V_{preB}1$ to Ig V-gene segments is further born out by its genomic organization. For the determination of this genomic organization the BamHI-XbaI fragment of the 7pB12 genomic clone was subcloned and sequenced as outlined in Figure 18. The sequence is given in Figure 23. It shows that $V_{preB}1$ is composed of two exons. Exon 1 codes for most of the leader peptide and is separated from exon II by 87 bp.

Sequences 5′ of the first exon of $V_{preB}1$ do not contain a TATA box as many Ig-V-gene segments do, nor an octamer consensus sequence (Parslow et al., Proc. Natl. Acad. Sci. USA 81, 2650-2654 [1984]) which is commonly found at 5′ ends of $V_L$ and $V_H$ genes of mouse and man. This is reminiscent of a similar lack of such sequences 5′ of the $\lambda_5$ gene and may indicate that different transcription regulating DNA sequences may be operative for pre-B cell specific expression of $V_{preB}1$ and $\lambda_5$. In fact, no homologies were detectable to any known nucleotide sequences.

The nucleotide sequences encoding the leader peptide show homology to those encoding $\lambda_1$, and $\lambda_2$ L chain leader peptides but not to those encoding the $x$L chain leader peptide in $V_x21$ (Figures 23 and 24). However, sequences of $V_{preB}1$ corresponding to the V segment of L chain genes shown almost equal homologies to those sequences encoding the V gene segment part of $\lambda$ as well as $x$ V regions of L chains. The V-like sequences in the $V_{preB}1$ gene are followed by sequences from position 768 to the end at position 1053 (Figure 23) where no homology to any known nucleotide sequence could be detected.

$V_{preB}1$ protein can associate either with itself, or with $V_H$ domains expressed in pre B cells, or even with $\lambda_5$ proteins. In this case $V_{preB}1$ and $\lambda_5$ proteins may form a complete V domain via noncovalent bonds which normally hold V gene segment-encoded amino acid in the three dimensional structure of a V domain together with J-gene segment encoded amino acids (Amzel, et al., supra). The amino terminal 62 amino acids of $\lambda_5$ and the carboxy terminal 26 amino acids of $V_{preB}1$ become in this case localized as a large protrusion at the CDR3 site. Together with the unusual extra 4 amino acids found in CDR 2 and two extra ones within FR 3 (Figure 23) $V_{preB}1$, in association with $\lambda_5$, constitutes an Ig domain with unusual, constant binding properties having some function in the regulation of pre-B cell development.

No recombination heptamer and nonamer signal sequences (Sakano et al., Nature, 280, 288-294 [1979]) can be found at the 3′ end of $V_{preB}1$ gene. It is therefore unlikely that $V_{preB}1$ can ever be rearranged as a V gene segment in the way that the Ig and T cell receptor gene loci do.

## Southern blot analysis detects two V~preB~ genes

Southern blot analysis was carried out with liver DNA from DBA/2 and C57BL/6 mice, and with DNA from the mouse pre-B cell line 70Z/3, generated in C57BL/6 x DBA/2 $F_1$ mice. High molecular weight DNAs were digested with five restriction enzymes, EcoRI, BamHI, HindIII, PstI or KpnI, the fragments separated by gel electrophoresis and probed with the $V_{preB}1$ cDNA clone pZ 121. The hybridization pattern shown in Figure 24 indicates that two bands are detectable in EcoRI, BamHI and Hind III digested DNAs from the three sources, while three and four bands were seen with KpnI and PstI digested DNAs. Multiple hybridizing bands with all five enzymes indicated that there were multiple $V_{preB}$ genes in the mouse genome and prompted a structural analysis of the difference of the two genomic DNA fragments.

When DNA of the genomic clone 7pB12 containing the λ5 associated $V_{preB}1$ gene was digested with either KpnI or PstI and probed in Southern blots with pZ 121, it was found to contain the first and the last of the three respectively four bands seen in Figure 24. We conclude that the middle band of KpnI, and the middle two bands of PstI digestions seen in Figure 24 must contain the other $V_{preB}$ gene, which we call $V_{preB}2$. It should be noted that the band with the smallest molecular weight of both KpnI as well as PstI digestions seen in Figure 24, in fact, contains sequences of both $V_{preB}1$ and $V_{preB}2$ genes, as analyses with the appropriate genomic clones (see below) have shown. No differences in the lengths of restriction fragments of DNA from liver and from 70Z/3 cell lines have been detected for $V_{preB}1$ as well as $V_{preB}2$. This indicates that $V_{preB}1$ and $V_{preB}2$ genes are not rearranged during pre B-cell development.

## Isolation and characterization of the V~preB~2 gene

A genomic library was constructed from DNA of the pre-B cell line 70Z/3, which was partially digested with MboI to an average size of 20kb, using the EMBL-3 vector (Frischauf et al., supra). The genomic library was screened with a $^{32}$P-labelled pZ 121 probe. Prehybaridization and hybridization were carried out with 50% formamide at 42°C (Maniatis et al., supra). Filters were finally washed with 0.2 x SSC, 0.1% SDS at 65°C. After screening of one million recombinant amplified clones, six independent clones were found and further examined by restriction map analysis. DNAs of possible clones were then digested with PstI and KpnI and probed in Southern blots with pZ 121. Two clones, 7pB60 and 7pB70, contained the appropriate bands, i.e. the $V_{preB}2$ gene.

The 4.4 kb BglII-BamHI fragment of 7pB70 was subcloned into BamHI site of pUC18 and was analyzed by restriction enzyme mapping (Figure 25) and DNA sequencing (Figure 22). Comparison to $V_{preB}1$ indicates that $V_{preB}2$ has a 97% nucleotide homology to $V_{preB}1$ in an area of 1 kb which covers the cDNA coding region of $V_{preB}1$.

More specifically $V_{preB}2$ differs in the nucleotide sequence of the coding exon from $V_{preB}1$ in nine bases, five of which constitute replacement mutations within the presumed amino acid sequences of the two $V_{preB}$ genes. The two $V_{preB}$ genes are apparently preserved as two copies in many laboratory strains of mice, in wild mice and in other closely related rodents such as hamsters, rats and guinea pigs. $V_{preB}2$ has not yet been linked at the molecular level to $V_{preB}1$ and λ5, nor have any of them been linked to any other known genes such as those encoding the λL chains. All three genes are located on chromosome 16 of the mouse, which also harbors and λL chain genes (D'Eustachio et al., J. Exp. Med. 153, 793-900, [1981]).

In a little more than the 10 kb of DNA on chromosome 16 in which $V_{preB}1$ and λ5 are located, five areas of DNA sequences can be discerned which appear to have evolved in quite separate ways. $V_{preB}1$ and λ5 are flanked at the 5$'$ and 3$'$ end by sequences with no homologies to known DNA sequences. The exon encoding the leader sequence of $V_{preB}1$ as well as the intron between this leader and the second exon of $V_{preB}1$ has strong homology to corresponding gene segments of V gene segments of λL chain genes (Tonegawa, et al., Proc. Natl. Acad. Sci. USA 75, 1485-1489, [1978]). $V_{preB}1$ (and $V_{preB}2$) has a much lower degree of homology to Vλ, a homology which is equally high to $V_K$ segments. This is followed by a third area encoding the 3$'$ end of $V_{preB}1$, the intervening non coding sequences between $V_{preB}1$ and λ5 and well as the 5$'$ located first exon of the λ5 gene which shows no homology to any known sequence. This area of sequence may well have evolved by an insertion into a preexisting V ($V_{preB}1$) and C (λ5). The fourth area of DNA, exon II, exon III and the intervening intron of the λ5 gene, shows again strong homology to λL chain genes, as do the leader sequence of $V_{preB}1$. The difference in homologies within the V segments of $V_{preB}1$ (and $V_{preB}2$) indicates that this area of the two genes may have been selected for functions which differ from those of λL chain V regions. The λ5-encoded protein is a potential candidate for heterodimer formation with H chains in pre-B cells therefore, the $V_{preB}1$ (and $V_{preB}2$) encoded proteins could well associate itself with H chains through the noncovalent association with λ5.

Detection of DNA sequences with homologies to the mouse VpreB genes in many mammalian species

A Southern blot survey of DNA from several species was conducted to determine if sequences homologous to the mouse $V_{preB}$ genes $V_{preB}1$ and $V_{preB}2$ could be detected by cross hybridization at various stringencies. High molecular weight DNAs were extracted from liver of mouse (C57BL/6), rat (Lewis), guinea pig (TRIK, Kleintierfarm Madörin AG, Füllinsdorf CH), rabbit (New Zealand white), hamster (Syrian), and from red blood cells of frog (Xenopus leavis) and from the human myeloid cell line U937 (ATCC No. CRL 1593) (Sundström et al., Int. J. Cancer 17, 565-577 [1976]) and the erythroid line K-562 (ATCC No. CCL 243) (Andersson et al., Int. J. Cancer 23 143-147 [1979]). High molecular weight calf thymus DNA was bought from Pharmacia (Uppsala, Sweden). After restriction enzyme digestion, 7 $\mu$g of DNA fragments were separated by electrophoresis on 0.7% agarose gels in TAE buffer (40 mM Tris/acetate pH 8.0, 1 mM EDTA) and transferred to nitrocellulose filters (BA85, Schleicher and Schüell) using 20 x SSC as transfer buffer. After transfer, filters were baked at 80°C under vaccum. Prehybridization and hybridization were done at 65°C in solutions containing 3 x SSC (1 x SSC = 150 mM NaCl, 15 mM sodium citrate), 10 x Denhardt's solution (1 x Denhardt's = 0.02% mg/ml bovine serum albumin, 0.02% polyvinyl pyrrolidone, 0.02% Ficoll), 0.1% SDS, 100 $\mu$g/ml salmon sperm DNA, and 1 mM EDTA. $^{32}$P-labelled (Feinberg and Vogelstein, supra) probes were used at 3 x $10^6$ cpm/ml (Cerenkov counts). The 5' EcoRI-AccI 560 bp fragment of $V_{preB}1$ contains all but the last 16 bases of the putative amino acid coding region and was isolated from $V_{preB}1$ cDNA plasmid clone pZ121. Washing of the filters was done first at room temperature in 2 x SSC, 0.1% SDS. For cross species hybridizations final washes were done with 3 twenty minute washes in 1 x SSC, 0.1% SDS at 65°C. Stringent washes were done in 0.2 x SSC, 0.1% SDS at 65°C.

Fig. 26 shows the pattern of restriction fragments hybridizing at a stringency calculated to allow 30% mismatched base pairs (Wetmur et al., J. Mol. Biol. 31 349-370 [1968]; Bonner et al., J. Mol. Biol. 81, 123-135 [1973]). Under these conditions DNA from all mammalian species of this survey hybridized to the mouse $V_{preB}1$ probe indicating a widespread conservation of this sequence at a surprisingly high level of homology. At least one hybridizing band was detected in every species. In mouse two $V_{preB}$ sequences with greater than 95% homology have been characterized. These sequences are on separate EcoRI fragments, giving rise to the two bands seen with mouse DNA in Fig. 26. These results suggest that sequences with homologies to the mouse $V_{preB}$ genes may be conserved in many species.

Isolation of human genomic DNA clones with sequences homologous to mouse $V_{preB}$

Human DNA contained a 15 kb EcoRI fragment that hybridized strongly to the mouse $V_{preB}1$ probe (Fig. 26). To determine the structure of this hybridizing fragment and its similarity with the mouse $V_{preB}$ genes a phage clone containing this 15 kb EcoRI fragment was isolated from a genomic library constructed from the human myeloid line U937.

DNA from human myeloid line U937 was digested to completion with EcoRI. 10 $\mu$g of digested DNA was separated by electrophoresis through 1% low melting point agarose (Bethesda Research Laboratories). DNA fragments ranging from 10 to 15 kbp were excised from the gel and purified by organic solvent extractions and ethanol precipitation. Purified, size-fractionated U937 DNA was used to construct a genomic phage library in the vector λgt WES•λB (ATCC No. 37028) (Leder et al., Science 196, 175-177 [1977]). The partial human genomic library was screened with $^{32}$P-labelled ECoRI-AccI fragment of mouse $V_{preB}1$ cDNA pZ121 under conditions of hybridization and washing used for cross species hybridization of Southern blots described above. Twenty positive clones were picked up in a screen of 3 x $10^6$ clones. One of these clones, termed HVPB, was further characterized by plasmid subcloning, restriction mapping and DNA sequencing. The clone HVPB was used to isolate a 2.7 kb HindIII fragment that hybridized strongly to the mouse $V_{preB}1$ cDNA probe. This fragment was inserted into the HindIII site of plasmid pUC18 to derive recombinant plasmid pHVPB-6.

DNA sequencing was performed on M13 mp18 and mp19 subclones of pHVPB-6 by the dideoxy chain termination method (Sanger et al., supra) using a universal M13 17-mer primer.

Structure and sequence of HVPB and comparison to other genes

Fig. 27 shows the restriction map determined for the genomic phage clone HVPB and the plasmid subclone pHVPB-6. A 2.7 kb HindIII fragment that hybridized strongly to mouse $V_{preB}1$ was subcloned in the HindIII site of pUC18. After more detailed restriction enzyme mapping, portions of this plasmid were further subcloned into M13 phage and sequenced according to the strategy shown in Fig. 27. Altogether 0.5 kb of DNA spanning the regions of strongest homology between human $V_{preB}$ and mouse $V_{preB}1$ were sequenced.

Alignment of the human and mouse nucleotide sequences revealed an overall homology of 76% (Fig. 27). Striking features of the comparison include the identical length of the putative leader sequences which are encoded by exon I and the 5′ 11 bp of exon II, and the similar lengths of intron I and exon II. The exon-intron boundaries contain characteristic splice sequences (Brethnach et al., supra). A split codon 16, with the first nucleotide separated from the second and third by intervening sequence, is present in human $V_{preB}$ and is a typical feature of Ig gene family V region leader sequences (Kabat et al., supra). Particularly noteworthy is that both the mouse and the human sequence diverge from typical V-region sequences at the same position, i.e. at the boundary to the CDR3 region of a typical V-gene. Comparison of these divergent sequences again show high homologies between mouse and man (Figure 27). The most remarkable feature in the similarities between mouse and human $V_{preB}$ are the two stretches of identical nucleotide sequences spanning the whole putative framework 2 (nucleotide positions 250 to 299) and a 3′ region of the putative framework 3 (nucleotide positions 399 to 420). This identity in nucleotide sequences is matched by an identity of the putative amino acid sequences (Figure 27).

A computer search of a sequence data bank (GenBank, Genetic Sequence Data Dank, Release 1986) revealed several regions within human $V_{preB}$ homologous to portions of immunoglobulin variable region sequences. Between the putative amino acid residues one and ninety-six, which constitute the mature V-region-like sequences, stretches of human and mouse $V_H$, $V_x$ and $V_\lambda$ sequences showed between 35-50% amino acid homology to human $V_{preB}$. In contrast, mouse $V_{preB}1$ and human $V_{preB}$ are 81% homologous in this region (Table IV).

## TABLE IV

## $V_{preB}$ gene comparisons: percent sequence homology

| | Leader | Intervening Sequence | V-like | non-Ig | Overall |
|---|---|---|---|---|---|
| Nucleotide | 77 | 60 | 87 | 64 | 76 |
| Amino acid | 74 | – | 81 | 54 | 76 |

Nucleotide residues 400 to 430 showed 94% homology to Vλ gene 4A, subgroup VII (Anderson et al., Nucleic Acids Res. 12, 6647-6661 [1984], 87% homology to three $V_H$ subgroup II genes ($V_H$32, $V_H$52 and H11; Matthyssens et al., Proc. Natl. Acad. Sci. USA 77, 6561-6565 [1980]; Rechavi et al., Proc. Natl. Acad. Sci. USA 79, 4405-4409 [1982]) and 79% homology to the $V_H$ gene HIGI (Kudo et al., Gene 33, 181-189 [1985]).

Genomic configuration of human $V_{preB}$ gene in lymphoid cell lines

Fig. 28 shows that no rearrangement of the human $V_{preB}$ gene was detected in pre-B cells e.g. line 207 (Findley et al., Blood 60, 1305-1309 [1982]), Nalm-6 (Hurwitz et al., Int. J. Cancer 23, 174-180 [1979]), in the multiple myeloma cell line IM-9 (ATCC No. CCL 159) (De Meyts in "Methods in Receptor Research" part I, M. Blecher ed, Marcel Dekker, Inc., New York, p. 309 [1976]) and the B lymphoblastoid cell line 1419 (Klobeck, Nature 309, 73-76 [1984]) or in cells from later stages of B cell differentiation e.g. LBW 4 (Hendershot et al., J. Exp. Med. 156, 1622-1634 [1982], GM607 (Klobeck, supra), in Burkitts lymphoma cell lines e.g. Raji (ATCC no. CCL 86) (Epstein et al., J. Natl. Cancer Inst. 34, 231-240 [1985]) and Daudi (ATCC No. CCL 213) (Klein et al., Cancer Res. 28, 1300-1310 [1968]) or in the myeloid cell line U937 (Sundström et al., supra), in the erythroid cell line K562 (Anderson et al., supra) and in the T cell line Jurkat (Schneider et al., Int. J. Cancer 19, 621-626 [1977]). All of these samples had only the germline 15 kb EcoRI hybridizing fragment. Therefore, neither mouse nor human $V_{preB}$ genes appear to be rearranged during B cell development.

## Expression of human V~preB~ in lymphoid cell lines

One of the important characteristics of the mouse $V_{preB}1$ gene is its restricted expression in mouse pre-B cell lines and therefore the pattern of expression of human $V_{preB}$ in human lymphoid lines by Northern blot analysis of poly(A)-selected RNA was examined.

Total RNA was isolated from cytoplasm after lysis of cells in 5% citric acid containing 0.1% NP-40 as described (Schibler et al., J. Mol. Biol. 142, 93-116 [1980]) and further purified by oligo(dT) cellulose chromatography as described above. 5 $\mu$g poly(A) enriched RNA was electrophoresed through 1% agarose gels containing 18 mM $Na_2HPO_4$, 2mM $NaH_2PO_4$, and 6% formaldehyde. Separated RNA was then blotted onto diazotized phenylthioether (DTP) paper (Schleicher and Schüell). Prehybridization of filters was done at 45°C in solutions containing 5 x SSPE (1 x SSPE = 150 mM NaCl, 10mM $NaH_2PO_4$, ImM EDTA), 5 x Denhardt's, 2 mM glycine, 50% deionized formamide, 100 $\mu$g/ml salmon sperm DNA, 20 $\mu$g/ml yeast tRNA and 1 $\mu$g/ml poly(A). Stringent hybridizations were done at 45°C in prehybridization solution lacking glycine but containing 10% dextran sulfate and $3 \times 10^6$ cpm/ml $^{32}$P-labelled probe. Cross species hybridizations were done at 37°C in hybridization solution containing only 30% formamide. Stringent washes were done at 65°C in 0.2 x SSC, 0.1% SDS. Cross species hybridization experiments were washed finally in 0.2 x SSC, 0.1% SDS at 37°C.

Human $V_{preB}$ is expressed only in pre-B cell lines 207, 697 (Findley et al., supra), Nalm-6 (Hurwitz et al., supra) but not in the cell lines LBW-4, Raji and Jurkat (Fig. 29). The human $V_{preB}$ poly(A)$^+$ mRNA is 0.85 kb in size as is the mRNA of its mouse homologue $V_{preB}1$. Under low stringency conditions the mouse $V_{preB}1$ gene also hybridizes to 0.85 kb RNA of human pre-B cell lines (Fig. 29). Similar intensities of hybridization and similar sizes of the RNAs which hybridize with the mouse $V_{preB}1$ probe and the human probe indicate that the same RNA molecules may hybridize to both probes. The upper band in Fig. 29B corresponds to the size of 28S ribosomal RNA and may be the result of crosshybridization of the mouse $V_{preB}1$ probe to human ribosomal RNA at low stringency. The pattern of RNA expression of human $V_{preB}$, so far, follows that of $V_{preB}1$ and $\lambda5$ in the mouse and indicates that human $V_{preB}$ is selectively expressed in human pre-B cell lines, but not in mature B cell or T cell lines.

## Claims

1. A polynucleotide which is capable of specifically hybridizing to a nucleotide sequence selectively expressed in human pre-B cells, which polynucleotide comprises a nucleotide sequence which is

GAGCTCTGCATGTCTGCACCATGTCCTGGGCTCCTGTCCTGCTCATGCACTTTGTCTACT

GCACAGGTGAGGGAACCCCCAGATCCCAAAGACTCCTGCCCCTTCCTTCATCCTGCCCTG

CCCCCACGGGCCACATGCATCTGTGTCACCAGGTTGTGGTCCTCAGCCGGTGCTACATCA

GCCGCCGGCCATGTCCTCGGCCCTTGGAACCACAATCCGCCTCACCTGCACCCTGAGGAA

CGACCATGACATCGGTGTGTACAGCGTCTACTGGTACCAGCAGAGGCCGGGCCACCCTCC

CAGGTTCCTGCTGAGATATTTCTCACAATCAGACAAGAGCCAGGGCCCCCAGGTCCCCCC

TCGCTTCTCTGGATCCAAAGATGTGGCCAGGAACAGGGGGTATTTGAGCATCTCTGAGCT

GCAGCCTGAGGACGAGGCTATGTATTACTGTGCTATGGGGGCCCGCAGCTCGGAGAAGGA

GGAGAGGGAGAGGGAGTGGGAGGAAGAAATGGAACCCACTGCAG

or a sub-part thereof or a homologous or degenerate sequence thereof.

2. A polynucleotide according to claim 1 which polynucleotide has the nucleotide sequence

GAGCTCTGCATGTCTGCACCATGTCCTGGGCTCCTGTCCTGCTCATGCACTTTGTCTACT
GCACAGGTGAGGGAACCCCCAGATCCCAAAGACTCCTGCCCCTTCCTTCATCCTGCCCTG
CCCCCACGGGCCACATGCATCTGTGTCACCAGGTTGTGGTCCTCAGCCGGTGCTACATCA
GCCGCCGGCCATGTCCTCGGCCCTTGGAACCACAATCCGCCTCACCTGCACCCTGAGGAA
CGACCATGACATCGGTGTGTACAGCGTCTACTGGTACCAGCAGAGGCCGGGCCACCCTCC
CAGGTTCCTGCTGAGATATTTCTCACAATCAGACAAGAGCCAGGGCCCCCAGGTCCCCCC
TCGCTTCTCTGGATCCAAAGATGTGGCCAGGAACAGGGGGTATTTGAGCATCTCTGAGCT
GCAGCCTGAGGACGAGGCTATGTATTACTGTGCTATGGGGGCCCGCAGCTCGGAGAAGGA
GGAGAGGGAGAGGGAGTGGGAGGAAGAAATGGAACCCACTGCAG

or a sub-part thereof.

3. A probe comprising a polynucleotide according to claim 1 or claim 2 and a label.

4. A probe comprising a polynucleotide having a sequence which is complementary to the sequence as defined in claim 1 or claim 2 and a label.

5. A replicable microbial vector comprising a polynucleotide as claimed in claim 1 or claim 2.

6. A replicable microbial vector comprising a polynucleotide having a sequence which is complementary to the sequence as defined in claim 1 or claim 2

7. A replicable microbial vector comprising a polynucleotide as claimed in claim 1 or claim 2 operatively linked to an expression control sequence.

8. A host organism transformed with a replicable microbial vector comprising a polynucleotide as claimed in claim 1 or claim 2, which host organism is capable of producing multiple copies of said polynucleotide.

9. A host organism transformed with a replicable microbial vector comprising a polynucleotide as claimed in claim 1 or claim 2 operatively linked to an expression control sequence, which host organism is capable of producing a polypeptide having an amino acid sequence encoded by said polynucleotide.

10. A polypeptide encoded by the nucleotide sequence of the polynucleotide as claimed in claim 1 or claim 2.

11. A polyclonal or monoclonal antibody raised against a polypeptide according to claim 10.

12. A method for the production of a polynucleotide as claimed in claim 1 or claim 2, which method comprises synthesizing said polynucleotide using conventional polynucleotide synthesis procedures.

13. A method for the production of a polynucleotide as claimed in claim 1 or claim 2, which method comprises culturing a host organism transformed with a replicable microbial vector comprising such a polynucleotide under conditions permitting production of multiple copies of the polynucleotide in said vector and isolating the polynucleotides produced from the culture.

14. A method for the production of a polypeptide as claimed in claim 10, which method comprises culturing a host organism transformed with a replicable microbial vector comprising a polynucleotide having a nucleotide sequence as defined in claim 1 or 2, which nucleotide sequence is operatively linked to an expression control sequence, under conditions permitting production of large amounts of polypeptide and isolating the polypeptide from the culture.

15. A method for the production of an antibody according to claim 11, which method comprises injecting a polypeptide according to claim 10 into a suitable host organism capable of eliciting an immune response against said polypeptide and isolating the antibody produced by methods known in the art.

16. A method for the production of a probe according to claim 3 or claim 4, which method comprises culturing a host organism according to claim 8 under conditions permitting production of multiple copies of the polynucleotide comprised in the probe and incorporating a label.

17. A method for determining the presence of a pre-B cell in a sample, which method comprises hybridizing a polynucleotide according to claim 1 or claim 2 or a probe according to claim 3 or claim 4 to RNA in said sample.

18. A method for determining the presence of a polypeptide encoded by a nucleotide sequence which is selectively expressed in pre-B cells in a sample, which method comprises using an antibody according to claim 11 in a manner known in the art.

19. Use of a polynucleotide according to claim 1 or claim 2 or a probe according to claim 3 or claim 4 for the determination of the presence of pre-B cells in a sample.

20. A probe according to claim 3 or 4 whenever prepared by a method as claimed in claim 16.

21. A test kit for determining the presence of a pre-B cell in a sample, which test kit comprises in a container a polynucleotide according to claim 1 or claim 2 or a probe according to claim 3 or claim 4.

22. A test kit for determining the presence of a polypeptide encoded by a nucleotide sequence which is selectively expressed in pre-B cells in a sample, which test kit comprises in a container a polyclonal or monoclonal antibody according to claim 11.

**Patentansprüche**

1. Ein Polynukleotid das fähig ist spezifisch an eine Nukleotidsequenz zu hybridisieren, die selektiv in pre-B Zellen exprimiert wird, wobei das Polynukleotid eine Nukleotidsequenz wie folgt enthält

```
GAGCTCTGCATGTCTGCACCATGTCCTGGGCTCCTGTCCTGCTCATGCACTTTGTCTACT
GCACAGGTGAGGGAACCCCCAGATCCCAAAGACTCCTGCCCCTTCCTTCATCCTGCCCTG
CCCCCACGGGCCACATGCATCTGTGTCACCAGGTTGTGGTCCTCAGCCGGTGCTACATCA
GCCGCCGGCCATGTCCTCGGCCCTTGGAACCACAATCCGCCTCACCTGCACCCTGAGGAA
CGACCATGACATCGGTGTGTACAGCGTCTACTGGTACCAGCAGAGGCCGGGCCACCCTCC
CAGGTTCCTGCTGAGATATTTCTCACAATCAGACAAGAGCCAGGGCCCCCAGGTCCCCCC
TCGCTTCTCTGGATCCAAAGATGTGGCCAGGAACAGGGGGTATTTGAGCATCTCTGAGCT
GCAGCCTGAGGACGAGGCTATGTATTACTGTGCTATGGGGGCCCGCAGCTCGGAGAAGGA
GGAGAGGGAGAGGGAGTGGGAGGAAGAAATGGAACCCACTGCAG
```

oder einen Teilabschnitt davon, oder eine homologe oder degenerierte Sequenz davon.

**2.** Ein Polynukleotid gemäss Anspruch 1, wobei das Polynukleotid die Nukleotidsequenz

```
GAGCTCTGCATGTCTGCACCATGTCCTGGGCTCCTGTCCTGCTCATGCACTTTGTCTACT
GCACAGGTGAGGGAACCCCCAGATCCCAAAGACTCCTGCCCCTTCCTTCATCCTGCCCTG
CCCCCACGGGCCACATGCATCTGTGTCACCAGGTTGTGGTCCTCAGCCGGTGCTACATCA
GCCGCCGGCCATGTCCTCGGCCCTTGGAACCACAATCCGCCTCACCTGCACCCTGAGGAA
CGACCATGACATCGGTGTGTACAGCGTCTACTGGTACCAGCAGAGGCCGGGCCACCCTCC
CAGGTTCCTGCTGAGATATTTCTCACAATCAGACAAGAGCCAGGGCCCCCAGGTCCCCCC
TCGCTTCTCTGGATCCAAAGATGTGGCCAGGAACAGGGGGTATTTGAGCATCTCTGAGCT
GCAGCCTGAGGACGAGGCTATGTATTACTGTGCTATGGGGGCCCGCAGCTCGGAGAAGGA
GGAGAGGGAGAGGGAGTGGGAGGAAGAAATGGAACCCACTGCAG
```

hat oder einen Teilabschnitt davon.

**3.** Eine Sonde enthaltend ein Polynukleotid gemäss Anspruch 1 oder 2 und einen Marker.

**4.** Eine Sonde enthaltend ein Polynukleotid mit einer Sequenz, die komplementär ist zur Sequenz, wie sie in Anspruch 1 oder 2 definiert ist und einen Marker.

**5.** Ein replizierbarer mikrobieller Vektor, der ein Polynukleotid enthält, wie es in Anspruch 1 oder Anspruch 2 beansprucht ist.

**6.** Ein replizierbarer mikrobieller Vektor, der ein Polynukleotid enthält, das eine Sequenz hat, die komplementär ist zur Sequenz wie sie in Anspruch 1 oder 2 definiert ist.

**7.** Ein replizierbarer mikrobieller Vektor, der ein Polynukleotid enthält wie es in Anspruch 1 oder 2 definiert ist und das operativ mit einer Expressionskontrollsequenz verbunden ist.

**8.** Ein Wirtsorganismus, der mit einem replizierbaren mikrobiellen Vektor, der ein Polynukleotid enthält wie es in Anspruch 1 oder 2 beansprucht ist, transformiert wurde, wobei der Wirtsorganismus fähig ist, mehrfache Kopien dieses Polynukleotids zu produzieren.

**9.** Ein Wirtsorganismus, der mit einem replizierbaren mikrobiellen Vektor transformiert ist, der ein Polynukleotid enthält wie es in Anspruch 1 oder 2 beansprucht ist und das operativ mit einer Expressionskontrollsequenz verbunden ist, wobei der Wirtsorganismus fähig ist, ein Polypeptid mit einer Aminosäuresequenz, wie sie vom Polynukleotid kodiert wird, zu produzieren.

**10.** Ein Polypeptid, das von der Nukleotidsequenz des Polynukleotides wie es in Anspruch 1 oder 2 beansprucht ist, kodiert wird.

**11.** Ein polyklonaler oder monoklonaler Antikörper, der gegen ein Polypeptid, wie es in Anspruch 10 definiert ist, hervorgerufen wird.

**12.** Eine Methode zur Herstellung eines Polynukleotids wie es in Anspruch 1 oder 2 beansprucht ist, wobei in der Methode das Polynukleotid unter der Verwendung konventioneller Polynukleotid-Synthese-Verfahren synthetisiert wird.

**13.** Eine Methode zur Herstellung eines Polynukleotides wie es in Anspruch 1 oder 2 definiert ist, wobei in der Methode ein Wirtsorganismus, der mit einem replizierbaren mikrobiellen Vektor transformiert ist, der ein solches Polynukleotid enthält, unter Bedingungen kultiviert wird, die die Herstellung von mehrfachen Kopien des Polynukleotides im genannten Vektor ermöglichen und wobei das Polynukleotid aus dieser Kultur isoliert wird.

**14.** Eine Methode zur Herstellung eines Polypeptids wie es in Anspruch 10 beansprucht ist, wobei in der Methode ein Wirtsorganismus kultiviert wird, der mit einem replizierbaren mikrobiellen Vektor transformiert ist, der ein Polynukleotid mit einer Nukleotidsequenz wie sie in Anspruch 1 oder 2 beansprucht ist, enthält, wobei die Nukleotidsequenz operativ mit einer Expressionskontrollsequenz verbunden ist, unter Bedingungen kultiviert wird, die die Herstellung einer grossen Menge des Polypeptides ermöglichen und wobei das Polypeptid aus der Kultur isoliert wird.

**15.** Eine Methode zur Herstellung eines Antikörpers gemäss Anspruch 11, wobei in der Methode ein Polypeptid gemäss Anspruch 10 in einen geeigneten Wirtsorganismus injiziert wird, der fähig ist, eine Immunantwort gegen das genannte Polypeptid hervorzurufen und wobei der so produzierte Antikörper mittels bekannter Methoden isoliert wird.

**16.** Eine Methode zur Herstellung einer Sonde gemäss Anspruch 3 oder 4, wobei in der Methode ein Wirtsorganismus gemäss Anspruch 8 unter Bedingungen kultiviert wird, die die Herstellung mehrfacher Kopien des Polynukleotids, das in der Probe enthalten ist, erlauben, und wobei ein Marker angefügt wird.

**17.** Eine Methode zur Bestimmung der Anwesenheit einer pre-B Zelle in einer Probe, wobei in der Methode ein Polynukleotid gemäss Anspruch 1 oder 2 oder eine Sonde gemäss Anspruch 3 oder 4 an RNA in der genannten Probe hybridisiert wird.

**18.** Eine Methode zur Bestimmung der Anwesenheit eines Polypeptides, das durch eine Nukleotidsequenz, die selektiv in pre-B Zellen exprimiert wird, kodiert wird, in einer Probe, wobei in der Methode ein Antikörper gemäss Anspruch 11 auf bekannte Art und Weise verwendet wird.

**19.** Die Verwendung eines Polynukleotides gemäss Anspruch 1 oder 2 oder einer Sonde gemäss Anspruch 3 oder 4 zur Bestimmung der Anwesenheit von pre-B Zellen in einer Probe.

**20.** Eine Sonde gemäss Anspruch 3 oder 4, die durch eine Methode gemäss Anspruch 16 hergestellt wurde.

**21.** Ein Testkit zur Bestimmung der Anwesenheit einer pre-B Zelle in einer Probe, wobei das Testkit in einem Behälter ein Polynukleotid gemäss Anspruch 1 oder 2 oder eine Sonde gemäss Anspruch 3 oder 4 enthält.

**22.** Ein Testkit zur Bestimmung der Anwesenheit eines Polypeptids das durch eine Nukleotidsequenz kodiert wird, die selektiv in pre-B Zellen exprimiert wird, in einer Probe, wobei das Testkit in einem Behälter einen polyklonalen oder monoklonalen Antikörper gemäss Anspruch 11 enthält.

**Revendications**

**1.** Polynucléotide qui est capable de s'hybrider spécifiquement à une séquence de nucléotides exprimée sélectivement dans des pré-B humaines, lequel polynucléotide comprend une séquence de polynucléotide qui est

EP 0 269 127 B1

```
GAGCTCTGCATGTCTGCACCATGTCCTGGGCTCCTGTCCTGCTCATGCACTTTGTCTACT
GCACAGGTGAGGGAACCCCCAGATCCCAAAGACTCCTGCCCCTTCCTTCATCCTGCCCTG
CCCCCACGGGCCACATGCATCTGTGTCACCAGGTTGTGGTCCTCAGCCGGTGCTACATCA
GCCGCCGGCCATGTCCTCGGCCCTTGGAACCACAATCCGCCTCACCTGCACCCTGAGGAA
CGACCATGACATCGGTGTGTACAGCGTCTACTGGTACCAGCAGAGGCCGGGCCACCCTCC
CAGGTTCCTGCTGAGATATTTCTCACAATCAGACAAGAGCCAGGGCCCCCAGGTCCCCCC
TCGCTTCTCTGGATCCAAAGATGTGGCCAGGAACAGGGGGTATTTGAGCATCTCTGAGCT
GCAGCCTGAGGACGAGGCTATGTATTACTGTGCTATGGGGGCCCGCAGCTCGGAGAAGGA
GGAGAGGGAGAGGGAGTGGGAGGAAGAAATGGAACCCACTGCAG
```

ou une sous-partie de celle-ci, ou une séquence homologue ou dégénérée de celle-ci.

**2.** Polynucléotide selon la revendication 1, lequel polynucléotide a la séquence de nucléotides

```
GAGCTCTGCATGTCTGCACCATGTCCTGGGCTCCTGTCCTGCTCATGCACTTTGTCTACT
GCACAGGTGAGGGAACCCCCAGATCCCAAAGACTCCTGCCCCTTCCTTCATCCTGCCCTG
CCCCCACGGGCCACATGCATCTGTGTCACCAGGTTGTGGTCCTCAGCCGGTGCTACATCA
GCCGCCGGCCATGTCCTCGGCCCTTGGAACCACAATCCGCCTCACCTGCACCCTGAGGAA
CGACCATGACATCGGTGTGTACAGCGTCTACTGGTACCAGCAGAGGCCGGGCCACCCTCC
CAGGTTCCTGCTGAGATATTTCTCACAATCAGACAAGAGCCAGGGCCCCCAGGTCCCCCC
TCGCTTCTCTGGATCCAAAGATGTGGCCAGGAACAGGGGGTATTTGAGCATCTCTGAGCT
GCAGCCTGAGGACGAGGCTATGTATTACTGTGCTATGGGGGCCCGCAGCTCGGAGAAGGA
GGAGAGGGAGAGGGAGTGGGAGGAAGAAATGGAACCCACTGCAG
```

ou une sous-partie de celle-ci

**3.** Sonde comprenant un polynucléotide selon la revendication 1 ou 2 et une marque.

**4.** Sonde comprenant un polynucléotide ayant une séquence qui est complémentaire de la séquence telle que définie dans les revendications 1 ou 2, et une marque.

**5.** Vecteur microbien réplicable comprenant un polynucléotide selon la revendication 1 ou 2.

**6.** Vecteur microbien réplicable comprenant un polynucléotide ayant une séquence qui est complémentaire de la séquence définie dans la revendication 1 ou 2.

**7.** Vecteur microbien réplicable comprenant un polynucléotide selon la revendication 1 ou 2, lié pour son fonctionnement à une séquence de commande de l'expression.

**8.** Organisme hôte transformé par un vecteur microbien réplicable comprenant un polynucléotide selon la revendication 1 ou 2, lequel organisme hôte est capable de produire des copies multiples de ce polynucléotide.

**9.** Organisme hôte transformé par un vecteur microbien réplicable comprenant un polynucléotide selon la revendication 1 ou 2, lié pour son fonctionnement à une séquence de commande de l'expression lequel organisme hôte est capable de produire un polypeptide ayant une séquence d'amino-acides codée par

33

ce polynucléotide.

**10.** Polypeptide codé par la séquence de nucléotides du polynucléotide selon la revendication 1 ou 2.

**11.** Anticorps polyclonal ou monoclonal produit contre un polypeptide selon la revendication 10.

**12.** Procédé de préparation d'un polynucléotide selon la revendication 1 ou 2, lequel procédé comprend la synthèse de ce polynucléotide en utilisant des techniques classiques de synthèse des polynucléotides.

**13.** Procédé de préparation d'un polynucléotide selon la revendication 1 ou 2, lequel procédé comprend la culture d'un organisme hôte transformé par un vecteur microbien réplicable comprenant un tel polynucléotide dans des conditions permettant la production de copies multiples du polynucléotide dans ce vecteur et l'isolement des polynucléotides formés dans la culture.

**14.** Procédé de préparation d'un polypeptide selon la revendication 10, lequel procédé comprend la culture d'un organisme hôte transformé par un vecteur microbien réplicable comprenant un nucléotide ayant une séquence de nucléotides telle que définie dans la revendication 1 ou 2, laquelle séquence de nucléotides est liée pour son fonctionnement à une séquence de commande de l'expression, dans des conditions permettant la production de grandes quantités de polypeptides et l'isolement du polypeptide de la culture.

**15.** Procédé de préparation d'un anticorps selon la revendication 11, lequel procédé comprend l'injection d'un polypeptide selon la revendication 10 dans un organisme hôte approprié capable de déclencher une réponse immunitaire contre ce polypeptide et l'isolement de l'anticorps produit par des procédés connus dans la technique.

**16.** Procédé de préparation d'une sonde selon la revendication 3 ou 4, lequel procédé comprend la culture d'un organisme hôte selon la revendication 8, dans des conditions permettant la production de copies multiples de polynucléotide compris dans la sonde et l'incorporation d'une marque.

**17.** Procédé de détermination de la présence d'une cellule pré-B dans un échantillon, lequel procédé comprend l'hybridation d'un polynucléotide selon la revendication 1 ou 2 ou d'une sonde selon la revendication 3 ou 4, à l'ARN contenu dans cet échantillon.

**18.** Procédé de détermination de la présence d'un polypeptide codé par une séquence de nucléotides qui est exprimée sélectivement dans les cellules pré-B d'un échantillon, lequel procédé comprend l'utilisation d'un échantillon selon la revendication 11, d'une manière connue dans la technique.

**19.** Utilisation d'un polynucléotide selon la revendication 1 ou 2 ou d'une sonde selon la revendication 3 ou 4 pour la détermination de la présence de cellules pré-B dans un échantillon.

**20.** Sonde selon la revendication 3 ou 4, chaque fois qu'elle est préparée par un procédé selon la revendication 16.

**21.** Trousse d'esai pour déterminer la présence d'une cellule pré-B dans un échantillon, laquelle trousse d'essai comprend dans un récipient un polynucléotide selon la revendication 1 ou 2 ou une sonde selon la revendication 3 ou 4.

**22.** Trousse d'essai pour déterminer la présence d'un polypeptide codé par une séquence de nucléotides qui est exprimée sélectivement dans des cellules pré-B présentes dans un échantillon, laquelle trousse d'essai comprend, dans un récipient, un anticorps polyclonal ou monoclonal selon la revendication 11.

Fig. 1

Fig. 2

# Fig. 3

EP 0 269 127 B1

# Fig. 4

bone marrow
spleen
thymus
kidney
liver
brain
heart
lung
70Z/3

a.

b.

## Fig. 5

## Fig. 6

Fig. 7

## Fig. 8

```
  1       10        20        30        40        50        60        70        80        90       100
TTGGGTCTAGTGGATGGTGTCCACCACATACTTTCCCCAAGCTCACGCAGAAAGGACGAGAGCTGTGGGCCCTGGAGCTTCAGTGGGAACAACAGGCCTA


         110       120       130       140       150       160       170       180       190       200
GCTATGGCCCTTCCCGGCAGCTCCTGTTCCAGATCATCCCACGGGGAGCAGGTCCCAGGTGCTCGTTTTATAGGCTTCCATCTAAGCCCCAGTTTTGGTA


         210       220       230       240       250       260       270       280       290       300
TGTCTTTGGTGGTGGGACCCAGCTCACAATCCTAGGTCAGCCCAAGTCTGACCCCTTGGTCACTCTGTTCCTGCCTTCCTTAAAGAATCTTCAGCCAACA


         310       320       330       340       350       360       370       380       390       400
AGGCCACAGCTAGTGTGTTTGGTGAGCGAATTCTACCCTGGTACTTTGGTGGTGGACTGGAAGGTAGATGGGGTCCCTGTCACTCAGGGTGTAGAGACAA


         410       420       430       440       450       460       470       480       490       500
CCCAACCCTCCAAACAGACCAACAACAAATACATGGTCAGCAGCTACCTGACACTGATATCTGACCAGTGGATGCCTCACAGTAGATACAGCTGCCGGGT


         510       520       530       540       550       560       570       580       590       600
CACTCATGAAGGAAACACTGTGGAGAAGAGTGTGTCACCTGCTGAGTGTTCTTAGACCACAATCCTCCCTGAAGCCTCAGGGGCCTGGATCTGAAGTGCC


         610       620       630       640       650       660       670       680       690       700
AGAAAAAGTTGTTTTTTGTTTTGTTTTTTGTTTTTTTTCCCATTAACCATCTCACTGTCTTTCCTGTGCCTAATACTCAATAAATATCTTACCACCAACCA (24)
```

# Fig. 9

# Fig.10

Fig.11

**Fig. 12**

**Fig. 13**

EP 0 269 127 B1

## Fig. 14

EP 0 269 127 B1

```
        10         20         30         40         50         60         70         80         90        100        110        120
CTGCAGAGAC TCTTGTTCCA TGGGGCAGGT GTTCAGTTGC TCTCTACGGC CCCTCTAAGC CCTGGGGACC TGCTCCACAG ACCCAGGGGC CCTCAGGGAC TGGATATCAG TCAGGCAGAG

       130        140        150        160        170        180        190        200        210        220        230        240
CTGCCCAGAG CTCTAGGGAT GGGTTAAGAC AGGCAGCTGT GAGTGAAAAC AGTTAGGCTT GCATCCCAAC CACAACCGGG TAGTCCTTTG GGAGAGAACT CCACAGATGG TGACCATGGG

                                            Exon I
       250        260        270        280        290        300        310        320        330        340        350        360
CACTGGGGTG TGCCTGCTGG TGGTGGAAAC TAGAGACAGC CTGGGGTTTG GCTACACAGA TCCACCTGCA CTGGAATAGC TTTTGGCCAC CAGAGGAGGA ACAATCCTTT TGCCGGGAGA
                                        a               b
       370        380        390        400        410        420        430        440        450        460        470        480
TCTACACTGC AAGTGAGGCT AGAGTTGACT TTGGACTTGA GGGTCAATGA AGCTCAGAGT AGGACAGACT CTGGGCACTA TCCCCAGGCA GTGTGAAGTT CTCCTCCTGC TGCTGCTGTT
                                                        M    K    L    R    V    G    Q    T    L    G    T    I    P    R    Q    C    E    V    L    L    L    L    L    L

       490        500        510        520        530        540        550        560        570        580        590        600
GGGTCTAGTG GATGGTGTCC ACCACATACT TTCCCCAAGC TCAGCAGAAA GGAGCAGAGC TGTGGGCCCT GGAGCTTCAG TGGGAAGCAA CAGGCCTAGC CTATGGGCCC TTCCCGGCAG
 G    L    V    D    G    V    H    H    I    L    S    P    S    S    A    E    R    S    R    A    V    G    P    G    A    S    V    G    S    N    R    P    S    L    W    A    L    P    G    R

       610        620        630        640        650        660        670        680        690        700        710        720
GTAAGAGACT TGCTTTTTGG GGAAGGTACG CGTGTAGGTC CACGGACTAG AGGCTAGAAT GAGTGACTGG GAAGGAAGGT GGCTATTGAG GCCATGGGTG TGAGAAGGAA GGATCTGCCT

       730        740        750        760        770        780
AAGAGGAGGG GGCTGTGCAA ATGCTAGCTT AAACTTGGTA CCTCGATCAA TTGCAAGGCC AGTGTTCT-----------------------650 bp-------------------------

        10         20         30         40         50         60         70         80         90        100        110        120
TTTGTGAGTT TGAGGCCAGC CTGGTCTACA TAGTAGTCTC ATGATAGAAA TAAAAACAAA AGAATCCTTG CAATATAGTG TGCATGGGTC CTGGGAGGAA GCATAGGGCC AGTAGGAACT

       130        140        150        160        170        180        190        200        210        220        230        240
GATGCACACA CGCATGTATG CACACACGCA CAAGCACATA TGCACACACA AATATTCACC AGAGATTCCT GGTTCCACCA CACCCACCAG AGAAGATATA CCACCCAGGA ATTTCTGAGA

       250        260        270        280        290        300        310        320        330        340        350        360
ATCTGCTGGG CCTGATATTG CCATCAACAC TGAAAAATTC AGAGATGGAA GTAAGAGATG GATAGATGGA CTTGTCGGGA TACTGGCTGC TGATCTATTT TCTGCCAAGG ACATAGTTTA
```

## Fig. 14 (cont.)

```
                                    Exon II
        370        380        390        400        410        420        430        440        450        460        470        480
TTTCCTGAAG TTCTGTGTCT GACTCACCCA ACAGGCTCCT GTTCCAGATC ATCCCACGGG GAGCAGGTCC CAGGTGCTCG CCCCATAGGC TTCCATCTAA GCCCCAGTTT TGGTATGTCT
                                     L  L   F  Q  I   I  P  R   G  A  G   P   R  C  S   P  H  R   L  P  S  K   P  Q  F   W  Y  V

        490        500        510        520        530        540        550
TTGGTGGTGG GACCCAGCTC ACAATCCTAG GTAAGTGGTT CTCATGGTCT CATGATCCAG CTGGCTCAGG------------------------1000 bp------------------------
 F  G  G  G   T  Q  L   T  I  L


         10         20         30         40         50         60         70         80         90        100        110        120
CTTAGTCTCA GCGGGGGAAC TGAGATTGCA AGGGTCTGGG TCTGGGTCAT TTTATCTGGA AGAGGAACAT GTTCTAATGG GATGCTAGGC TGTCTGCTCT CCAGGGGACT CAAGTGGTCA

        130        140        150        160        170        180        190        200        210        220        230        240
GAGGAGAAGA AGGAAGCATC CCTGGATGGA AGACTGATGC TGTAGTGAAT GGCCACAGAG CTCCTGATAA GAGAAGGACG CTTCCTTATC ACGTGGGCTC TCCTATGCTA ACTCTTATCT

                    Exon III
        250        260        270        280        290        300        310        320        330        340        350        360
CCTTCTCTAT CTGCGCAGGT CAGCCCAAGT CTGACCCCTT GGTCACTCTG TTCCTGCCTT CCTTAAAGAA TCTTCAGCCA ACAAGGCCAC ACGTAGTGTG TTTGGTGAGC GAATTCTACC
                  G   Q  P  K   S  D  P  L   V  T  L   F  L  P   S  L  K   N   L  Q  P   T  R  P   H  V  V  C   L  V  S   E  F  Y

        370        380        390        400        410        420        430        440        450        460        470        480
CAGGTACTTT GGTGGTGGAC TGGAAGGTAG ATGGGGTCCC TGTCACTCAG GGTGTAGAGA CAACCCAACC CTCCAAACAG ACCAACAACA AATACATGGT CAGCAGCTAC CTGACACTGA
 P  G  T  L   V  V  D   W  K  V   D  G  V  P   V  T  Q   G  V  E   T  T  Q  P   S  K  Q   T  N  N   K  Y  M  V   S  S  Y   L  T  L

        490        500        510        520        530        540        550        560        570        580        590        600
TATCTGACCA GTGGATGCCT CACAGTAGAT ACAGCTGCCG GGTCACTCAT GAAGGAAACA CTGTGGAGAA GAGTGTGTCA CCTGCTGAGT GTTCTTAGAG CACAATCCTC CCTGAAGCCT
 I  S  D  Q   W  M  P   H  S  R   Y  S  C  R   V  T  H   E  G  N   T  V  E  K   S  V  S   P  A  E   C  S  *

        610        620        630        640        650        660        670        680        690        700        710        720
CAGGGGCCTG GATCTGAAGT GCCAGAAAAA GTTGTTTTTT GTTTTGTTTT TTGTTTTTTT TCCCATTAAC CATCTCACTG TCTTTCCTGT GCCTAATACT CAATAAATAT CTTACCACCA
                                                                                                                      ******

        730        740        750
ACCAGAGAGT CTTGTCGATC TCATCACTTG CATG
```

EP 0 269 127 B1

## Fig. 15

## Fig. 15 (cont.)

```
                                                                    Exon II
      339            350                                             400
7pB12  TTCTGCCAAGGACATAGTTTATTTCCTGAAGTTCTGTGTCTGACTCACCCAACAG|GCTCCTGTTCCAGATCATCCCACGGGGAGCAGG
       I I   III I   I I  I   I II       I   II     I    I I I I  I      I    III III I   I II IIII
J-λ-2  TACCACCCACTGC TTCTCAAGTGAGGTCATAGCTCCACCCATTGTAGCTAGCTAGTAGTTTGATCAGCTCAGCTGTGAGAGAACAGG
```

```
                 450                                              500                520

      |TCCCAGGTGCTCGCCCCATAGGCTTCCATCTAAGCCCCAG    TTTTGGTATGTCTTTGGTGGTGGGACCCAGCTCACAATCCTAG|GTAAGTGGTT
       IIIIIIIII IIIIIIIIII II      I  I    II   II II IIIII II II IIIII III II IIII .IIIIIIIIIIII III
       ACCAGGTGCTGGCCCCATAGGTTTTGGGTTGGGTTTTAGTCATTGTG|TTATGTTTTCGGCGGTGGAACCAAGGTCACTGTCCTAG|GTAAGTAGTT
```

**Fig. 16**

```
       450              460              470                         480              490
        Q   C   E   V   L   L   L   L   L   L               L   G   L   V   D   G
7pB12   CAGTGTGAAGTTCTCCTCCTGCTGCTGCTG-----TTGGGTCTAGTGGATGGTG
                 | | | |   | | | | | | | | | | |   | | |       | | | | |
        GTTCACCTCCTGCTGCCTCTGGGTTTTTGGG
       113                                                       143
```

Tcr-γ Leader

```
   500           510           520           530           540           550           560
    V   H   H   I   L   S   P   S   S   A   E   R   S   R   A   V   G   P   G   A   S   V
   TCCACCACATACTTTCCCCAAGCT-CAGCAGAAAGGAGCAGAGCT-GTGGGCCCTGGAGCTTCAGTG
             | | | |   | | | | | | |     | | | |   |   | | | | |   | | |       | | | | |   | |   | | | | | |
   AGCTTCAGCAGTCAGGACCTGAGCTGGTGAAACCTGGGGCCTCAGTG
           348                                                                 394
```

V<sub>HII</sub>  FR1

EP 0 269 127 B1

## Fig. 17

Fig. 18

Fig.19

0.85kb—

←1.2kb

$V_{pre}B^1$          $\lambda_5$

Fig. 20

EP 0 269 127 B1

**Fig. 21**

# Fig. 22

EP 0 269 127 B1

```
7pB12-2  GATCTGCTTATTTGGGGCTCAGCCTCTCAAGGGGAGTTGAGGTCACTCTCCCTCAGGGAGACAAGCCCGCAGTCCTGGGTTCTGGCCCATGACGTTCCAGCAGGTGCTTCCTCCCAGAAT  120
7pB70-1  ---------C-G------------------G----------------------------------TG----A--------------------------T-----------------------
```

```
7pB12-2  GCTTCCCTGGGTCAAACCCAGAGCCACAAAGGCTTCCATTAGACCATTCTGGTAAGTGACAGAGTCACAGAGCCCAGAAAGCCTGGGAGGGTGGTGAGCAGGAACCAGGGGTGCAGTGAC  240
pZ121                                                                                    -------------------------------
7pB70-1  ----------------------------------------------------------T-----------A-------------------------------------------------
```

```
                                                                                                          -19
                                                                                                          M  A  W  T  S  V  L  L  M
7pB12-2  CCTCTCCCCAAAGCAGGGAGGAGAGTGCTTCCCAGCTGGTCAGGGCCCAGGAGCAGTGGCTGTAGGGGGCAGGGTGCTGCAGGTCTGGAGCCATGGCCTGGACGTCTGTCCTGCTCATGC  360
pZ121    ----------------------------------------------------------------------------------------------------------------------
7pB70-1  --------------------A----A-------------------T----------------------------------------------------------------------------
```

```
         -10                                                                                                      -1 +1
         L  L  A  Y  L  T  ↓                                                                                    ↓G  C  G  P  Q
7pB12-2  TGCTGGCCTATCTCACAGGTAAGGAAACTCTTGGGGCCCAGGGCTTCTTTGCTCCTCCTATGGCCTTGCTCTGCCCCAGTGATGGACATGCTTCTATCTTCTCAGGTTGTGGCCCTCAGC  480
pZ121    ------------------                                                                              ---------------
7pB70-1  --------C-C-------------G-----------T---------C-----------G--.
         (H)
```

```
                    +10                        +20                        +30                        +40
         P  M  V  H  Q  P  P  L  A  S  S  S  L  G  A  T  I  R  L  S  C  T  L  S  N  D  H  N  I  G  I  Y  S  I  Y  W  Y  Q  Q  R
7pB12-2  CCATGGTGCATCAGCCACCATTAGCATCTTCTTCCCTTGGAGCCACCATCCGCCTCTCCTGTACCCTGAGCAACGACCATAACATTGGCATTTACAGCATTTACTGGTACCAGCAGAGGC  600
pZ121    ----------------------------------------------------------------------------------------------------------------------
7pB70-1  --------------------C--------------------------------------------------------------------------------------------------
                       (S)
```

## Fig. 22 (cont.)

EP 0 269 127 B1

```
                    +50                        +60                        +70                        +80
          P  G  H  P  P  R  F  L  L  R  Y  F  S  H  S  D  K  H  Q  G  P  D  I  P  P  R  F  S  G  S  K  D  T  T  R  N  L  G  Y  L
7pB12-2   CGGGCCACCCTCCCAGGTTCCTGCTGAGATACTTCTCACACTCAGACAAGCACCAGGGTCCCGATATCCCACCTCGCTTCTCCGGGTCCAAAGATACGACCAGGAACCTGGGGTATCTGA   720
pZ121     ------------------------------------------------------------------------------------------------------------------------
7pB70-1   -----------------------------------------------------------------------------------------------------------G------------
                                                                                                                           (A)


                    +90                        +100                       +110                       +120
          S  I  S  E  L  Q  P  E  D  Q  A  V  Y  Y  C  A  V  G  L  R  S  Q  E  K  K  R  M  E  R  E  W  E  G  E  K  S  Y  T  D  L
7pB12-2   GCATCTCTGAACTGCAGCCTGAGGACGAGGCTGTGTATTACTGTGCCGTGGGGCTCCGGAGCCAGGAAAAGAAGAGGATGGAGAGGGAGTGGGAAGGAGAAAAGTCGTATACAGATTTGG   840
pZ121     ------------------------------------------------------------------------------------------------------------------------
7pB70-1   -----------------------------------------------C--T----------------C-----------A---------A------------------------------
                                                                           (H)


          G  S  *
7pB12-2   GATCTTAGGCTCTGGAGACATTCAGACCCTGAACTGAAGACAGAGTTTGCTTTGCTCGGCTAGTCTGGTATGGGAAGGAGGGGGTAGAACGTGAGGTTTTGCAGAGCCTAGAAGATGGAAT   960
pZ121     ------------------------------------------------------------------------------------------------------------------------
7pB70-1   ------------------------------------------------C-----------------------------------------T---G--C---------------------G-


                                                      .......
7pB12-2   TATGCAGCTTTTCCTTGTTCTGCGGTGTTGCTATGAGCCCCCATTGGAGGCTGGATTGTAGAATTAAAGCTGTTTTTACTGAATTTGGTCTGG                                1053
pZ121     --------------------------------------------------------------------------------------------- poly A ~20
7pB70-1   ------------------------------------------C-----------C------------------------G----------
```

# Fig. 23

LEADER

|  | | | | −20 | | | | | | | | | | | −10 | | | | | |
|---|---|---|---|---|---|---|---|---|---|---|---|---|---|---|---|---|---|---|---|---|
| V_preB1 | | | | | M | A | W | T | S | V | L | L | M | L | L | A | Y | L |
| Vλ1 | | | | | M | A | W | I | S | L | I | L | S | L | L | A | L | S |
| Vλ2 | | | | | M | A | W | T | S | L | I | L | S | L | L | A | L | C |
| Vκ21 | M | H | Q | T | S | M | G | I | K | M | E | S | N | T | L | V | F | I | S | I | L | L | W | L |

CDRI · FRII

|  | | | | +30 | | | | | | | | | | | | | +40 | | | | |
|---|---|---|---|---|---|---|---|---|---|---|---|---|---|---|---|---|---|---|---|---|---|
| V_preB1 | S | C | T | L | S | N | D | H | N | I | G | I | Y | S | I | Y | W | Y | Q | Q | R | P | G | H |
| Vλ1 | T | C | R | S | S | T | G | A | V | T | T | G | N | Y | A | N | W | V | Q | E | K | P | D | H |
| Vλ2 | T | C | R | S | S | T | G | A | V | T | T | G | N | Y | A | N | W | I | Q | E | K | P | D | H |
| Vκ21 | T | C | K | A | S | E | − | − | − | N | V | V | T | Y | V | S | W | Y | Q | Q | K | P | E | Q |

FRIII

|  | | | | +80 | | | | | | | | | | | | | +90 | | | | |
|---|---|---|---|---|---|---|---|---|---|---|---|---|---|---|---|---|---|---|---|---|---|
| V_preB1 | S | K | D | T | T | R | N | L | G | Y | L | S | I | S | E | L | Q | P | E | D | Q | A | V | Y |
| Vλ1 | S | L | I | G | N | K | A | A | − | − | L | T | I | T | G | T | Q | P | T | E | D | E | A | I | Y |
| Vλ2 | S | L | I | G | D | K | A | A | − | − | L | T | I | T | G | A | Q | P | T | E | D | D | A | M | Y |
| Vκ21 | S | G | S | A | T | D | F | T | − | − | L | T | I | S | S | V | Q | A | E | D | L | A | D | Y |

EP 0 269 127 B1

**Fig. 23 (cont.)**

## Fig. 24

# Fig. 25

EP 0 269 127 B1

## Fig. 26

Mouse V$_{pre}$B 1

HVPB

Left Arm    R    H  X    B  H    X    B                    R    Right Arm

1kb

pHVPB-6

HP    PS    S  BP    H

1kb

S    K  B P  P

100bp

-19
Leader →
      M   S   W   A   P   V   L   L   M   H   F   V   Y   C   T
Human ATG TCC TGG GCT CCT GTC CTG CTC ATG CAC TTT GTC TAC TGC ACA G
          *       * * *           ** * * * * **
Mouse ATG GCC TGG ACG TCT GTC CTG CTC ATG CTG CTG GCC CAC CTC ACA G
      M   A   W   T   S   V   L   L   M   L   L   A   H   L   T
                                      +   +   +   +   +

EP 0 269 127 B1

# Fig. 27 (cont.)

← IVS →

Human  [GT] GAGGGAACCCCCAGATCCCAAAGACTCC TGCCCCTTCCTTCATCCTGCCCTGCCCCCACGGGCCACATGCA   120

        *        * *** ***       ** **    *    *   * ** *** *    *      *** ****    *

Mouse  [GT] AAGGGAACTCTTGGGGTCCAGGGCTTCCTTGCTCCTCCTGTGGCCTTGCTCTGCCCCAGTGATGGACATGCT

← Leader | FRI →

        G   C   G   P   Q   P   V   L   H   Q   P   P   A   M   S   S

Human  TCTGTGTCACC [AG] GT TGT GGT CCT CAG CCG GTG CTA CAT CAG CCG CCG GCC ATG TCC TCG   180

        * * ***         *          ** **  ** **          *   * ** *** ***   *   *

Mouse  TCTATCTTCTC [AG] GT TGT GGC CCT CAG CCC ATG GTG CAT CAG CCA CCA TCA GCA TCT TCT

        G   C   G   P   Q   P   M   V   H   Q   P   P   S   A   S   S

                                    +   +                   +   +

13

        A   L   G   T   T   I   R   L   T   C   T   L   R   N   D   H   D   I   G   V

Human  GCC CTT GGA ACC ACA ATC CGC CTC ACC TGC ACC CTG AGG AAC GAC CAT GAC ATC GGT GTG   240

        *           *   *               *   *   *       *               *   *   ** *

Mouse  TCC CTT GGA GCC ACC ATC CGC CTC TCC TGT ACC CTG AGC AAC GAC CAT AAC ATT GGC ATT

        S   L   G   A   T   I   R   L   S   C   T   L   S   N   D   H   N   I   G   I

        +           +                   +               +               +           +

33              ← CDRI | FRII →                                    ← FRII | CDRII →

        Y   S   V   Y   W   Y   Q   Q   R   P   G   H   P   P   R   F   L   L   R   Y

Human  TAC AGC GTC TAC TGG TAC CAG CAG AGG CCG GGC CAC CCT CCC AGG TTC CTG CTG AGA TAT   300

                * *                                                               *

Mouse  TAC AGC ATT TAC TGG TAC CAG CAG AGG CCG GGC CAC CCT CCC AGG TTC CTG CTG AGA TAC

        Y   S   I   Y   W   Y   Q   Q   R   P   G   H   P   P   R   F   L   L   R   Y

                +

EP 0 269 127 B1

## Fig. 27 (cont.)

<pre>
53                                    ← CDRII | FRIII →
         F   S   Q   S   D   K   S   Q   G   P   Q   V   P   P   R   F   S   G   S   K
Human   TTC TCA CAA TCA GAC AAG AGC CAG GGC CCC CAG GTC CCC CCT CGC TTC TCT GGA TCC AAA    360
                 *               **          *   * * *       *               *   *
Mouse   TTC TCA CAC TCA GAC AAG CAC CAG GGT CCC GAT ATC CCA CCT CGC TTC TCC GGG TCC AAA
         F   S   H   S   D   K   H   Q   G   P   D   I   P   P   R   F   S   G   S   K
                 +               +               +   +


73                                    ← FRIII →
         D   V   A   R   N   R   G   Y   L   S   I   S   E   L   Q   P   E   D   E   A
Human   GAT GTG GCC AGG AAC AGG GGG TAT TTG AGC ATC TCT GAG CTG CAG CCT GAG GAC GAG GCT    420
             **              **          *               *
Mouse   GAT ACG GCC AGG AAC CTG GGG TAT CTG AGC ATC TCT GAA CTG CAG CCT GAG GAC GAG GCT
         D   T   A   R   N   L   G   Y   L   S   I   S   E   L   Q   P   E   D   E   A
             +               +


93      ← FRIII | Non Ig →
         M   Y   Y   C   A   M   G   A   R   S   S   E   K   E   E   R   E   R   E   W
Human   ATG TAT TAC TGT GCT ATG GGG GCC CGC AGC TCG GAG AAG GAG GAG AGG GAG AGG GAG TGG    480
         *               *       **      *   ***       *   ***   *           *
Mouse   GTG TAT TAC TGC GCT GTG GGG CTC CGG AGC CAC GAA AAG AAG AGA ATG GAG AGA GAG TGG
         V   Y   Y   C   A   V   G   L   R   S   H   E   K   K   R   M   E   R   E   W
         +               +       +           +           +   +   +


113
         E   E   E   M   E   P   T   A
Human   GAG GAA GAA ATG GAA CCC ACT GCA   -   -   -   -
         *   *       *   *** *** *   **
Mouse   GAA GGA GAA AAG TCG TAT ACA GAT TTG GGA TCT TAG
         E   G   E   K   S   Y   T   D   L   G   S   █
         +       +   +   +       +
</pre>

EP 0 269 127 B1

**Fig. 28**

Human V$_{preB}$

# Fig. 29

Human $V_{pre}B$

Mouse $V_{pre}B$ 1